# EUROPEAN PATENT SPECIFICATION

(11) **EP 3 494 124 B1**
(45) Date of publication and mention of the grant of the patent: **09.03.2022**
(21) Application number: 17837580.4
(22) Date of filing: 02.08.2017
(51) Int. Cl.: C07H 21/04, G01N 33/50, B82Y 5/00, B82Y 40/00, B82Y 30/00, C07H 1/00, C07H 21/00

(54) **CRISSCROSS COOPERATIVE SELF-ASSEMBLY**
KREUZKOOPERATIVE SELBSTANORDNUNG
AUTO-ASSEMBLAGE COOPÉRATIF CROISÉ

(30) Priority: 02.08.2016 US 201662370098 P
(43) Date of publication of application: 12.06.2019
(73) Proprietor: President and Fellows of Harvard College, Cambridge, MA 02138 (US); Dana Farber Cancer Institute, Inc., Boston, Massachusetts 02215 (US)
(72) Inventor: MINEV, Dionis, Boston MA 02115 (US); WINTERSINGER, Christopher, Boston MA 02115 (GB); SHIH, William, M., Cambridge MA 02139 (US)
(74) Representative: Hoffmann Eitle
(86) International application number: PCT/US2017/045013
(87) International publication number: WO 2018/026880

(56) References cited:
- WO-A1-2014/018675
- WO-A1-2015/130805
- KR-A- 20110 014 258
- US-A1- 2007 117 109
- US-A1- 2013 136 925
- US-A1- 2014 220 655
- D. HAN ET AL: "DNA Gridiron Nanostructures Based on Four-Arm Junctions", SCIENCE, vol. 339, no. 6126, 22 March 2013 (2013-03-22), pages 1412-1415, XP055675850, US ISSN: 0036-8075, DOI: 10.1126/science.1232252
- STEPHANIE S. SIMMEL ET AL: "Wireframe and Tensegrity DNA Nanostructures", ACCOUNTS OF CHEMICAL RESEARCH., vol. 47, no. 6, 10 April 2014 (2014-04-10) , pages 1691-1699, XP055675812, US ISSN: 0001-4842, DOI: 10.1021/ar400319n
- SCHLICHTHAERLE THOMAS ET AL: "DNA nanotechnology and fluorescence applications", CURRENT OPINION IN BIOTECHNOLOGY, LONDON, GB, vol. 39, 13 January 2016 (2016-01-13), pages 41-47, XP029569329, ISSN: 0958-1669, DOI: 10.1016/J.COPBIO.2015.12.014
- ARIVAZHAGAN RAJENDRAN ET AL: "Single-Molecule Analysis Using DNA Origami", ANGEWANDTE CHEMIE INTERNATIONAL EDITION, vol. 51, no. 4, 23 January 2012 (2012-01-23), pages 874-890, XP055133799, ISSN: 1433-7851, DOI: 10.1002/anie.201102113
- SHAWN M DOUGLAS ET AL: "Self-assembly of DNA into nanoscale three-dimensional shapes", NATURE, MACMILLAN JOURNALS LTD, LONDON, vol. 459, no. 7245, 21 May 2009 (2009-05-21), pages 414-418, XP002690757, ISSN: 0028-0836, DOI: 10.1038/NATURE08016
- SANTOS A ET AL: "Low-cost fabrication technologies for nanostructures: state-of-the-art and potential", NANOTECHNOLOGY, INSTITUTE OF PHYSICS PUBLISHING, GB, vol. 26, no. 4, 8 January 2015 (2015-01-08), page 42001, XP020277295, ISSN: 0957-4484, DOI: 10.1088/0957-4484/26/4/042001 [retrieved on 2015-01-08]

### SEQUENCE LISTING

<110> President and Fellows of Harvard College Dana-Farber Cancer Institute, Inc.
<120> CRISSCROSS COOPERATIVE SELF-ASSEMBLY
<130> H0498.70616WO00
<140> Not Yet Assigned
   <141> 2017-08-02
<150> US 62/370,098
   <151> 2016-08-02
<160> 688
<170> PatentIn version 3.5
<210> 1
   <211> 42
   <212> DNA
   <213> Artificial Sequence
<220>
   <223> Synthetic Polynucleotide
<400> 1
   atctgaactc gctacggcgg ggggagcccc cgatttagag ct 42
<210> 2
   <211> 42
   <212> DNA
   <213> Artificial Sequence
<220>
   <223> Synthetic Polynucleotide
<400> 2
   cattgctgat accgtttagc taacaaacat caagaaaaca aa 42
<210> 3
   <211> 42
   <212> DNA
   <213> Artificial Sequence
<220>
   <223> Synthetic Polynucleotide
<400> 3
   gatacttgcc ctctctgtac ataattaatt ttcccttaga at 42
<210> 4
   <211> 42
   <212> DNA
   <213> Artificial Sequence
<220>
   <223> Synthetic Polynucleotide
<400> 4
   gattgggcgt tatcaatgtt gttttgtcac aatcaataga aa 42
<210> 5
   <211> 42
   <212> DNA
   <213> Artificial Sequence
<220>
   <223> Synthetic Polynucleotide
<400> 5
   tctaatgaag acaaatcccc acgtcaccga cttgagccat tt 42
<210> 6
   <211> 42
   <212> DNA
   <213> Artificial Sequence
<220>
   <223> Synthetic Polynucleotide
<400> 6
   aaacatcggg ttgagtatta tgtggcgaga aaggaaggga ag 42
<210> 7
   <211> 42
   <212> DNA
   <213> Artificial Sequence
<220>
   <223> Synthetic Polynucleotide
<400> 7
   cgctggcatt cgcatcaaag gcgaattatt catttcaatt ac 42
<210> 8
   <211> 42
   <212> DNA
   <213> Artificial Sequence
<220>
   <223> Synthetic Polynucleotide
<400> 8
   agtttataaa tgagtatcaa tttagattaa gacgctgaga ag 42
<210> 9
   <211> 42
   <212> DNA
   <213> Artificial Sequence
<220>
   <223> Synthetic Polynucleotide
<400> 9
   tatcgacatc attacgcatc gcaacatata aaagaaacgc aa 42
<210> 10
   <211> 42
   <212> DNA
   <213> Artificial Sequence
<220>
   <223> Synthetic Polynucleotide
<400> 10
   ccatgcagac atcacgaagg tcaccagtag caccattacc at 42
<210> 11
   <211> 42
   <212> DNA
   <213> Artificial Sequence
<220>
   <223> Synthetic Polynucleotide
<400> 11
   aagataacgc ttgtgaaaat gagggcgctg gcaagtgtag cg 42
<210> 12
   <211> 42
   <212> DNA
   <213> Artificial Sequence
<220>
   <223> Synthetic Polynucleotide
<400> 12
   gctaacagta gggaaactgc ggcctgattg ctttgaatac ca 42
<210> 13
   <211> 42
   <212> DNA
   <213> Artificial Sequence
<220>
   <223> Synthetic Polynucleotide
<400> 13
   atgggttcag gatgcaggtg aaatcatagg tctgagagac ta 42
<210> 14
   <211> 42
   <212> DNA
   <213> Artificial Sequence
<220>
   <223> Synthetic Polynucleotide
<400> 14
   ctcggatggg agtaagcgta tgcagtatgt tagcaaacgt ag 42
<210> 15
   <211> 42
   <212> DNA
   <213> Artificial Sequence
<220>
   <223> Synthetic Polynucleotide
<400> 15
   agagtttctg cggcagttaa tcaatgaaac catcgatagc ag 42
<210> 16
   <211> 42
   <212> DNA
   <213> Artificial Sequence
<220>
   <223> Synthetic Polynucleotide
<400> 16
   gcaatacatc aaacgccgcg aacacccgcc gcgcttaatg cg 42
<210> 17
   <211> 42
   <212> DNA
   <213> Artificial Sequence
<220>
   <223> Synthetic Polynucleotide
<400> 17
   tcaggcactg cgtgaagcgg cagtaacagt accttttaca tc 42
<210> 18
   <211> 42
   <212> DNA
   <213> Artificial Sequence
<220>
   <223> Synthetic Polynucleotide
<400> 18
   atcaaaactc aacgagcagc ggttgggtta tataactata tg 42
<210> 19
   <211> 42
   <212> DNA
   <213> Artificial Sequence
<220>
   <223> Synthetic Polynucleotide
<400> 19
   agggttgtcg gacttgtgca aggaataccc aaaagaactg gc 42
<210> 20
   <211> 42
   <212> DNA
   <213> Artificial Sequence
<220>
   <223> Synthetic Polynucleotide
<400> 20
   agtccgtgaa gacggaaacc aaatcaagtt tgcctttagc gt 42
<210> 21
   <211> 42
   <212> DNA
   <213> Artificial Sequence
<220>
   <223> Synthetic Polynucleotide
<400> 21
   ctggggattt gacgcagacc tggttgcttt gacgagcacg ta 42
<210> 22
   <211> 42
   <212> DNA
   <213> Artificial Sequence
<220>
   <223> Synthetic Polynucleotide
<400> 22
   ttttcccagt cacgacgttg tgaaattgcg tagattttca gg 42
<210> 23
   <211> 42
   <212> DNA
   <213> Artificial Sequence
<220>
   <223> Synthetic Polynucleotide
<400> 23
   ttatcagtaa acagagaggt ttcgcaagac aaagaacgcg ag 42
<210> 24
   <211> 42
   <212> DNA
   <213> Artificial Sequence
<220>
   <223> Synthetic Polynucleotide
<400> 24
   tcagggatta atgaaagatg gaacaaagtt accagaagga aa 42
<210> 25
   <211> 42
   <212> DNA
   <213> Artificial Sequence
<220>
   <223> Synthetic Polynucleotide
<400> 25
   agtgtggcga tccgatagat gcggcatttt cggtcatagc cc 42
<210> 26
   <211> 42
   <212> DNA
   <213> Artificial Sequence
<220>
   <223> Synthetic Polynucleotide
<400> 26
   gggggatgtg ctgcaaggcg aatcagagcg ggagctaaac ag 42
<210> 27
   <211> 42
   <212> DNA
   <213> Artificial Sequence
<220>
   <223> Synthetic Polynucleotide
<400> 27
   agccagcttt ccggcaccgc tacctaccat atcaaaatta tt 42
<210> 28
   <211> 42
   <212> DNA
   <213> Artificial Sequence
<220>
   <223> Synthetic Polynucleotide
<400> 28
   ctttattatt cgcattcacc ctagttaatt tcatcttctg ac 42
<210> 29
   <211> 42
   <212> DNA
   <213> Artificial Sequence
<220>
   <223> Synthetic Polynucleotide
<400> 29
   ttggtgtaga tgggcgcatc gatcttaccg aagccctttt ta 42
<210> 30
   <211> 42
   <212> DNA
   <213> Artificial Sequence
<220>
   <223> Synthetic Polynucleotide
<400> 30
   cagaaataga agaattacag ctttcataat caaaatcacc gg 42
<210> 31
   <211> 42
   <212> DNA
   <213> Artificial Sequence
<220>
   <223> Synthetic Polynucleotide
<400> 31
   aagcgccatt cgccattcag gagacaggaa cggtacgcca ga 42
<210> 32
   <211> 42
   <212> DNA
   <213> Artificial Sequence
<220>
   <223> Synthetic Polynucleotide
<400> 32
   tcagaaaagc cccaaaaaca gctgattgtt tggattatac tt 42
<210> 33
   <211> 42
   <212> DNA
   <213> Artificial Sequence
<220>
   <223> Synthetic Polynucleotide
<400> 33
   gaggggacga cgacagtatc gaccgaccgt gtgataaata ag 42
<210> 34
   <211> 42
   <212> DNA
   <213> Artificial Sequence
<220>
   <223> Synthetic Polynucleotide
<400> 34
   tttttgttaa atcagctcat tagcccaata ataagagcaa ga 42
<210> 35
   <211> 42
   <212> DNA
   <213> Artificial Sequence
<220>
   <223> Synthetic Polynucleotide
<400> 35
   gtgggaacaa acggcggatt gcgcctccct cagagccgcc ac 42
<210> 36
   <211> 42
   <212> DNA
   <213> Artificial Sequence
<220>
   <223> Synthetic Polynucleotide
<400> 36
   tcgtaaaact agcatgtcaa tatcagtgag gccaccgagt aa 42
<210> 37
   <211> 42
   <212> DNA
   <213> Artificial Sequence
<220>
   <223> Synthetic Polynucleotide
<400> 37
   atgatattca accgttctag catattcctg attatcagat ga 42
<210> 38
   <211> 42
   <212> DNA
   <213> Artificial Sequence
<220>
   <223> Synthetic Polynucleotide
<400> 38
   ttaaattgta aacgttaata tcggaatcat aattactaga aa 42
<210> 39
   <211> 42
   <212> DNA
   <213> Artificial Sequence
<220>
   <223> Synthetic Polynucleotide
<400> 39
   tattttaaat gcaatgcctg atgagcgcta atatcagaga ga 42
<210> 40
   <211> 42
   <212> DNA
   <213> Artificial Sequence
<220>
   <223> Synthetic Polynucleotide
<400> 40
   tcaaaaataa ttcgcgtctg gagccaccac cctcagagcc gc 42
<210> 41
   <211> 42
   <212> DNA
   <213> Artificial Sequence
<220>
   <223> Synthetic Polynucleotide
<400> 41
   ggtagctatt tttgagagat cattaaccgt tgtagcaata ct 42
<210> 42
   <211> 42
   <212> DNA
   <213> Artificial Sequence
<220>
   <223> Synthetic Polynucleotide
<400> 42
   atggtcaata acctgtttag cttgcggaac aaagaaacca cc 42
<210> 43
   <211> 42
   <212> DNA
   <213> Artificial Sequence
<220>
   <223> Synthetic Polynucleotide
<400> 43
   aaaagggtga gaaaggccgg acgttataca aattcttacc ag 42
<210> 44
   <211> 42
   <212> DNA
   <213> Artificial Sequence
<220>
   <223> Synthetic Polynucleotide
<400> 44
   aacatccaat aaatcataca ggggagaatt aactgaacac cc 42
<210> 45
   <211> 42
   <212> DNA
   <213> Artificial Sequence
<220>
   <223> Synthetic Polynucleotide
<400> 45
   ctttatttca acgcaaggat acgccgccag cattgacagg ag 42
<210> 46
   <211> 42
   <212> DNA
   <213> Artificial Sequence
<220>
   <223> Synthetic Polynucleotide
<400> 46
   cgaacgagta gatttagttt gacttgcctg agtagaagaa ct 42
<210> 47
   <211> 42
   <212> DNA
   <213> Artificial Sequence
<220>
   <223> Synthetic Polynucleotide
<400> 47
   catttttgcg gatggcttag accgaacgtt attaatttta aa 42
<210> 48
   <211> 42
   <212> DNA
   <213> Artificial Sequence
<220>
   <223> Synthetic Polynucleotide
<400> 48
   agctgaaaag gtggcatcaa ttagggctta attgagaatc gc 42
<210> 49
   <211> 42
   <212> DNA
   <213> Artificial Sequence
<220>
   <223> Synthetic Polynucleotide
<400> 49
   agcttcaaag cgaaccagac ctttacagag agaataacat aa 42
<210> 50
   <211> 42
   <212> DNA
   <213> Artificial Sequence
<220>
   <223> Synthetic Polynucleotide
<400> 50
   attaagcaat aaagcctcag aggccttgat attcacaaac aa 42
<210> 51
   <211> 42
   <212> DNA
   <213> Artificial Sequence
<220>
   <223> Synthetic Polynucleotide
<400> 51
   ctgtagctca acatgtttta aaatatccag aacaatatta cc 42
<210> 52
   <211> 42
   <212> DNA
   <213> Artificial Sequence
<220>
   <223> Synthetic Polynucleotide
<400> 52
   ggcttttgca aaagaagttt tagactttac aaacaattcg ac 42
<210> 53
   <211> 42
   <212> DNA
   <213> Artificial Sequence
<220>
   <223> Synthetic Polynucleotide
<400> 53
   aggattagag agtaccttta agtaatttag gcagaggcat tt 42
<210> 54
   <211> 42
   <212> DNA
   <213> Artificial Sequence
<220>
   <223> Synthetic Polynucleotide
<400> 54
   aatattcatt gaatccccct cgaaacgatt ttttgtttaa cg 42
<210> 55
   <211> 42
   <212> DNA
   <213> Artificial Sequence
<220>
   <223> Synthetic Polynucleotide
<400> 55
   aagaggaagc ccgaaagact taatggaaag cgcagtctct ga 42
<210> 56
   <211> 42
   <212> DNA
   <213> Artificial Sequence
<220>
   <223> Synthetic Polynucleotide
<400> 56
   accctcgttt accagacgac gaacgctcat ggaaatacct ac 42
<210> 57
   <211> 42
   <212> DNA
   <213> Artificial Sequence
<220>
   <223> Synthetic Polynucleotide
<400> 57
   taacggaaca acattattac aagagccgtc aatagataat ac 42
<210> 58
   <211> 42
   <212> DNA
   <213> Artificial Sequence
<220>
   <223> Synthetic Polynucleotide
<400> 58
   atgtttagac tggatagcgt cataaagtac cgacaaaagg ta 42
<210> 59
   <211> 42
   <212> DNA
   <213> Artificial Sequence
<220>
   <223> Synthetic Polynucleotide
<400> 59
   tgaattacct tatgcgattt tttacaaaat aaacagccat at 42
<210> 60
   <211> 42
   <212> DNA
   <213> Artificial Sequence
<220>
   <223> Synthetic Polynucleotide
<400> 60
   aaacgagaat gaccataaat ccatacatgg cttttgatga ta 42
<210> 61
   <211> 42
   <212> DNA
   <213> Artificial Sequence
<220>
   <223> Synthetic Polynucleotide
<400> 61
   agatttagga ataccacatt caaatggatt atttacattg gc 42
<210> 62
   <211> 42
   <212> DNA
   <213> Artificial Sequence
<220>
   <223> Synthetic Polynucleotide
<400> 62
   cgaggcgcag acggtcaatc agttatctaa aatatcttta gg 42
<210> 63
   <211> 42
   <212> DNA
   <213> Artificial Sequence
<220>
   <223> Synthetic Polynucleotide
<400> 63
   gtcaggacgt tgggaagaaa agacaataaa caacatgttc ag 42
<210> 64
   <211> 42
   <212> DNA
   <213> Artificial Sequence
<220>
   <223> Synthetic Polynucleotide
<400> 64
   aggctggctg accttcatca ataccaacgc taacgagcgt ct 42
<210> 65
   <211> 42
   <212> DNA
   <213> Artificial Sequence
<220>
   <223> Synthetic Polynucleotide
<400> 65
   taaattgggc ttgagatggt tttttaacgg ggtcagtgcc tt 42
<210> 66
   <211> 42
   <212> DNA
   <213> Artificial Sequence
<220>
   <223> Synthetic Polynucleotide
<400> 66
   tgtgtcgaaa tccgcgacct gagtaataaa agggacattc tg 42
<210> 67
   <211> 42
   <212> DNA
   <213> Artificial Sequence
<220>
   <223> Synthetic Polynucleotide
<400> 67
   tacgaaggca ccaacctaaa actggtcagt tggcaaatca ac 42
<210> 68
   <211> 42
   <212> DNA
   <213> Artificial Sequence
<220>
   <223> Synthetic Polynucleotide
<400> 68
   ctttgaaaga ggacagatga atatcaacaa tagataagtc ct 42
<210> 69
   <211> 42
   <212> DNA
   <213> Artificial Sequence
<220>
   <223> Synthetic Polynucleotide
<400> 69
   gtagcaacgg ctacagaggc ttagttgcta ttttgcaccc ag 42
<210> 70
   <211> 42
   <212> DNA
   <213> Artificial Sequence
<220>
   <223> Synthetic Polynucleotide
<400> 70
   gatattcatt acccaaatca acagttaatg ccccctgcct at 42
<210> 71
   <211> 42
   <212> DNA
   <213> Artificial Sequence
<220>
   <223> Synthetic Polynucleotide
<400> 71
   ctaaaacact catctttgac cctgacctga aagcgtaaga at 42
<210> 72
   <211> 42
   <212> DNA
   <213> Artificial Sequence
<220>
   <223> Synthetic Polynucleotide
<400> 72
   cgaataataa ttttttcacg tatcaccttg ctgaacctca aa 42
<210> 73
   <211> 42
   <212> DNA
   <213> Artificial Sequence
<220>
   <223> Synthetic Polynucleotide
<400> 73
   atgaggaagt ttccattaaa catcctaatt tacgagcatg ta 42
<210> 74
   <211> 42
   <212> DNA
   <213> Artificial Sequence
<220>
   <223> Synthetic Polynucleotide
<400> 74
   ttcgaggtga atttcttaaa cacctcccga cttgcgggag gt 42
<210> 75
   <211> 42
   <212> DNA
   <213> Artificial Sequence
<220>
   <223> Synthetic Polynucleotide
<400> 75
   ggatcgtcac cctcagcagc gacatgaaag tattaagagg ct 42
<210> 76
   <211> 42
   <212> DNA
   <213> Artificial Sequence
<220>
   <223> Synthetic Polynucleotide
<400> 76
   tttcagcgga gtgagaatag atgaatggct attagtcttt aa 42
<210> 77
   <211> 42
   <212> DNA
   <213> Artificial Sequence
<220>
   <223> Synthetic Polynucleotide
<400> 77
   ctacaacgcc tgtagcattc cagtgccacg ctgagagcca gc 42
<210> 78
   <211> 42
   <212> DNA
   <213> Artificial Sequence
<220>
   <223> Synthetic Polynucleotide
<400> 78
   ctccaaaagg agcctttaat tgtctttcct tatcattcca ag 42
<210> 79
   <211> 42
   <212> DNA
   <213> Artificial Sequence
<220>
   <223> Synthetic Polynucleotide
<400> 79
   cagagccacc accctcattt taaggcttat ccggtattct aa 42
<210> 80
   <211> 42
   <212> DNA
   <213> Artificial Sequence
<220>
   <223> Synthetic Polynucleotide
<400> 80
   ccgacaatga caacaaccat ctaggattag cggggttttg ct 42
<210> 81
   <211> 49
   <212> DNA
   <213> Artificial Sequence
<220>
   <223> Synthetic Polynucleotide
<400> 81
   tcgtaaaact agcatgtcaa tatcagtgag gccaccgagt aagaaaaac 49
<210> 82
   <211> 49
   <212> DNA
   <213> Artificial Sequence
<220>
   <223> Synthetic Polynucleotide
<400> 82
   atgatattca accgttctag catattcctg attatcagat gaagagtcc 49
<210> 83
   <211> 49
   <212> DNA
   <213> Artificial Sequence
<220>
   <223> Synthetic Polynucleotide
<400> 83
   ttaaattgta aacgttaata tcggaatcat aattactaga aaaatagcc 49
<210> 84
   <211> 49
   <212> DNA
   <213> Artificial Sequence
<220>
   <223> Synthetic Polynucleotide
<400> 84
   tattttaaat gcaatgcctg atgagcgcta atatcagaga gaatggtgg 49
<210> 85
   <211> 49
   <212> DNA
   <213> Artificial Sequence
<220>
   <223> Synthetic Polynucleotide
<400> 85
   tcaaaaataa ttcgcgtctg gagccaccac cctcagagcc gccggtcca 49
<210> 86
   <211> 49
   <212> DNA
   <213> Artificial Sequence
<220>
   <223> Synthetic Polynucleotide
<400> 86
   ggtagctatt tttgagagat cattaaccgt tgtagcaata ctcggtcca 49
<210> 87
   <211> 49
   <212> DNA
   <213> Artificial Sequence
<220>
   <223> Synthetic Polynucleotide
<400> 87
   atggtcaata acctgtttag cttgcggaac aaagaaacca ccatggtgg 49
<210> 88
   <211> 49
   <212> DNA
   <213> Artificial Sequence
<220>
   <223> Synthetic Polynucleotide
<400> 88
   aaaagggtga gaaaggccgg acgttataca aattcttacc agaatagcc 49
<210> 89
   <211> 49
   <212> DNA
   <213> Artificial Sequence
<220>
   <223> Synthetic Polynucleotide
<400> 89
   aacatccaat aaatcataca ggggagaatt aactgaacac ccagagtcc 49
<210> 90
   <211> 49
   <212> DNA
   <213> Artificial Sequence
<220>
   <223> Synthetic Polynucleotide
<400> 90
   ctttatttca acgcaaggat acgccgccag cattgacagg aggaaaaac 49
<210> 91
   <211> 52
   <212> DNA
   <213> Artificial Sequence
<220>
   <223> Synthetic Polynucleotide
<400> 91
   tcgtaaaact agcatgtcaa tatcagtgag gccaccgagt aagaaaaacc gt 52
<210> 92
   <211> 52
   <212> DNA
   <213> Artificial Sequence
<220>
   <223> Synthetic Polynucleotide
<400> 92
   atgatattca accgttctag catattcctg attatcagat gaagagtcca ct 52
<210> 93
   <211> 52
   <212> DNA
   <213> Artificial Sequence
<220>
   <223> Synthetic Polynucleotide
<400> 93
   ttaaattgta aacgttaata tcggaatcat aattactaga aaaatagccc ga 52
<210> 94
   <211> 52
   <212> DNA
   <213> Artificial Sequence
<220>
   <223> Synthetic Polynucleotide
<400> 94
   tattttaaat gcaatgcctg atgagcgcta atatcagaga gaatggtggt tc 52
<210> 95
   <211> 52
   <212> DNA
   <213> Artificial Sequence
<220>
   <223> Synthetic Polynucleotide
<400> 95
   tcaaaaataa ttcgcgtctg gagccaccac cctcagagcc gccggtccac gc 52
<210> 96
   <211> 52
   <212> DNA
   <213> Artificial Sequence
<220>
   <223> Synthetic Polynucleotide
<400> 96
   ggtagctatt tttgagagat cattaaccgt tgtagcaata ctcggtccac gc 52
<210> 97
   <211> 52
   <212> DNA
   <213> Artificial Sequence
<220>
   <223> Synthetic Polynucleotide
<400> 97
   atggtcaata acctgtttag cttgcggaac aaagaaacca ccatggtggt tc 52
<210> 98
   <211> 52
   <212> DNA
   <213> Artificial Sequence
<220>
   <223> Synthetic Polynucleotide
<400> 98
   aaaagggtga gaaaggccgg acgttataca aattcttacc agaatagccc ga 52
<210> 99
   <211> 52
   <212> DNA
   <213> Artificial Sequence
<220>
   <223> Synthetic Polynucleotide
<400> 99
   aacatccaat aaatcataca ggggagaatt aactgaacac ccagagtcca ct 52
<210> 100
   <211> 52
   <212> DNA
   <213> Artificial Sequence
<220>
   <223> Synthetic Polynucleotide
<400> 100
   ctttatttca acgcaaggat acgccgccag cattgacagg aggaaaaacc gt 52
<210> 101
   <211> 42
   <212> DNA
   <213> Artificial Sequence
<220>
   <223> Synthetic Polynucleotide
<400> 101
   caatattaca taacaatcct ccatttgaat tacctttttt aa 42
<210> 102
   <211> 42
   <212> DNA
   <213> Artificial Sequence
<220>
   <223> Synthetic Polynucleotide
<400> 102
   actgataccg tgcaaaatta tcaaagacaa aagggcgaca tt 42
<210> 103
   <211> 42
   <212> DNA
   <213> Artificial Sequence
<220>
   <223> Synthetic Polynucleotide
<400> 103
   cgtaacgatc taaagttttg taacatcgcc attaaaaata cc 42
<210> 104
   <211> 42
   <212> DNA
   <213> Artificial Sequence
<220>
   <223> Synthetic Polynucleotide
<400> 104
   gaacccatgt accgtaacac tcgcactcat cgagaacaag ca 42
<210> 105
   <211> 42
   <212> DNA
   <213> Artificial Sequence
<220>
   <223> Synthetic Polynucleotide
<400> 105
   taccgccacc ctcagaaccg ccgtcgagag ggttgatata ag 42
<210> 106
   <211> 42
   <212> DNA
   <213> Artificial Sequence
<220>
   <223> Synthetic Polynucleotide
<400> 106
   tcattaaagg tgaattatca cttctgcaat gtgcgagaaa tg 42
<210> 107
   <211> 42
   <212> DNA
   <213> Artificial Sequence
<220>
   <223> Synthetic Polynucleotide
<400> 107
   gggaattaga gccagcaaaa tgtttatgta gatgaaggta ta 42
<210> 108
   <211> 42
   <212> DNA
   <213> Artificial Sequence
<220>
   <223> Synthetic Polynucleotide
<400> 108
   caccgtaatc agtagcgaca ggtttcttgt tgttcgccat cc 42
<210> 109
   <211> 42
   <212> DNA
   <213> Artificial Sequence
<220>
   <223> Synthetic Polynucleotide
<400> 109
   ccttattagc gtttgccatc tgcaacacag caataaaaat gc 42
<210> 110
   <211> 42
   <212> DNA
   <213> Artificial Sequence
<220>
   <223> Synthetic Polynucleotide
<400> 110
   cctcagaacc gccaccctca gccttcctgt agccagcttt ca 42
<210> 111
   <211> 42
   <212> DNA
   <213> Artificial Sequence
<220>
   <223> Synthetic Polynucleotide
<400> 111
   gttgaggcag gtcagacgat tgcataaagc taaatcggtt gt 42
<210> 112
   <211> 42
   <212> DNA
   <213> Artificial Sequence
<220>
   <223> Synthetic Polynucleotide
<400> 112
   atttaccgtt ccagtaagcg taaaaatcag gtctttaccc tg 42
<210> 113
   <211> 42
   <212> DNA
   <213> Artificial Sequence
<220>
   <223> Synthetic Polynucleotide
<400> 113
   gagtaacagt gcccgtataa acgtaacaaa gctgctcatt ca 42
<210> 114
   <211> 42
   <212> DNA
   <213> Artificial Sequence
<220>
   <223> Synthetic Polynucleotide
<400> 114
   gagactcctc aagagaagga tgcccacgca taaccgatat at 42
<210> 115
   <211> 42
   <212> DNA
   <213> Artificial Sequence
<220>
   <223> Synthetic Polynucleotide
<400> 115
   tagcaagcaa atcagatata gcagggatag caagcccaat ag 42
<210> 116
   <211> 42
   <212> DNA
   <213> Artificial Sequence
<220>
   <223> Synthetic Polynucleotide
<400> 116
   gcttctgtaa atcgtcgcta taaacatata gatgattaaa cc 42
<210> 117
   <211> 42
   <212> DNA
   <213> Artificial Sequence
<220>
   <223> Synthetic Polynucleotide
<400> 117
   agtattaaca ccgcctgcaa cacagacagc cctcatagtt ag 42
<210> 118
   <211> 42
   <212> DNA
   <213> Artificial Sequence
<220>
   <223> Synthetic Polynucleotide
<400> 118
   gcactaaatc ggaaccctaa attttgtttt atggagatga ta 42
<210> 119
   <211> 42
   <212> DNA
   <213> Artificial Sequence
<220>
   <223> Synthetic Polynucleotide
<400> 119
   aaagcgaaag gagcgggcgc tctgaatttc gcgtcgtctt ca 42
<210> 120
   <211> 42
   <212> DNA
   <213> Artificial Sequence
<220>
   <223> Synthetic Polynucleotide
<400> 120
   ccgctacagg gcgcgtacta ttttccatga attggtaaca cc 42
<210> 121
   <211> 42
   <212> DNA
   <213> Artificial Sequence
<220>
   <223> Synthetic Polynucleotide
<400> 121
   gaggccgatt aaagggattt tctgcgcaac tgttgggaag gg 42
<210> 122
   <211> 42
   <212> DNA
   <213> Artificial Sequence
<220>
   <223> Synthetic Polynucleotide
<400> 122
   aagagtctgt ccatcacgca atacaaaggc tatcaggtca tt 42
<210> 123
   <211> 42
   <212> DNA
   <213> Artificial Sequence
<220>
   <223> Synthetic Polynucleotide
<400> 123
   caaactatcg gccttgctgg tatatgcaac taaagtacgg tg 42
<210> 124
   <211> 42
   <212> DNA
   <213> Artificial Sequence
<220>
   <223> Synthetic Polynucleotide
<400> 124
   attttgacgc tcaatcgtct gaactaatgc agatacataa cg 42
<210> 125
   <211> 42
   <212> DNA
   <213> Artificial Sequence
<220>
   <223> Synthetic Polynucleotide
<400> 125
   gccaacagag atagaaccct tcccagcgat tataccaagc gc 42
<210> 126
   <211> 42
   <212> DNA
   <213> Artificial Sequence
<220>
   <223> Synthetic Polynucleotide
<400> 126
   tgcgcgaact gatagcccta acgtctttcc agacgttagt aa 42
<210> 127
   <211> 40
   <212> DNA
   <213> Artificial Sequence
<220>
   <223> Synthetic Polynucleotide
<400> 127
   caaagggcga aaaaccaaca gctgattgcc ctgcgccagg 40
<210> 128
   <211> 40
   <212> DNA
   <213> Artificial Sequence
<220>
   <223> Synthetic Polynucleotide
<400> 128
   agtccactat taaagaagag agttgcagca agcaacgcgc 40
<210> 129
   <211> 40
   <212> DNA
   <213> Artificial Sequence
<220>
   <223> Synthetic Polynucleotide
<400> 129
   tagggttgag tgttgtgccc cagcaggcga aaacctgtcg 40
<210> 130
   <211> 32
   <212> DNA
   <213> Artificial Sequence
<220>
   <223> Synthetic Polynucleotide
<400> 130
   aatcccttat aaatcagttc cgaaatcggc aa 32
<210> 131
   <211> 19
   <212> DNA
   <213> Artificial Sequence
<220>
   <223> Synthetic Polynucleotide
<400> 131
   gtgagacggg cgtctatca 19
<210> 132
   <211> 24
   <212> DNA
   <213> Artificial Sequence
<220>
   <223> Synthetic Polynucleotide
<400> 132
   gtggtttttg tttcctgtgt gaaa 24
<210> 133
   <211> 40
   <212> DNA
   <213> Artificial Sequence
<220>
   <223> Synthetic Polynucleotide
<400> 133
   cgtattggtc accgcctggc cctgacgtgg actccaacgt 40
<210> 134
   <211> 24
   <212> DNA
   <213> Artificial Sequence
<220>
   <223> Synthetic Polynucleotide
<400> 134
   ggggagagat tccacacaac atac 24
<210> 135
   <211> 40
   <212> DNA
   <213> Artificial Sequence
<220>
   <223> Synthetic Polynucleotide
<400> 135
   gaatcggccg gtccacgctg gttttccagt ttggaacaag 40
<210> 136
   <211> 24
   <212> DNA
   <213> Artificial Sequence
<220>
   <223> Synthetic Polynucleotide
<400> 136
   tgccagctgt gtaaagcctg gggt 24
<210> 137
   <211> 40
   <212> DNA
   <213> Artificial Sequence
<220>
   <223> Synthetic Polynucleotide
<400> 137
   gtcgggaaat cctgtttgat ggtgaaagaa tagcccgaga 40
<210> 138
   <211> 32
   <212> DNA
   <213> Artificial Sequence
<220>
   <223> Synthetic Polynucleotide
<400> 138
   gttgcgctca ctgcccaact cacattaatt gc 32
<210> 139
   <211> 19
   <212> DNA
   <213> Artificial Sequence
<220>
   <223> Synthetic Polynucleotide
<400> 139
   gtcatagctc ttttcacca 19
<210> 140
   <211> 24
   <212> DNA
   <213> Artificial Sequence
<220>
   <223> Synthetic Polynucleotide
<400> 140
   ttgttatccg ctcacagcgg tttg 24
<210> 141
   <211> 24
   <212> DNA
   <213> Artificial Sequence
<220>
   <223> Synthetic Polynucleotide
<400> 141
   gagccggaag cataaagcat taat 24
<210> 142
   <211> 24
   <212> DNA
   <213> Artificial Sequence
<220>
   <223> Synthetic Polynucleotide
<400> 142
   gcctaatgag tgagctgctt tcca 24
<210> 143
   <211> 42
   <212> DNA
   <213> Artificial Sequence
<220>
   <223> Synthetic Polynucleotide
<400> 143
   aaaatacata cataaaggtg gctattacgg ggttggaggt ca 42
<210> 144
   <211> 42
   <212> DNA
   <213> Artificial Sequence
<220>
   <223> Synthetic Polynucleotide
<400> 144
   tagcaaggcc ggaaacgtca ccgaacaaga cccgttagta ac 42
<210> 145
   <211> 42
   <212> DNA
   <213> Artificial Sequence
<220>
   <223> Synthetic Polynucleotide
<400> 145
   cagactgtag cgcgttttca taacgaagac gcctggtcgt tc 42
<210> 146
   <211> 42
   <212> DNA
   <213> Artificial Sequence
<220>
   <223> Synthetic Polynucleotide
<400> 146
   aaccagagcc accaccggaa caccgtaatg ggataggtca cg 42
<210> 147
   <211> 42
   <212> DNA
   <213> Artificial Sequence
<220>
   <223> Synthetic Polynucleotide
<400> 147
   caccagaacc accaccagag caaaattttt agaaccctca ta 42
<210> 148
   <211> 42
   <212> DNA
   <213> Artificial Sequence
<220>
   <223> Synthetic Polynucleotide
<400> 148
   ataaatcctc attaaagcca gcaaatatcg cgttttaatt cg 42
<210> 149
   <211> 42
   <212> DNA
   <213> Artificial Sequence
<220>
   <223> Synthetic Polynucleotide
<400> 149
   caggagtgta ctggtaataa gtaatttcaa ctttaatcat tg 42
<210> 150
   <211> 42
   <212> DNA
   <213> Artificial Sequence
<220>
   <223> Synthetic Polynucleotide
<400> 150
   ttcggaacct attattctga aaaagacagc atcggaacga gg 42
<210> 151
   <211> 42
   <212> DNA
   <213> Artificial Sequence
<220>
   <223> Synthetic Polynucleotide
<400> 151
   cagtaccagg cggataagtg ccaccctcag aaccgccacc ct 42
<210> 152
   <211> 42
   <212> DNA
   <213> Artificial Sequence
<220>
   <223> Synthetic Polynucleotide
<400> 152
   attcatatgg tttaccagcg ctatcacgag tacggtggaa ac 42
<210> 153
   <211> 42
   <212> DNA
   <213> Artificial Sequence
<220>
   <223> Synthetic Polynucleotide
<400> 153
   agacaccacg gaataagttt atgcagatcc ggtgtcttgt ct 42
<210> 154
   <211> 42
   <212> DNA
   <213> Artificial Sequence
<220>
   <223> Synthetic Polynucleotide
<400> 154
   atgattaaga ctccttatta ctgctaaact ggaaagcaac ga 42
<210> 155
   <211> 42
   <212> DNA
   <213> Artificial Sequence
<220>
   <223> Synthetic Polynucleotide
<400> 155
   ccgaggaaac gcaataataa cgttgccagg aggatctgga ac 42
<210> 156
   <211> 42
   <212> DNA
   <213> Artificial Sequence
<220>
   <223> Synthetic Polynucleotide
<400> 156
   agaaaagtaa gcagatagcc gcagacatca ttgattcagc at 42
<210> 157
   <211> 42
   <212> DNA
   <213> Artificial Sequence
<220>
   <223> Synthetic Polynucleotide
<400> 157
   aacaatgaaa tagcaatagc ttaaccgtgc atctgccagt tt 42
<210> 158
   <211> 42
   <212> DNA
   <213> Artificial Sequence
<220>
   <223> Synthetic Polynucleotide
<400> 158
   taacccacaa gaattgagtt attttaacca ataggaacgc ca 42
<210> 159
   <211> 42
   <212> DNA
   <213> Artificial Sequence
<220>
   <223> Synthetic Polynucleotide
<400> 159
   tgaacaaagt cagagggtaa tgtaatgtgt aggtaaagat tc 42
<210> 160
   <211> 42
   <212> DNA
   <213> Artificial Sequence
<220>
   <223> Synthetic Polynucleotide
<400> 160
   aaacagggaa gcgcattaga cgcaaggcaa agaattagca aa 42
<210> 161
   <211> 42
   <212> DNA
   <213> Artificial Sequence
<220>
   <223> Synthetic Polynucleotide
<400> 161
   tcaaaaatga aaatagcagc cggaagcaaa ctccaacagg tc 42
<210> 162
   <211> 42
   <212> DNA
   <213> Artificial Sequence
<220>
   <223> Synthetic Polynucleotide
<400> 162
   tatttatccc aatccaaata aaaatgcttt aaacagttca ga 42
<210> 163
   <211> 42
   <212> DNA
   <213> Artificial Sequence
<220>
   <223> Synthetic Polynucleotide
<400> 163
   ttccagagcc taatttgcca gaagaactgg ctcattatac ca 42
<210> 164
   <211> 42
   <212> DNA
   <213> Artificial Sequence
<220>
   <223> Synthetic Polynucleotide
<400> 164
   ctacaatttt atcctgaatc tgagtaatct tgacaagaac cg 42
<210> 165
   <211> 42
   <212> DNA
   <213> Artificial Sequence
<220>
   <223> Synthetic Polynucleotide
<400> 165
   tttgaagcct taaatcaaga tttgaggact aaagactttt tc 42
<210> 166
   <211> 42
   <212> DNA
   <213> Artificial Sequence
<220>
   <223> Synthetic Polynucleotide
<400> 166
   gaacgcgagg cgttttagcg aagcttgata ccgatagttg cg 42
<210> 167
   <211> 42
   <212> DNA
   <213> Artificial Sequence
<220>
   <223> Synthetic Polynucleotide
<400> 167
   ccttgaaaac atagcgatag cgagttagag tctgagcaaa aa 42
<210> 168
   <211> 42
   <212> DNA
   <213> Artificial Sequence
<220>
   <223> Synthetic Polynucleotide
<400> 168
   agtcaatagt gaatttatca agtatctgca tatgatgtct ga 42
<210> 169
   <211> 42
   <212> DNA
   <213> Artificial Sequence
<220>
   <223> Synthetic Polynucleotide
<400> 169
   cctttttaac ctccggctta gtgagtatta cgaaggtgtt at 42
<210> 170
   <211> 42
   <212> DNA
   <213> Artificial Sequence
<220>
   <223> Synthetic Polynucleotide
<400> 170
   taaatgctga tgcaaatcca acgaagtgag cgaaattaac tc 42
<210> 171
   <211> 42
   <212> DNA
   <213> Artificial Sequence
<220>
   <223> Synthetic Polynucleotide
<400> 171
   aaaacttttt caaatatatt ttcatgcgta ttaaccaaca gt 42
<210> 172
   <211> 42
   <212> DNA
   <213> Artificial Sequence
<220>
   <223> Synthetic Polynucleotide
<400> 172
   ctaaatttaa tggtttgaaa tgcctcagga agatcgcact cc 42
<210> 173
   <211> 42
   <212> DNA
   <213> Artificial Sequence
<220>
   <223> Synthetic Polynucleotide
<400> 173
   gcgttaaata agaataaaca ctttgttaaa attcgcatta aa 42
<210> 174
   <211> 42
   <212> DNA
   <213> Artificial Sequence
<220>
   <223> Synthetic Polynucleotide
<400> 174
   aagcctgttt agtatcatat ggacagtcaa atcaccatca at 42
<210> 175
   <211> 42
   <212> DNA
   <213> Artificial Sequence
<220>
   <223> Synthetic Polynucleotide
<400> 175
   tataaagcca acgctcaaca gtctactaat agtagtagca tt 42
<210> 176
   <211> 42
   <212> DNA
   <213> Artificial Sequence
<220>
   <223> Synthetic Polynucleotide
<400> 176
   catatttaac aacgccaaca tttgctcctt ttgataagag gt 42
<210> 177
   <211> 42
   <212> DNA
   <213> Artificial Sequence
<220>
   <223> Synthetic Polynucleotide
<400> 177
   tcgagccagt aataagagaa tcaatactgc ggaatcgtca ta 42
<210> 178
   <211> 42
   <212> DNA
   <213> Artificial Sequence
<220>
   <223> Synthetic Polynucleotide
<400> 178
   aagtaattct gtccagacga catctacgtt aataaaacga ac 42
<210> 179
   <211> 42
   <212> DNA
   <213> Artificial Sequence
<220>
   <223> Synthetic Polynucleotide
<400> 179
   ctaatgcaga acgcgcctgt tcggtgtaca gaccaggcgc at 42
<210> 180
   <211> 42
   <212> DNA
   <213> Artificial Sequence
<220>
   <223> Synthetic Polynucleotide
<400> 180
   gaacaagaaa aataatatcc cgggtaaaat acgtaatgcc ac 42
<210> 181
   <211> 42
   <212> DNA
   <213> Artificial Sequence
<220>
   <223> Synthetic Polynucleotide
<400> 181
   gaaaccaatc aataatcggc tgtatcggtt tatcagcttg ct 42
<210> 182
   <211> 42
   <212> DNA
   <213> Artificial Sequence
<220>
   <223> Synthetic Polynucleotide
<400> 182
   aacgggtatt aaaccaagta cgagtttcgt caccagtaca aa 42
<210> 183
   <211> 42
   <212> DNA
   <213> Artificial Sequence
<220>
   <223> Synthetic Polynucleotide
<400> 183
   attaattaca tttaacaatt tgcactcgcg gggatttatt tt 42
<210> 184
   <211> 42
   <212> DNA
   <213> Artificial Sequence
<220>
   <223> Synthetic Polynucleotide
<400> 184
   ctgagcaaaa gaagatgatg agaaacgaca tacattgcaa gg 42
<210> 185
   <211> 42
   <212> DNA
   <213> Artificial Sequence
<220>
   <223> Synthetic Polynucleotide
<400> 185
   agttacaaaa tcgcgcagag gagagtgaga tcggttttgt aa 42
<210> 186
   <211> 42
   <212> DNA
   <213> Artificial Sequence
<220>
   <223> Synthetic Polynucleotide
<400> 186
   gggagaaaca ataacggatt ctgttgagct tgaaacagca aa 42
<210> 187
   <211> 42
   <212> DNA
   <213> Artificial Sequence
<220>
   <223> Synthetic Polynucleotide
<400> 187
   tttaacgtca gatgaatata cagagcaggc aatgcatgac ga 42
<210> 188
   <211> 42
   <212> DNA
   <213> Artificial Sequence
<220>
   <223> Synthetic Polynucleotide
<400> 188
   tgcacgtaaa acagaaataa aaaaacgacg gccagtgcca ag 42
<210> 189
   <211> 42
   <212> DNA
   <213> Artificial Sequence
<220>
   <223> Synthetic Polynucleotide
<400> 189
   ctgaataatg gaagggttag atctggtgcc ggaaaccagg ca 42
<210> 190
   <211> 42
   <212> DNA
   <213> Artificial Sequence
<220>
   <223> Synthetic Polynucleotide
<400> 190
   tggcaattca tcaatataat cgaagattgt ataagcaaat at 42
<210> 191
   <211> 42
   <212> DNA
   <213> Artificial Sequence
<220>
   <223> Synthetic Polynucleotide
<400> 191
   agaaggagcg gaattatcat ctgataaatt aatgccggag ag 42
<210> 192
   <211> 42
   <212> DNA
   <213> Artificial Sequence
<220>
   <223> Synthetic Polynucleotide
<400> 192
   agtttgagta acattatcat ttatattttc atttggggcg cg 42
<210> 193
   <211> 42
   <212> DNA
   <213> Artificial Sequence
<220>
   <223> Synthetic Polynucleotide
<400> 193
   aactcgtatt aaatcctttg cgcttaattg ctgaatataa tg 42
<210> 194
   <211> 42
   <212> DNA
   <213> Artificial Sequence
<220>
   <223> Synthetic Polynucleotide
<400> 194
   atttgaggat ttagaagtat tgccagaggg ggtaatagta aa 42
<210> 195
   <211> 42
   <212> DNA
   <213> Artificial Sequence
<220>
   <223> Synthetic Polynucleotide
<400> 195
   agcactaaca actaatagat tggtagaaag attcatcagt tg 42
<210> 196
   <211> 42
   <212> DNA
   <213> Artificial Sequence
<220>
   <223> Synthetic Polynucleotide
<400> 196
   agttgaaagg aattgaggaa gtaagggaac cgaactgacc aa 42
<210> 197
   <211> 42
   <212> DNA
   <213> Artificial Sequence
<220>
   <223> Synthetic Polynucleotide
<400> 197
   tatcaaaccc tcaatcaata tcgaaagagg caaaagaata ca 42
<210> 198
   <211> 42
   <212> DNA
   <213> Artificial Sequence
<220>
   <223> Synthetic Polynucleotide
<400> 198
   agcaaatgaa aaatctaaag ctgaaaatct ccaaaaaaaa gg 42
<210> 199
   <211> 42
   <212> DNA
   <213> Artificial Sequence
<220>
   <223> Synthetic Polynucleotide
<400> 199
   tgacggggaa agccggcgaa ccttactgtt tctttacata aa 42
<210> 200
   <211> 42
   <212> DNA
   <213> Artificial Sequence
<220>
   <223> Synthetic Polynucleotide
<400> 200
   gtcacgctgc gcgtaaccac cccaggagaa cgaggatatt gc 42
<210> 201
   <211> 42
   <212> DNA
   <213> Artificial Sequence
<220>
   <223> Synthetic Polynucleotide
<400> 201
   taacgtgctt tcctcgttag attaagttgg gtaacgccag gg 42
<210> 202
   <211> 42
   <212> DNA
   <213> Artificial Sequence
<220>
   <223> Synthetic Polynucleotide
<400> 202
   atcctgagaa gtgtttttat acatatgtac cccggttgat aa 42
<210> 203
   <211> 42
   <212> DNA
   <213> Artificial Sequence
<220>
   <223> Synthetic Polynucleotide
<400> 203
   tctttgatta gtaataacat caccattaga tacatttcgc aa 42
<210> 204
   <211> 42
   <212> DNA
   <213> Artificial Sequence
<220>
   <223> Synthetic Polynucleotide
<400> 204
   gccagccatt gcaacaggaa aataaaaacc aaaatagcga ga 42
<210> 205
   <211> 42
   <212> DNA
   <213> Artificial Sequence
<220>
   <223> Synthetic Polynucleotide
<400> 205
   agattcacca gtcacacgac cctccatgtt acttagccgg aa 42
<210> 206
   <211> 42
   <212> DNA
   <213> Artificial Sequence
<220>
   <223> Synthetic Polynucleotide
<400> 206
   acgtggcaca gacaatattt taaggaacaa ctaaaggaat tg 42
<210> 207
   <211> 49
   <212> DNA
   <213> Artificial Sequence
<220>
   <223> Synthetic Polynucleotide
<400> 207
   atctgaactc gctacggcgg ggggagcccc cgatttagag ctcggtcca 49
<210> 208
   <211> 49
   <212> DNA
   <213> Artificial Sequence
<220>
   <223> Synthetic Polynucleotide
<400> 208
   cattgctgat accgtttagc taacaaacat caagaaaaca aaatggtgg 49
<210> 209
   <211> 49
   <212> DNA
   <213> Artificial Sequence
<220>
   <223> Synthetic Polynucleotide
<400> 209
   gatacttgcc ctctctgtac ataattaatt ttcccttaga ataatagcc 49
<210> 210
   <211> 49
   <212> DNA
   <213> Artificial Sequence
<220>
   <223> Synthetic Polynucleotide
<400> 210
   gattgggcgt tatcaatgtt gttttgtcac aatcaataga aaagagtcc 49
<210> 211
   <211> 49
   <212> DNA
   <213> Artificial Sequence
<220>
   <223> Synthetic Polynucleotide
<400> 211
   tctaatgaag acaaatcccc acgtcaccga cttgagccat ttgaaaaac 49
<210> 212
   <211> 49
   <212> DNA
   <213> Artificial Sequence
<220>
   <223> Synthetic Polynucleotide
<400> 212
   aaacatcggg ttgagtatta tgtggcgaga aaggaaggga aggaaaaac 49
<210> 213
   <211> 49
   <212> DNA
   <213> Artificial Sequence
<220>
   <223> Synthetic Polynucleotide
<400> 213
   cgctggcatt cgcatcaaag gcgaattatt catttcaatt acagagtcc 49
<210> 214
   <211> 49
   <212> DNA
   <213> Artificial Sequence
<220>
   <223> Synthetic Polynucleotide
<400> 214
   agtttataaa tgagtatcaa tttagattaa gacgctgaga agaatagcc 49
<210> 215
   <211> 49
   <212> DNA
   <213> Artificial Sequence
<220>
   <223> Synthetic Polynucleotide
<400> 215
   tatcgacatc attacgcatc gcaacatata aaagaaacgc aaatggtgg 49
<210> 216
   <211> 49
   <212> DNA
   <213> Artificial Sequence
<220>
   <223> Synthetic Polynucleotide
<400> 216
   ccatgcagac atcacgaagg tcaccagtag caccattacc atcggtcca 49
<210> 217
   <211> 49
   <212> DNA
   <213> Artificial Sequence
<220>
   <223> Synthetic Polynucleotide
<400> 217
   aagataacgc ttgtgaaaat gagggcgctg gcaagtgtag cgcggtcca 49
<210> 218
   <211> 49
   <212> DNA
   <213> Artificial Sequence
<220>
   <223> Synthetic Polynucleotide
<400> 218
   gctaacagta gggaaactgc ggcctgattg ctttgaatac caatggtgg 49
<210> 219
   <211> 49
   <212> DNA
   <213> Artificial Sequence
<220>
   <223> Synthetic Polynucleotide
<400> 219
   atgggttcag gatgcaggtg aaatcatagg tctgagagac taaatagcc 49
<210> 220
   <211> 49
   <212> DNA
   <213> Artificial Sequence
<220>
   <223> Synthetic Polynucleotide
<400> 220
   ctcggatggg agtaagcgta tgcagtatgt tagcaaacgt agagagtcc 49
<210> 221
   <211> 49
   <212> DNA
   <213> Artificial Sequence
<220>
   <223> Synthetic Polynucleotide
<400> 221
   agagtttctg cggcagttaa tcaatgaaac catcgatagc aggaaaaac 49
<210> 222
   <211> 49
   <212> DNA
   <213> Artificial Sequence
<220>
   <223> Synthetic Polynucleotide
<400> 222
   gcaatacatc aaacgccgcg aacacccgcc gcgcttaatg cggaaaaac 49
<210> 223
   <211> 49
   <212> DNA
   <213> Artificial Sequence
<220>
   <223> Synthetic Polynucleotide
<400> 223
   tcaggcactg cgtgaagcgg cagtaacagt accttttaca tcagagtcc 49
<210> 224
   <211> 49
   <212> DNA
   <213> Artificial Sequence
<220>
   <223> Synthetic Polynucleotide
<400> 224
   atcaaaactc aacgagcagc ggttgggtta tataactata tgaatagcc 49
<210> 225
   <211> 49
   <212> DNA
   <213> Artificial Sequence
<220>
   <223> Synthetic Polynucleotide
<400> 225
   agggttgtcg gacttgtgca aggaataccc aaaagaactg gcatggtgg 49
<210> 226
   <211> 49
   <212> DNA
   <213> Artificial Sequence
<220>
   <223> Synthetic Polynucleotide
<400> 226
   agtccgtgaa gacggaaacc aaatcaagtt tgcctttagc gtcggtcca 49
<210> 227
   <211> 49
   <212> DNA
   <213> Artificial Sequence
<220>
   <223> Synthetic Polynucleotide
<400> 227
   ctggggattt gacgcagacc tggttgcttt gacgagcacg tacggtcca 49
<210> 228
   <211> 49
   <212> DNA
   <213> Artificial Sequence
<220>
   <223> Synthetic Polynucleotide
<400> 228
   ttttcccagt cacgacgttg tgaaattgcg tagattttca ggatggtgg 49
<210> 229
   <211> 49
   <212> DNA
   <213> Artificial Sequence
<220>
   <223> Synthetic Polynucleotide
<400> 229
   ttatcagtaa acagagaggt ttcgcaagac aaagaacgcg agaatagcc 49
<210> 230
   <211> 49
   <212> DNA
   <213> Artificial Sequence
<220>
   <223> Synthetic Polynucleotide
<400> 230
   tcagggatta atgaaagatg gaacaaagtt accagaagga aaagagtcc 49
<210> 231
   <211> 49
   <212> DNA
   <213> Artificial Sequence
<220>
   <223> Synthetic Polynucleotide
<400> 231
   agtgtggcga tccgatagat gcggcatttt cggtcatagc ccgaaaaac 49
<210> 232
   <211> 49
   <212> DNA
   <213> Artificial Sequence
<220>
   <223> Synthetic Polynucleotide
<400> 232
   gggggatgtg ctgcaaggcg aatcagagcg ggagctaaac aggaaaaac 49
<210> 233
   <211> 49
   <212> DNA
   <213> Artificial Sequence
<220>
   <223> Synthetic Polynucleotide
<400> 233
   agccagcttt ccggcaccgc tacctaccat atcaaaatta ttagagtcc 49
<210> 234
   <211> 49
   <212> DNA
   <213> Artificial Sequence
<220>
   <223> Synthetic Polynucleotide
<400> 234
   ctttattatt cgcattcacc ctagttaatt tcatcttctg acaatagcc 49
<210> 235
   <211> 49
   <212> DNA
   <213> Artificial Sequence
<220>
   <223> Synthetic Polynucleotide
<400> 235
   ttggtgtaga tgggcgcatc gatcttaccg aagccctttt taatggtgg 49
<210> 236
   <211> 49
   <212> DNA
   <213> Artificial Sequence
<220>
   <223> Synthetic Polynucleotide
<400> 236
   cagaaataga agaattacag ctttcataat caaaatcacc ggcggtcca 49
<210> 237
   <211> 49
   <212> DNA
   <213> Artificial Sequence
<220>
   <223> Synthetic Polynucleotide
<400> 237
   aagcgccatt cgccattcag gagacaggaa cggtacgcca gacggtcca 49
<210> 238
   <211> 49
   <212> DNA
   <213> Artificial Sequence
<220>
   <223> Synthetic Polynucleotide
<400> 238
   tcagaaaagc cccaaaaaca gctgattgtt tggattatac ttatggtgg 49
<210> 239
   <211> 49
   <212> DNA
   <213> Artificial Sequence
<220>
   <223> Synthetic Polynucleotide
<400> 239
   gaggggacga cgacagtatc gaccgaccgt gtgataaata agaatagcc 49
<210> 240
   <211> 49
   <212> DNA
   <213> Artificial Sequence
<220>
   <223> Synthetic Polynucleotide
<400> 240
   tttttgttaa atcagctcat tagcccaata ataagagcaa gaagagtcc 49
<210> 241
   <211> 49
   <212> DNA
   <213> Artificial Sequence
<220>
   <223> Synthetic Polynucleotide
<400> 241
   gtgggaacaa acggcggatt gcgcctccct cagagccgcc acgaaaaac 49
<210> 242
   <211> 49
   <212> DNA
   <213> Artificial Sequence
<220>
   <223> Synthetic Polynucleotide
<400> 242
   cgaacgagta gatttagttt gacttgcctg agtagaagaa ctgaaaaac 49
<210> 243
   <211> 49
   <212> DNA
   <213> Artificial Sequence
<220>
   <223> Synthetic Polynucleotide
<400> 243
   catttttgcg gatggcttag accgaacgtt attaatttta aaagagtcc 49
<210> 244
   <211> 49
   <212> DNA
   <213> Artificial Sequence
<220>
   <223> Synthetic Polynucleotide
<400> 244
   agctgaaaag gtggcatcaa ttagggctta attgagaatc gcaatagcc 49
<210> 245
   <211> 49
   <212> DNA
   <213> Artificial Sequence
<220>
   <223> Synthetic Polynucleotide
<400> 245
   agcttcaaag cgaaccagac ctttacagag agaataacat aaatggtgg 49
<210> 246
   <211> 49
   <212> DNA
   <213> Artificial Sequence
<220>
   <223> Synthetic Polynucleotide
<400> 246
   attaagcaat aaagcctcag aggccttgat attcacaaac aacggtcca 49
<210> 247
   <211> 49
   <212> DNA
   <213> Artificial Sequence
<220>
   <223> Synthetic Polynucleotide
<400> 247
   ctgtagctca acatgtttta aaatatccag aacaatatta cccggtcca 49
<210> 248
   <211> 49
   <212> DNA
   <213> Artificial Sequence
<220>
   <223> Synthetic Polynucleotide
<400> 248
   ggcttttgca aaagaagttt tagactttac aaacaattcg acatggtgg 49
<210> 249
   <211> 49
   <212> DNA
   <213> Artificial Sequence
<220>
   <223> Synthetic Polynucleotide
<400> 249
   aggattagag agtaccttta agtaatttag gcagaggcat ttaatagcc 49
<210> 250
   <211> 49
   <212> DNA
   <213> Artificial Sequence
<220>
   <223> Synthetic Polynucleotide
<400> 250
   aatattcatt gaatccccct cgaaacgatt ttttgtttaa cgagagtcc 49
<210> 251
   <211> 49
   <212> DNA
   <213> Artificial Sequence
<220>
   <223> Synthetic Polynucleotide
<400> 251
   aagaggaagc ccgaaagact taatggaaag cgcagtctct gagaaaaac 49
<210> 252
   <211> 49
   <212> DNA
   <213> Artificial Sequence
<220>
   <223> Synthetic Polynucleotide
<400> 252
   accctcgttt accagacgac gaacgctcat ggaaatacct acgaaaaac 49
<210> 253
   <211> 49
   <212> DNA
   <213> Artificial Sequence
<220>
   <223> Synthetic Polynucleotide
<400> 253
   taacggaaca acattattac aagagccgtc aatagataat acagagtcc 49
<210> 254
   <211> 49
   <212> DNA
   <213> Artificial Sequence
<220>
   <223> Synthetic Polynucleotide
<400> 254
   atgtttagac tggatagcgt cataaagtac cgacaaaagg taaatagcc 49
<210> 255
   <211> 49
   <212> DNA
   <213> Artificial Sequence
<220>
   <223> Synthetic Polynucleotide
<400> 255
   tgaattacct tatgcgattt tttacaaaat aaacagccat atatggtgg 49
<210> 256
   <211> 49
   <212> DNA
   <213> Artificial Sequence
<220>
   <223> Synthetic Polynucleotide
<400> 256
   aaacgagaat gaccataaat ccatacatgg cttttgatga tacggtcca 49
<210> 257
   <211> 49
   <212> DNA
   <213> Artificial Sequence
<220>
   <223> Synthetic Polynucleotide
<400> 257
   agatttagga ataccacatt caaatggatt atttacattg gccggtcca 49
<210> 258
   <211> 49
   <212> DNA
   <213> Artificial Sequence
<220>
   <223> Synthetic Polynucleotide
<400> 258
   cgaggcgcag acggtcaatc agttatctaa aatatcttta ggatggtgg 49
<210> 259
   <211> 49
   <212> DNA
   <213> Artificial Sequence
<220>
   <223> Synthetic Polynucleotide
<400> 259
   gtcaggacgt tgggaagaaa agacaataaa caacatgttc agaatagcc 49
<210> 260
   <211> 49
   <212> DNA
   <213> Artificial Sequence
<220>
   <223> Synthetic Polynucleotide
<400> 260
   aggctggctg accttcatca ataccaacgc taacgagcgt ctagagtcc 49
<210> 261
   <211> 49
   <212> DNA
   <213> Artificial Sequence
<220>
   <223> Synthetic Polynucleotide
<400> 261
   taaattgggc ttgagatggt tttttaacgg ggtcagtgcc ttgaaaaac 49
<210> 262
   <211> 49
   <212> DNA
   <213> Artificial Sequence
<220>
   <223> Synthetic Polynucleotide
<400> 262
   tgtgtcgaaa tccgcgacct gagtaataaa agggacattc tggaaaaac 49
<210> 263
   <211> 49
   <212> DNA
   <213> Artificial Sequence
<220>
   <223> Synthetic Polynucleotide
<400> 263
   tacgaaggca ccaacctaaa actggtcagt tggcaaatca acagagtcc 49
<210> 264
   <211> 49
   <212> DNA
   <213> Artificial Sequence
<220>
   <223> Synthetic Polynucleotide
<400> 264
   ctttgaaaga ggacagatga atatcaacaa tagataagtc ctaatagcc 49
<210> 265
   <211> 49
   <212> DNA
   <213> Artificial Sequence
<220>
   <223> Synthetic Polynucleotide
<400> 265
   gtagcaacgg ctacagaggc ttagttgcta ttttgcaccc agatggtgg 49
<210> 266
   <211> 49
   <212> DNA
   <213> Artificial Sequence
<220>
   <223> Synthetic Polynucleotide
<400> 266
   gatattcatt acccaaatca acagttaatg ccccctgcct atcggtcca 49
<210> 267
   <211> 49
   <212> DNA
   <213> Artificial Sequence
<220>
   <223> Synthetic Polynucleotide
<400> 267
   ctaaaacact catctttgac cctgacctga aagcgtaaga atcggtcca 49
<210> 268
   <211> 49
   <212> DNA
   <213> Artificial Sequence
<220>
   <223> Synthetic Polynucleotide
<400> 268
   cgaataataa ttttttcacg tatcaccttg ctgaacctca aaatggtgg 49
<210> 269
   <211> 49
   <212> DNA
   <213> Artificial Sequence
<220>
   <223> Synthetic Polynucleotide
<400> 269
   atgaggaagt ttccattaaa catcctaatt tacgagcatg taaatagcc 49
<210> 270
   <211> 49
   <212> DNA
   <213> Artificial Sequence
<220>
   <223> Synthetic Polynucleotide
<400> 270
   ttcgaggtga atttcttaaa cacctcccga cttgcgggag gtagagtcc 49
<210> 271
   <211> 49
   <212> DNA
   <213> Artificial Sequence
<220>
   <223> Synthetic Polynucleotide
<400> 271
   ggatcgtcac cctcagcagc gacatgaaag tattaagagg ctgaaaaac 49
<210> 272
   <211> 49
   <212> DNA
   <213> Artificial Sequence
<220>
   <223> Synthetic Polynucleotide
<400> 272
   tttcagcgga gtgagaatag atgaatggct attagtcttt aagaaaaac 49
<210> 273
   <211> 49
   <212> DNA
   <213> Artificial Sequence
<220>
   <223> Synthetic Polynucleotide
<400> 273
   ctacaacgcc tgtagcattc cagtgccacg ctgagagcca gcagagtcc 49
<210> 274
   <211> 49
   <212> DNA
   <213> Artificial Sequence
<220>
   <223> Synthetic Polynucleotide
<400> 274
   ctccaaaagg agcctttaat tgtctttcct tatcattcca agaatagcc 49
<210> 275
   <211> 49
   <212> DNA
   <213> Artificial Sequence
<220>
   <223> Synthetic Polynucleotide
<400> 275
   cagagccacc accctcattt taaggcttat ccggtattct aaatggtgg 49
<210> 276
   <211> 49
   <212> DNA
   <213> Artificial Sequence
<220>
   <223> Synthetic Polynucleotide
<400> 276
   ccgacaatga caacaaccat ctaggattag cggggttttg ctcggtcca 49
<210> 277
   <211> 52
   <212> DNA
   <213> Artificial Sequence
<220>
   <223> Synthetic Polynucleotide
<400> 277
   atctgaactc gctacggcgg ggggagcccc cgatttagag ctcggtccac gc 52
<210> 278
   <211> 52
   <212> DNA
   <213> Artificial Sequence
<220>
   <223> Synthetic Polynucleotide
<400> 278
   cattgctgat accgtttagc taacaaacat caagaaaaca aaatggtggt tc 52
<210> 279
   <211> 52
   <212> DNA
   <213> Artificial Sequence
<220>
   <223> Synthetic Polynucleotide
<400> 279
   gatacttgcc ctctctgtac ataattaatt ttcccttaga ataatagccc ga 52
<210> 280
   <211> 52
   <212> DNA
   <213> Artificial Sequence
<220>
   <223> Synthetic Polynucleotide
<400> 280
   gattgggcgt tatcaatgtt gttttgtcac aatcaataga aaagagtcca ct 52
<210> 281
   <211> 52
   <212> DNA
   <213> Artificial Sequence
<220>
   <223> Synthetic Polynucleotide
<400> 281
   tctaatgaag acaaatcccc acgtcaccga cttgagccat ttgaaaaacc gt 52
<210> 282
   <211> 52
   <212> DNA
   <213> Artificial Sequence
<220>
   <223> Synthetic Polynucleotide
<400> 282
   aaacatcggg ttgagtatta tgtggcgaga aaggaaggga aggaaaaacc gt 52
<210> 283
   <211> 52
   <212> DNA
   <213> Artificial Sequence
<220>
   <223> Synthetic Polynucleotide
<400> 283
   cgctggcatt cgcatcaaag gcgaattatt catttcaatt acagagtcca ct 52
<210> 284
   <211> 52
   <212> DNA
   <213> Artificial Sequence
<220>
   <223> Synthetic Polynucleotide
<400> 284
   agtttataaa tgagtatcaa tttagattaa gacgctgaga agaatagccc ga 52
<210> 285
   <211> 52
   <212> DNA
   <213> Artificial Sequence
<220>
   <223> Synthetic Polynucleotide
<400> 285
   tatcgacatc attacgcatc gcaacatata aaagaaacgc aaatggtggt tc 52
<210> 286
   <211> 52
   <212> DNA
   <213> Artificial Sequence
<220>
   <223> Synthetic Polynucleotide
<400> 286
   ccatgcagac atcacgaagg tcaccagtag caccattacc atcggtccac gc 52
<210> 287
   <211> 52
   <212> DNA
   <213> Artificial Sequence
<220>
   <223> Synthetic Polynucleotide
<400> 287
   aagataacgc ttgtgaaaat gagggcgctg gcaagtgtag cgcggtccac gc 52
<210> 288
   <211> 52
   <212> DNA
   <213> Artificial Sequence
<220>
   <223> Synthetic Polynucleotide
<400> 288
   gctaacagta gggaaactgc ggcctgattg ctttgaatac caatggtggt tc 52
<210> 289
   <211> 52
   <212> DNA
   <213> Artificial Sequence
<220>
   <223> Synthetic Polynucleotide
<400> 289
   atgggttcag gatgcaggtg aaatcatagg tctgagagac taaatagccc ga 52
<210> 290
   <211> 52
   <212> DNA
   <213> Artificial Sequence
<220>
   <223> Synthetic Polynucleotide
<400> 290
   ctcggatggg agtaagcgta tgcagtatgt tagcaaacgt agagagtcca ct 52
<210> 291
   <211> 52
   <212> DNA
   <213> Artificial Sequence
<220>
   <223> Synthetic Polynucleotide
<400> 291
   agagtttctg cggcagttaa tcaatgaaac catcgatagc aggaaaaacc gt 52
<210> 292
   <211> 52
   <212> DNA
   <213> Artificial Sequence
<220>
   <223> Synthetic Polynucleotide
<400> 292
   gcaatacatc aaacgccgcg aacacccgcc gcgcttaatg cggaaaaacc gt 52
<210> 293
   <211> 52
   <212> DNA
   <213> Artificial Sequence
<220>
   <223> Synthetic Polynucleotide
<400> 293
   tcaggcactg cgtgaagcgg cagtaacagt accttttaca tcagagtcca ct 52
<210> 294
   <211> 52
   <212> DNA
   <213> Artificial Sequence
<220>
   <223> Synthetic Polynucleotide
<400> 294
   atcaaaactc aacgagcagc ggttgggtta tataactata tgaatagccc ga 52
<210> 295
   <211> 52
   <212> DNA
   <213> Artificial Sequence
<220>
   <223> Synthetic Polynucleotide
<400> 295
   agggttgtcg gacttgtgca aggaataccc aaaagaactg gcatggtggt tc 52
<210> 296
   <211> 52
   <212> DNA
   <213> Artificial Sequence
<220>
   <223> Synthetic Polynucleotide
<400> 296
   agtccgtgaa gacggaaacc aaatcaagtt tgcctttagc gtcggtccac gc 52
<210> 297
   <211> 52
   <212> DNA
   <213> Artificial Sequence
<220>
   <223> Synthetic Polynucleotide
<400> 297
   ctggggattt gacgcagacc tggttgcttt gacgagcacg tacggtccac gc 52
<210> 298
   <211> 52
   <212> DNA
   <213> Artificial Sequence
<220>
   <223> Synthetic Polynucleotide
<400> 298
   ttttcccagt cacgacgttg tgaaattgcg tagattttca ggatggtggt tc 52
<210> 299
   <211> 52
   <212> DNA
   <213> Artificial Sequence
<220>
   <223> Synthetic Polynucleotide
<400> 299
   ttatcagtaa acagagaggt ttcgcaagac aaagaacgcg agaatagccc ga 52
<210> 300
   <211> 52
   <212> DNA
   <213> Artificial Sequence
<220>
   <223> Synthetic Polynucleotide
<400> 300
   tcagggatta atgaaagatg gaacaaagtt accagaagga aaagagtcca ct 52
<210> 301
   <211> 52
   <212> DNA
   <213> Artificial Sequence
<220>
   <223> Synthetic Polynucleotide
<400> 301
   agtgtggcga tccgatagat gcggcatttt cggtcatagc ccgaaaaacc gt 52
<210> 302
   <211> 52
   <212> DNA
   <213> Artificial Sequence
<220>
   <223> Synthetic Polynucleotide
<400> 302
   gggggatgtg ctgcaaggcg aatcagagcg ggagctaaac aggaaaaacc gt 52
<210> 303
   <211> 52
   <212> DNA
   <213> Artificial Sequence
<220>
   <223> Synthetic Polynucleotide
<400> 303
   agccagcttt ccggcaccgc tacctaccat atcaaaatta ttagagtcca ct 52
<210> 304
   <211> 52
   <212> DNA
   <213> Artificial Sequence
<220>
   <223> Synthetic Polynucleotide
<400> 304
   ctttattatt cgcattcacc ctagttaatt tcatcttctg acaatagccc ga 52
<210> 305
   <211> 52
   <212> DNA
   <213> Artificial Sequence
<220>
   <223> Synthetic Polynucleotide
<400> 305
   ttggtgtaga tgggcgcatc gatcttaccg aagccctttt taatggtggt tc 52
<210> 306
   <211> 52
   <212> DNA
   <213> Artificial Sequence
<220>
   <223> Synthetic Polynucleotide
<400> 306
   cagaaataga agaattacag ctttcataat caaaatcacc ggcggtccac gc 52
<210> 307
   <211> 52
   <212> DNA
   <213> Artificial Sequence
<220>
   <223> Synthetic Polynucleotide
<400> 307
   aagcgccatt cgccattcag gagacaggaa cggtacgcca gacggtccac gc 52
<210> 308
   <211> 52
   <212> DNA
   <213> Artificial Sequence
<220>
   <223> Synthetic Polynucleotide
<400> 308
   tcagaaaagc cccaaaaaca gctgattgtt tggattatac ttatggtggt tc 52
<210> 309
   <211> 52
   <212> DNA
   <213> Artificial Sequence
<220>
   <223> Synthetic Polynucleotide
<400> 309
   gaggggacga cgacagtatc gaccgaccgt gtgataaata agaatagccc ga 52
<210> 310
   <211> 52
   <212> DNA
   <213> Artificial Sequence
<220>
   <223> Synthetic Polynucleotide
<400> 310
   tttttgttaa atcagctcat tagcccaata ataagagcaa gaagagtcca ct 52
<210> 311
   <211> 52
   <212> DNA
   <213> Artificial Sequence
<220>
   <223> Synthetic Polynucleotide
<400> 311
   gtgggaacaa acggcggatt gcgcctccct cagagccgcc acgaaaaacc gt 52
<210> 312
   <211> 52
   <212> DNA
   <213> Artificial Sequence
<220>
   <223> Synthetic Polynucleotide
<400> 312
   cgaacgagta gatttagttt gacttgcctg agtagaagaa ctgaaaaacc gt 52
<210> 313
   <211> 52
   <212> DNA
   <213> Artificial Sequence
<220>
   <223> Synthetic Polynucleotide
<400> 313
   catttttgcg gatggcttag accgaacgtt attaatttta aaagagtcca ct 52
<210> 314
   <211> 52
   <212> DNA
   <213> Artificial Sequence
<220>
   <223> Synthetic Polynucleotide
<400> 314
   agctgaaaag gtggcatcaa ttagggctta attgagaatc gcaatagccc ga 52
<210> 315
   <211> 52
   <212> DNA
   <213> Artificial Sequence
<220>
   <223> Synthetic Polynucleotide
<400> 315
   agcttcaaag cgaaccagac ctttacagag agaataacat aaatggtggt tc 52
<210> 316
   <211> 52
   <212> DNA
   <213> Artificial Sequence
<220>
   <223> Synthetic Polynucleotide
<400> 316
   attaagcaat aaagcctcag aggccttgat attcacaaac aacggtccac gc 52
<210> 317
   <211> 52
   <212> DNA
   <213> Artificial Sequence
<220>
   <223> Synthetic Polynucleotide
<400> 317
   ctgtagctca acatgtttta aaatatccag aacaatatta cccggtccac gc 52
<210> 318
   <211> 52
   <212> DNA
   <213> Artificial Sequence
<220>
   <223> Synthetic Polynucleotide
<400> 318
   ggcttttgca aaagaagttt tagactttac aaacaattcg acatggtggt tc 52
<210> 319
   <211> 52
   <212> DNA
   <213> Artificial Sequence
<220>
   <223> Synthetic Polynucleotide
<400> 319
   aggattagag agtaccttta agtaatttag gcagaggcat ttaatagccc ga 52
<210> 320
   <211> 52
   <212> DNA
   <213> Artificial Sequence
<220>
   <223> Synthetic Polynucleotide
<400> 320
   aatattcatt gaatccccct cgaaacgatt ttttgtttaa cgagagtcca ct 52
<210> 321
   <211> 52
   <212> DNA
   <213> Artificial Sequence
<220>
   <223> Synthetic Polynucleotide
<400> 321
   aagaggaagc ccgaaagact taatggaaag cgcagtctct gagaaaaacc gt 52
<210> 322
   <211> 52
   <212> DNA
   <213> Artificial Sequence
<220>
   <223> Synthetic Polynucleotide
<400> 322
   accctcgttt accagacgac gaacgctcat ggaaatacct acgaaaaacc gt 52
<210> 323
   <211> 52
   <212> DNA
   <213> Artificial Sequence
<220>
   <223> Synthetic Polynucleotide
<400> 323
   taacggaaca acattattac aagagccgtc aatagataat acagagtcca ct 52
<210> 324
   <211> 52
   <212> DNA
   <213> Artificial Sequence
<220>
   <223> Synthetic Polynucleotide
<400> 324
   atgtttagac tggatagcgt cataaagtac cgacaaaagg taaatagccc ga 52
<210> 325
   <211> 52
   <212> DNA
   <213> Artificial Sequence
<220>
   <223> Synthetic Polynucleotide
<400> 325
   tgaattacct tatgcgattt tttacaaaat aaacagccat atatggtggt tc 52
<210> 326
   <211> 52
   <212> DNA
   <213> Artificial Sequence
<220>
   <223> Synthetic Polynucleotide
<400> 326
   aaacgagaat gaccataaat ccatacatgg cttttgatga tacggtccac gc 52
<210> 327
   <211> 52
   <212> DNA
   <213> Artificial Sequence
<220>
   <223> Synthetic Polynucleotide
<400> 327
   agatttagga ataccacatt caaatggatt atttacattg gccggtccac gc 52
<210> 328
   <211> 52
   <212> DNA
   <213> Artificial Sequence
<220>
   <223> Synthetic Polynucleotide
<400> 328
   cgaggcgcag acggtcaatc agttatctaa aatatcttta ggatggtggt tc 52
<210> 329
   <211> 52
   <212> DNA
   <213> Artificial Sequence
<220>
   <223> Synthetic Polynucleotide
<400> 329
   gtcaggacgt tgggaagaaa agacaataaa caacatgttc agaatagccc ga 52
<210> 330
   <211> 52
   <212> DNA
   <213> Artificial Sequence
<220>
   <223> Synthetic Polynucleotide
<400> 330
   aggctggctg accttcatca ataccaacgc taacgagcgt ctagagtcca ct 52
<210> 331
   <211> 52
   <212> DNA
   <213> Artificial Sequence
<220>
   <223> Synthetic Polynucleotide
<400> 331
   taaattgggc ttgagatggt tttttaacgg ggtcagtgcc ttgaaaaacc gt 52
<210> 332
   <211> 52
   <212> DNA
   <213> Artificial Sequence
<220>
   <223> Synthetic Polynucleotide
<400> 332
   tgtgtcgaaa tccgcgacct gagtaataaa agggacattc tggaaaaacc gt 52
<210> 333
   <211> 52
   <212> DNA
   <213> Artificial Sequence
<220>
   <223> Synthetic Polynucleotide
<400> 333
   tacgaaggca ccaacctaaa actggtcagt tggcaaatca acagagtcca ct 52
<210> 334
   <211> 52
   <212> DNA
   <213> Artificial Sequence
<220>
   <223> Synthetic Polynucleotide
<400> 334
   ctttgaaaga ggacagatga atatcaacaa tagataagtc ctaatagccc ga 52
<210> 335
   <211> 52
   <212> DNA
   <213> Artificial Sequence
<220>
   <223> Synthetic Polynucleotide
<400> 335
   gtagcaacgg ctacagaggc ttagttgcta ttttgcaccc agatggtggt tc 52
<210> 336
   <211> 52
   <212> DNA
   <213> Artificial Sequence
<220>
   <223> Synthetic Polynucleotide
<400> 336
   gatattcatt acccaaatca acagttaatg ccccctgcct atcggtccac gc 52
<210> 337
   <211> 52
   <212> DNA
   <213> Artificial Sequence
<220>
   <223> Synthetic Polynucleotide
<400> 337
   ctaaaacact catctttgac cctgacctga aagcgtaaga atcggtccac gc 52
<210> 338
   <211> 52
   <212> DNA
   <213> Artificial Sequence
<220>
   <223> Synthetic Polynucleotide
<400> 338
   cgaataataa ttttttcacg tatcaccttg ctgaacctca aaatggtggt tc 52
<210> 339
   <211> 52
   <212> DNA
   <213> Artificial Sequence
<220>
   <223> Synthetic Polynucleotide
<400> 339
   atgaggaagt ttccattaaa catcctaatt tacgagcatg taaatagccc ga 52
<210> 340
   <211> 52
   <212> DNA
   <213> Artificial Sequence
<220>
   <223> Synthetic Polynucleotide
<400> 340
   ttcgaggtga atttcttaaa cacctcccga cttgcgggag gtagagtcca ct 52
<210> 341
   <211> 52
   <212> DNA
   <213> Artificial Sequence
<220>
   <223> Synthetic Polynucleotide
<400> 341
   ggatcgtcac cctcagcagc gacatgaaag tattaagagg ctgaaaaacc gt 52
<210> 342
   <211> 52
   <212> DNA
   <213> Artificial Sequence
<220>
   <223> Synthetic Polynucleotide
<400> 342
   tttcagcgga gtgagaatag atgaatggct attagtcttt aagaaaaacc gt 52
<210> 343
   <211> 52
   <212> DNA
   <213> Artificial Sequence
<220>
   <223> Synthetic Polynucleotide
<400> 343
   ctacaacgcc tgtagcattc cagtgccacg ctgagagcca gcagagtcca ct 52
<210> 344
   <211> 52
   <212> DNA
   <213> Artificial Sequence
<220>
   <223> Synthetic Polynucleotide
<400> 344
   ctccaaaagg agcctttaat tgtctttcct tatcattcca agaatagccc ga 52
<210> 345
   <211> 52
   <212> DNA
   <213> Artificial Sequence
<220>
   <223> Synthetic Polynucleotide
<400> 345
   cagagccacc accctcattt taaggcttat ccggtattct aaatggtggt tc 52
<210> 346
   <211> 52
   <212> DNA
   <213> Artificial Sequence
<220>
   <223> Synthetic Polynucleotide
<400> 346
   ccgacaatga caacaaccat ctaggattag cggggttttg ctcggtccac gc 52
<210> 347
   <211> 49
   <212> DNA
   <213> Artificial Sequence
<220>
   <223> Synthetic Polynucleotide
<400> 347
   tcatcaacat taaaagaacg cgagaaaatt gttaaatcag accgtgcat 49
<210> 348
   <211> 42
   <212> DNA
   <213> Artificial Sequence
<220>
   <223> Synthetic Polynucleotide
<400> 348
   taatcgtaaa actaatcttc tgacctaaag ctatttttga ta 42
<210> 349
   <211> 42
   <212> DNA
   <213> Artificial Sequence
<220>
   <223> Synthetic Polynucleotide
<400> 349
   atatatttta aatggataaa taaggcgtaa aaacattatg tc 42
<210> 350
   <211> 42
   <212> DNA
   <213> Artificial Sequence
<220>
   <223> Synthetic Polynucleotide
<400> 350
   gcgagctgaa aaggttacta gaaaaagcac gagtagattt ct 42
<210> 351
   <211> 42
   <212> DNA
   <213> Artificial Sequence
<220>
   <223> Synthetic Polynucleotide
<400> 351
   ggtcattttt gcggttctta ccagtatatt caaagcgaac cc 42
<210> 352
   <211> 42
   <212> DNA
   <213> Artificial Sequence
<220>
   <223> Synthetic Polynucleotide
<400> 352
   agaaaacgag aatgtgagaa tcgccatatt tagactggat ag 42
<210> 353
   <211> 42
   <212> DNA
   <213> Artificial Sequence
<220>
   <223> Synthetic Polynucleotide
<400> 353
   acgccaaaag gaatagaggc attttcgacg gaacaacatt ag 42
<210> 354
   <211> 42
   <212> DNA
   <213> Artificial Sequence
<220>
   <223> Synthetic Polynucleotide
<400> 354
   tagtaaattg ggctacaaaa ggtaaagtat tcattaccca ag 42
<210> 355
   <211> 42
   <212> DNA
   <213> Artificial Sequence
<220>
   <223> Synthetic Polynucleotide
<400> 355
   gaacgaggcg cagaacatgt tcagctaaac aaagtacaac ca 42
<210> 356
   <211> 42
   <212> DNA
   <213> Artificial Sequence
<220>
   <223> Synthetic Polynucleotide
<400> 356
   ttcatgagga agttgataag tcctgaactc gtcaccctca tt 42
<210> 357
   <211> 42
   <212> DNA
   <213> Artificial Sequence
<220>
   <223> Synthetic Polynucleotide
<400> 357
   gctttcgagg tgaacgagca tgtagaaaat aataattttt tg 42
<210> 358
   <211> 42
   <212> DNA
   <213> Artificial Sequence
<220>
   <223> Synthetic Polynucleotide
<400> 358
   tagcgtaacg atcttcattc caagaacgcc catgtaccgt aa 42
<210> 359
   <211> 42
   <212> DNA
   <213> Artificial Sequence
<220>
   <223> Synthetic Polynucleotide
<400> 359
   aagtatagcc cggaagaaca agcaagccac tcctcaagag ca 42
<210> 360
   <211> 42
   <212> DNA
   <213> Artificial Sequence
<220>
   <223> Synthetic Polynucleotide
<400> 360
   atacaggagt gtacccgcgc ccaatagcaa tcctcattaa tc 42
<210> 361
   <211> 42
   <212> DNA
   <213> Artificial Sequence
<220>
   <223> Synthetic Polynucleotide
<400> 361
   caccctcaga accgggtatt ctaagaacta ttagcgtttg ac 42
<210> 362
   <211> 42
   <212> DNA
   <213> Artificial Sequence
<220>
   <223> Synthetic Polynucleotide
<400> 362
   cattagcaag gccgtgcggg aggttttgtt aaaggtgaat tt 42
<210> 363
   <211> 42
   <212> DNA
   <213> Artificial Sequence
<220>
   <223> Synthetic Polynucleotide
<400> 363
   caaagacacc acggttgcac ccagctacat taagactcct gg 42
<210> 364
   <211> 42
   <212> DNA
   <213> Artificial Sequence
<220>
   <223> Synthetic Polynucleotide
<400> 364
   agaaacaatg aaatacgagc gtctttccga attaactgaa aa 42
<210> 365
   <211> 28
   <212> DNA
   <213> Artificial Sequence
<220>
   <223> Synthetic Polynucleotide
<400> 365
   gaacaaacgg cggattgaca ataattcg 28
<210> 366
   <211> 42
   <212> DNA
   <213> Artificial Sequence
<220>
   <223> Synthetic Polynucleotide
<400> 366
   cgtctggcct tcctgtcccg gttgataatc agaagagtct gg 42
<210> 367
   <211> 42
   <212> DNA
   <213> Artificial Sequence
<220>
   <223> Synthetic Polynucleotide
<400> 367
   agcaaacaag agaatcaggt aaagattcaa aagtttcaac gc 42
<210> 368
   <211> 42
   <212> DNA
   <213> Artificial Sequence
<220>
   <223> Synthetic Polynucleotide
<400> 368
   aaggataaaa atttttagta gtagcattaa catcaataac ct 42
<210> 369
   <211> 42
   <212> DNA
   <213> Artificial Sequence
<220>
   <223> Synthetic Polynucleotide
<400> 369
   gtttagctat attttcctga atataatgct gtatagagag ta 42
<210> 370
   <211> 42
   <212> DNA
   <213> Artificial Sequence
<220>
   <223> Synthetic Polynucleotide
<400> 370
   cctttaattg ctccttgtct ttaccctgac tattcattga at 42
<210> 371
   <211> 42
   <212> DNA
   <213> Artificial Sequence
<220>
   <223> Synthetic Polynucleotide
<400> 371
   ccccctcaaa tgctttaaca ctatcataac ccttaggaat ac 42
<210> 372
   <211> 42
   <212> DNA
   <213> Artificial Sequence
<220>
   <223> Synthetic Polynucleotide
<400> 372
   cacattcaac taatgccttt aatcattgtg aattaaggct tg 42
<210> 373
   <211> 42
   <212> DNA
   <213> Artificial Sequence
<220>
   <223> Synthetic Polynucleotide
<400> 373
   ccctgacgag aaacaccgaa ctgaccaact ttgcgaaatc cg 42
<210> 374
   <211> 42
   <212> DNA
   <213> Artificial Sequence
<220>
   <223> Synthetic Polynucleotide
<400> 374
   cgacctgctc catgttacgt aatgccacta cgaaacggct ac 42
<210> 375
   <211> 42
   <212> DNA
   <213> Artificial Sequence
<220>
   <223> Synthetic Polynucleotide
<400> 375
   agaggctttg aggactccga tagttgcgcc gacaaaggag cc 42
<210> 376
   <211> 42
   <212> DNA
   <213> Artificial Sequence
<220>
   <223> Synthetic Polynucleotide
<400> 376
   tttaattgta tcggttagac gttagtaaat gaaacgcctg ta 42
<210> 377
   <211> 42
   <212> DNA
   <213> Artificial Sequence
<220>
   <223> Synthetic Polynucleotide
<400> 377
   gcattccaca gacagccagg aggtttagta ccgccaggcg ga 42
<210> 378
   <211> 42
   <212> DNA
   <213> Artificial Sequence
<220>
   <223> Synthetic Polynucleotide
<400> 378
   taagtgccgt cgagagggtc agtgccttga gtaccgttcc ag 42
<210> 379
   <211> 42
   <212> DNA
   <213> Artificial Sequence
<220>
   <223> Synthetic Polynucleotide
<400> 379
   taagcgtcat acatggcctc agagccgcca ccagagccac ca 42
<210> 380
   <211> 42
   <212> DNA
   <213> Artificial Sequence
<220>
   <223> Synthetic Polynucleotide
<400> 380
   ccggaaccgc ctccctcatc gatagcagca ccgttagagc ca 42
<210> 381
   <211> 42
   <212> DNA
   <213> Artificial Sequence
<220>
   <223> Synthetic Polynucleotide
<400> 381
   gcaaaatcac cagtagaatc aatagaaaat tcaacataca ta 42
<210> 382
   <211> 49
   <212> DNA
   <213> Artificial Sequence
<220>
   <223> Synthetic Polynucleotide
<400> 382
   aaggtggcaa catataaagc cctttttaag aaaagaattg agttaagcc 49
<210> 383
   <211> 42
   <212> DNA
   <213> Artificial Sequence
<220>
   <223> Synthetic Polynucleotide
<400> 383
   acaggaagat tgaataggaa cgccatcaaa cgtaatggga ta 42
<210> 384
   <211> 35
   <212> DNA
   <213> Artificial Sequence
<220>
   <223> Synthetic Polynucleotide
<400> 384
   agatgggcgc atcgtactca ttttttaacc tataa 35
<210> 385
   <211> 42
   <212> DNA
   <213> Artificial Sequence
<220>
   <223> Synthetic Polynucleotide
<400> 385
   gcaaatattt aaattgtaaa cgtgagatct acaaaggaat ca 42
<210> 386
   <211> 42
   <212> DNA
   <213> Artificial Sequence
<220>
   <223> Synthetic Polynucleotide
<400> 386
   ccatcaatat gatattcaac cgtaccctgt aatacttaag aa 42
<210> 387
   <211> 42
   <212> DNA
   <213> Artificial Sequence
<220>
   <223> Synthetic Polynucleotide
<400> 387
   ttagcaaaat taagcaataa agcagtttga ccattagcta aa 42
<210> 388
   <211> 42
   <212> DNA
   <213> Artificial Sequence
<220>
   <223> Synthetic Polynucleotide
<400> 388
   gtacggtgtc tggaagtttc attcagaccg gaagcaacat ca 42
<210> 389
   <211> 42
   <212> DNA
   <213> Artificial Sequence
<220>
   <223> Synthetic Polynucleotide
<400> 389
   aaaagattaa gaggaagccc gaaagcgtcc aatactgcaa aa 42
<210> 390
   <211> 42
   <212> DNA
   <213> Artificial Sequence
<220>
   <223> Synthetic Polynucleotide
<400> 390
   tagcgagagg cttttgcaaa agaattacag gtagaaagct ca 42
<210> 391
   <211> 42
   <212> DNA
   <213> Artificial Sequence
<220>
   <223> Synthetic Polynucleotide
<400> 391
   ttataccagt caggacgttg ggaaatcaac gtaacaaaga cc 42
<210> 392
   <211> 42
   <212> DNA
   <213> Artificial Sequence
<220>
   <223> Synthetic Polynucleotide
<400> 392
   aggcgcatag gctggctgac cttggagatt tgtatcagca aa 42
<210> 393
   <211> 42
   <212> DNA
   <213> Artificial Sequence
<220>
   <223> Synthetic Polynucleotide
<400> 393
   agaatacact aaaacactca tctgcagcga aagacagata ac 42
<210> 394
   <211> 42
   <212> DNA
   <213> Artificial Sequence
<220>
   <223> Synthetic Polynucleotide
<400> 394
   cgatatattc ggtcgctgag gcttcacgtt gaaaatccaa ct 42
<210> 395
   <211> 42
   <212> DNA
   <213> Artificial Sequence
<220>
   <223> Synthetic Polynucleotide
<400> 395
   ttcaacagtt tcagcggagt gagaacactg agtttcggaa cc 42
<210> 396
   <211> 42
   <212> DNA
   <213> Artificial Sequence
<220>
   <223> Synthetic Polynucleotide
<400> 396
   gccaccctca gagccaccac cctaaggatt aggattagcc cc 42
<210> 397
   <211> 42
   <212> DNA
   <213> Artificial Sequence
<220>
   <223> Synthetic Polynucleotide
<400> 397
   ctgcctattt cggaacctat tatagccaga atggaaagca tt 42
<210> 398
   <211> 42
   <212> DNA
   <213> Artificial Sequence
<220>
   <223> Synthetic Polynucleotide
<400> 398
   gacaggaggt tgaggcaggt cagccatctt ttcataattg cc 42
<210> 399
   <211> 42
   <212> DNA
   <213> Artificial Sequence
<220>
   <223> Synthetic Polynucleotide
<400> 399
   tttagcgtca gactgtagcg cgttatcacc gtcaccgaaa gg 42
<210> 400
   <211> 42
   <212> DNA
   <213> Artificial Sequence
<220>
   <223> Synthetic Polynucleotide
<400> 400
   gcgacattca accgattgag ggatattacg cagtatgtac ca 42
<210> 401
   <211> 49
   <212> DNA
   <213> Artificial Sequence
<220>
   <223> Synthetic Polynucleotide
<400> 401
   gaaggaaacc gaggaaacgc aatcaccctg aacaaagtca gataatatc 49
<210> 402
   <211> 42
   <212> DNA
   <213> Artificial Sequence
<220>
   <223> Synthetic Polynucleotide
<400> 402
   atattttgtt aaaattcgca ttaaatttct ttttcaaata ta 42
<210> 403
   <211> 42
   <212> DNA
   <213> Artificial Sequence
<220>
   <223> Synthetic Polynucleotide
<400> 403
   tagctgataa attaatgccg gagagggtat ttaatggttt ga 42
<210> 404
   <211> 42
   <212> DNA
   <213> Artificial Sequence
<220>
   <223> Synthetic Polynucleotide
<400> 404
   cagagcataa agctaaatcg gttgtaccta aataagaata aa 42
<210> 405
   <211> 42
   <212> DNA
   <213> Artificial Sequence
<220>
   <223> Synthetic Polynucleotide
<400> 405
   atataacagt tgattcccaa ttctgcgact gtttagtatc at 42
<210> 406
   <211> 42
   <212> DNA
   <213> Artificial Sequence
<220>
   <223> Synthetic Polynucleotide
<400> 406
   acttcaaata tcgcgtttta attcgagcaa gccaacgctc aa 42
<210> 407
   <211> 42
   <212> DNA
   <213> Artificial Sequence
<220>
   <223> Synthetic Polynucleotide
<400> 407
   ttttgccaga gggggtaata gtaaaatgtt taacaacgcc aa 42
<210> 408
   <211> 42
   <212> DNA
   <213> Artificial Sequence
<220>
   <223> Synthetic Polynucleotide
<400> 408
   aaaaatctac gttaataaaa cgaactaagc cagtaataag ag 42
<210> 409
   <211> 42
   <212> DNA
   <213> Artificial Sequence
<220>
   <223> Synthetic Polynucleotide
<400> 409
   tcaagagtaa tcttgacaag aaccggataa ttctgtccag ac 42
<210> 410
   <211> 42
   <212> DNA
   <213> Artificial Sequence
<220>
   <223> Synthetic Polynucleotide
<400> 410
   gacccccagc gattatacca agcgcgaatg cagaacgcgc ct 42
<210> 411
   <211> 42
   <212> DNA
   <213> Artificial Sequence
<220>
   <223> Synthetic Polynucleotide
<400> 411
   cagggagtta aaggccgctt ttgcgggaaa gaaaaataat at 42
<210> 412
   <211> 42
   <212> DNA
   <213> Artificial Sequence
<220>
   <223> Synthetic Polynucleotide
<400> 412
   tagaaaggaa caactaaagg aattgcgacc aatcaataat cg 42
<210> 413
   <211> 42
   <212> DNA
   <213> Artificial Sequence
<220>
   <223> Synthetic Polynucleotide
<400> 413
   ttttcaggga tagcaagccc aataggaagg tattaaacca ag 42
<210> 414
   <211> 42
   <212> DNA
   <213> Artificial Sequence
<220>
   <223> Synthetic Polynucleotide
<400> 414
   tgaaacatga aagtattaag aggctgaggt ttttattttc at 42
<210> 415
   <211> 42
   <212> DNA
   <213> Artificial Sequence
<220>
   <223> Synthetic Polynucleotide
<400> 415
   gattggcctt gatattcaca aacaaataaa gcaaatcaga ta 42
<210> 416
   <211> 42
   <212> DNA
   <213> Artificial Sequence
<220>
   <223> Synthetic Polynucleotide
<400> 416
   tcatcggcat tttcggtcat agccccctgc gaggcgtttt ag 42
<210> 417
   <211> 42
   <212> DNA
   <213> Artificial Sequence
<220>
   <223> Synthetic Polynucleotide
<400> 417
   gaaggtaaat attgacggaa attattcaaa gccttaaatc aa 42
<210> 418
   <211> 42
   <212> DNA
   <213> Artificial Sequence
<220>
   <223> Synthetic Polynucleotide
<400> 418
   taacggaata cccaaaagaa ctggcatgaa ttttatcctg aa 42
<210> 419
   <211> 21
   <212> DNA
   <213> Artificial Sequence
<220>
   <223> Synthetic Polynucleotide
<400> 419
   cgcattagac gggaagagcc t 21
<210> 420
   <211> 28
   <212> DNA
   <213> Artificial Sequence
<220>
   <223> Synthetic Polynucleotide
<400> 420
   aagacaatgt gagcgagtaa caacccgt 28
<210> 421
   <211> 42
   <212> DNA
   <213> Artificial Sequence
<220>
   <223> Synthetic Polynucleotide
<400> 421
   ttttagttaa tttcgcatgt caatcatatg tacagccagc tt 42
<210> 422
   <211> 42
   <212> DNA
   <213> Artificial Sequence
<220>
   <223> Synthetic Polynucleotide
<400> 422
   aataccgacc gtgtcaatgc ctgagtaatg tgtgatgaac gg 42
<210> 423
   <211> 42
   <212> DNA
   <213> Artificial Sequence
<220>
   <223> Synthetic Polynucleotide
<400> 423
   caccggaatc ataatggcat caattctact aatagaaccc tc 42
<210> 424
   <211> 42
   <212> DNA
   <213> Artificial Sequence
<220>
   <223> Synthetic Polynucleotide
<400> 424
   atgcgttata caaaatggct tagagcttaa ttgatttggg gc 42
<210> 425
   <211> 42
   <212> DNA
   <213> Artificial Sequence
<220>
   <223> Synthetic Polynucleotide
<400> 425
   cagtagggct taataccata aatcaaaaat cagttgataa ga 42
<210> 426
   <211> 42
   <212> DNA
   <213> Artificial Sequence
<220>
   <223> Synthetic Polynucleotide
<400> 426
   catgtaattt aggctacgag gcatagtaag agcaaacagt tc 42
<210> 427
   <211> 42
   <212> DNA
   <213> Artificial Sequence
<220>
   <223> Synthetic Polynucleotide
<400> 427
   aatataaagt accgtgagat ggtttaattt caaagataca ta 42
<210> 428
   <211> 42
   <212> DNA
   <213> Artificial Sequence
<220>
   <223> Synthetic Polynucleotide
<400> 428
   gacgacaata aacacggtca atcataaggg aaccagaacg ag 42
<210> 429
   <211> 42
   <212> DNA
   <213> Artificial Sequence
<220>
   <223> Synthetic Polynucleotide
<400> 429
   gtttatcaac aatatccatt aaacgggtaa aatacttagc cg 42
<210> 430
   <211> 42
   <212> DNA
   <213> Artificial Sequence
<220>
   <223> Synthetic Polynucleotide
<400> 430
   cccatcctaa tttatttctt aaacagcttg ataaaagact tt 42
<210> 431
   <211> 42
   <212> DNA
   <213> Artificial Sequence
<220>
   <223> Synthetic Polynucleotide
<400> 431
   gctgtctttc cttaaaagtt ttgtcgtctt tcctatcagc tt 42
<210> 432
   <211> 42
   <212> DNA
   <213> Artificial Sequence
<220>
   <223> Synthetic Polynucleotide
<400> 432
   taccgcactc atcgataggt gtatcaccgt actcctcata gt 42
<210> 433
   <211> 42
   <212> DNA
   <213> Artificial Sequence
<220>
   <223> Synthetic Polynucleotide
<400> 433
   cgtaggaatc attatggtaa taagttttaa cggggttgat at 42
<210> 434
   <211> 42
   <212> DNA
   <213> Artificial Sequence
<220>
   <223> Synthetic Polynucleotide
<400> 434
   tagaaggctt atccccaccc tcagagccac caccttttga tg 42
<210> 435
   <211> 42
   <212> DNA
   <213> Artificial Sequence
<220>
   <223> Synthetic Polynucleotide
<400> 435
   cgaacctccc gactgaaacg tcaccaatga aaccagagcc gc 42
<210> 436
   <211> 42
   <212> DNA
   <213> Artificial Sequence
<220>
   <223> Synthetic Polynucleotide
<400> 436
   gattagttgc tattaataag tttattttgt caccaccatt ac 42
<210> 437
   <211> 42
   <212> DNA
   <213> Artificial Sequence
<220>
   <223> Synthetic Polynucleotide
<400> 437
   tcttaccaac gctaagcaat agctatctta ccgaaagaaa cg 42
<210> 438
   <211> 42
   <212> DNA
   <213> Artificial Sequence
<220>
   <223> Synthetic Polynucleotide
<400> 438
   cggagacagt cactatcagg tcattgcctg aaagccccaa aa 42
<210> 439
   <211> 42
   <212> DNA
   <213> Artificial Sequence
<220>
   <223> Synthetic Polynucleotide
<400> 439
   acaggcaagg cattgcggga gaagccttta ggtgagaaag gc 42
<210> 440
   <211> 42
   <212> DNA
   <213> Artificial Sequence
<220>
   <223> Synthetic Polynucleotide
<400> 440
   ttaaatatgc aaatacattt cgcaaatggt ccaataaatc at 42
<210> 441
   <211> 42
   <212> DNA
   <213> Artificial Sequence
<220>
   <223> Synthetic Polynucleotide
<400> 441
   caaagcggat tgactccaac aggtcaggat gctcaacatg tt 42
<210> 442
   <211> 42
   <212> DNA
   <213> Artificial Sequence
<220>
   <223> Synthetic Polynucleotide
<400> 442
   gacgataaaa accggaatcg tcataaatat tatagtcaga ag 42
<210> 443
   <211> 42
   <212> DNA
   <213> Artificial Sequence
<220>
   <223> Synthetic Polynucleotide
<400> 443
   ttttaagaac tggattcatc agttgagatt cgtttaccag ac 42
<210> 444
   <211> 42
   <212> DNA
   <213> Artificial Sequence
<220>
   <223> Synthetic Polynucleotide
<400> 444
   tgaacggtgt acagctgctc attcagtgaa taccttatgc ga 42
<210> 445
   <211> 42
   <212> DNA
   <213> Artificial Sequence
<220>
   <223> Synthetic Polynucleotide
<400> 445
   aaaacgaaag agtcgcctga taaattgtgt aaagaggaca ga 42
<210> 446
   <211> 42
   <212> DNA
   <213> Artificial Sequence
<220>
   <223> Synthetic Polynucleotide
<400> 446
   catcgcccac gccatcggaa cgagggtagc aggcaccaac ct 42
<210> 447
   <211> 42
   <212> DNA
   <213> Artificial Sequence
<220>
   <223> Synthetic Polynucleotide
<400> 447
   gattttgcta aatccaaaaa aaaggctcca aatgacaaca ac 42
<210> 448
   <211> 42
   <212> DNA
   <213> Artificial Sequence
<220>
   <223> Synthetic Polynucleotide
<400> 448
   ccgccaccct catcaccagt acaaactaca ttttctgtat gg 42
<210> 449
   <211> 42
   <212> DNA
   <213> Artificial Sequence
<220>
   <223> Synthetic Polynucleotide
<400> 449
   taaacagtta atgcggggtt ttgctcagta ccaccctcag aa 42
<210> 450
   <211> 42
   <212> DNA
   <213> Artificial Sequence
<220>
   <223> Synthetic Polynucleotide
<400> 450
   gagccgccgc cagcgcagtc tctgaattta acagtgcccg ta 42
<210> 451
   <211> 42
   <212> DNA
   <213> Artificial Sequence
<220>
   <223> Synthetic Polynucleotide
<400> 451
   acagaatcaa gttcaaaatc accggaacca gaaccaccac ca 42
<210> 452
   <211> 42
   <212> DNA
   <213> Artificial Sequence
<220>
   <223> Synthetic Polynucleotide
<400> 452
   gcgccaaaga caacttgagc catttgggaa taatcagtag cg 42
<210> 453
   <211> 41
   <212> DNA
   <213> Artificial Sequence
<220>
   <223> Synthetic Polynucleotide
<400> 453
   ccgaacaaag tttagcaaac gtagaaaatt atggtttacc a 41
<210> 454
   <211> 29
   <212> DNA
   <213> Artificial Sequence
<220>
   <223> Synthetic Polynucleotide
<400> 454
   agagagataa cccacaagta agcagatag 29
<210> 455
   <211> 63
   <212> DNA
   <213> Artificial Sequence
<220>
   <223> Synthetic Polynucleotide
<400> 455
   agaagatgaa attaactaaa atatatttga aaaagttttc tcgcgttctt tgtcttgcga 60
   ttg 63
<210> 456
   <211> 42
   <212> DNA
   <213> Artificial Sequence
<220>
   <223> Synthetic Polynucleotide
<400> 456
   atttatcaca cggtcggtat ttcaaaccat taaatttagg tc 42
<210> 457
   <211> 42
   <212> DNA
   <213> Artificial Sequence
<220>
   <223> Synthetic Polynucleotide
<400> 457
   ctagtaatta tgattccggt gtttattctt atttaacgcc tt 42
<210> 458
   <211> 42
   <212> DNA
   <213> Artificial Sequence
<220>
   <223> Synthetic Polynucleotide
<400> 458
   gtaagaattt gtataacgca tatgatacta aacaggcttt tt 42
<210> 459
   <211> 42
   <212> DNA
   <213> Artificial Sequence
<220>
   <223> Synthetic Polynucleotide
<400> 459
   attctcaatt aagccctact gttgagcgtt ggctttatac tg 42
<210> 460
   <211> 42
   <212> DNA
   <213> Artificial Sequence
<220>
   <223> Synthetic Polynucleotide
<400> 460
   tgcctctgcc taaattacat gttggcgttg ttaaatatgg cg 42
<210> 461
   <211> 42
   <212> DNA
   <213> Artificial Sequence
<220>
   <223> Synthetic Polynucleotide
<400> 461
   cttttgtcgg tactttatat tctcttatta ctggctcgaa aa 42
<210> 462
   <211> 42
   <212> DNA
   <213> Artificial Sequence
<220>
   <223> Synthetic Polynucleotide
<400> 462
   aacatgttgt ttattgtcgt cgtctggaca gaattacttt ac 42
<210> 463
   <211> 42
   <212> DNA
   <213> Artificial Sequence
<220>
   <223> Synthetic Polynucleotide
<400> 463
   acttatctat tgttgataaa caggcgcgtt ctgcattagc tg 42
<210> 464
   <211> 42
   <212> DNA
   <213> Artificial Sequence
<220>
   <223> Synthetic Polynucleotide
<400> 464
   atgctcgtaa attaggatgg gatattattt ttcttgttca gg 42
<210> 465
   <211> 42
   <212> DNA
   <213> Artificial Sequence
<220>
   <223> Synthetic Polynucleotide
<400> 465
   ggaatgataa ggaaagacag ccgattattg attggtttct ac 42
<210> 466
   <211> 42
   <212> DNA
   <213> Artificial Sequence
<220>
   <223> Synthetic Polynucleotide
<400> 466
   ttgttctcga tgagtgcggt acttggttta atacccgttc tt 42
<210> 467
   <211> 42
   <212> DNA
   <213> Artificial Sequence
<220>
   <223> Synthetic Polynucleotide
<400> 467
   ggcgcggtaa tgattcctac gatgaaaata aaaacggctt gc 42
<210> 468
   <211> 42
   <212> DNA
   <213> Artificial Sequence
<220>
   <223> Synthetic Polynucleotide
<400> 468
   gaataccgga taagccttct atatctgatt tgcttgctat tg 42
<210> 469
   <211> 42
   <212> DNA
   <213> Artificial Sequence
<220>
   <223> Synthetic Polynucleotide
<400> 469
   tcccgcaagt cgggaggttc gctaaaacgc ctcgcgttct ta 42
<210> 470
   <211> 42
   <212> DNA
   <213> Artificial Sequence
<220>
   <223> Synthetic Polynucleotide
<400> 470
   ggtgcaaaat agcaactaat cttgatttaa ggcttcaaaa cc 42
<210> 471
   <211> 63
   <212> DNA
   <213> Artificial Sequence
<220>
   <223> Synthetic Polynucleotide
<400> 471
   gcaaattagg ctctggaaag acgctcgtta gcgttggtaa gattcaggat aaaattgtag 60
   ctg 63
<210> 472
   <211> 42
   <212> DNA
   <213> Artificial Sequence
<220>
   <223> Synthetic Polynucleotide
<400> 472
   attctcaatt agcgtggacc gttgagcgtt ggctttatac tg 42
<210> 473
   <211> 42
   <212> DNA
   <213> Artificial Sequence
<220>
   <223> Synthetic Polynucleotide
<400> 473
   tgcctctgcc tgaaccacca tttggcgttg ttaaatatgg cg 42
<210> 474
   <211> 42
   <212> DNA
   <213> Artificial Sequence
<220>
   <223> Synthetic Polynucleotide
<400> 474
   cttttgtcgg ttcgggctat tctcttatta ctggctcgaa aa 42
<210> 475
   <211> 42
   <212> DNA
   <213> Artificial Sequence
<220>
   <223> Synthetic Polynucleotide
<400> 475
   aacatgttgt tagtggactc tgtctggaca gaattacttt ac 42
<210> 476
   <211> 42
   <212> DNA
   <213> Artificial Sequence
<220>
   <223> Synthetic Polynucleotide
<400> 476
   acttatctat tacggttttt caggcgcgtt ctgcattagc tg 42
<210> 477
   <211> 32
   <212> DNA
   <213> Artificial Sequence
<220>
   <223> Synthetic Polynucleotide
<400> 477
   taataccata aatcaaaaat cagttgataa ga 32
<210> 478
   <211> 32
   <212> DNA
   <213> Artificial Sequence
<220>
   <223> Synthetic Polynucleotide
<400> 478
   aggctacgag gcatagtaag agcaaacagt tc 32
<210> 479
   <211> 32
   <212> DNA
   <213> Artificial Sequence
<220>
   <223> Synthetic Polynucleotide
<400> 479
   accgtgagat ggtttaattt caaagataca ta 32
<210> 480
   <211> 32
   <212> DNA
   <213> Artificial Sequence
<220>
   <223> Synthetic Polynucleotide
<400> 480
   aacacggtca atcataaggg aaccagaacg ag 32
<210> 481
   <211> 32
   <212> DNA
   <213> Artificial Sequence
<220>
   <223> Synthetic Polynucleotide
<400> 481
   aatatccatt aaacgggtaa aatacttagc cg 32
<210> 482
   <211> 42
   <212> DNA
   <213> Artificial Sequence
<220>
   <223> Synthetic Polynucleotide
<400> 482
   aacggcatct ccgtgagcct cctcacagag cctggggtgc ct 42
<210> 483
   <211> 42
   <212> DNA
   <213> Artificial Sequence
<220>
   <223> Synthetic Polynucleotide
<400> 483
   ggcagcaccc atcccttaca ctggtgtggt tgcgctcact gc 42
<210> 484
   <211> 42
   <212> DNA
   <213> Artificial Sequence
<220>
   <223> Synthetic Polynucleotide
<400> 484
   aaatcccgtg gtctggtcag cagcaacccc agctgcatta at 42
<210> 485
   <211> 42
   <212> DNA
   <213> Artificial Sequence
<220>
   <223> Synthetic Polynucleotide
<400> 485
   gagccgccaa gcagttgggc ggttgtgttt tgcgtattgg gc 42
<210> 486
   <211> 42
   <212> DNA
   <213> Artificial Sequence
<220>
   <223> Synthetic Polynucleotide
<400> 486
   ggcaccgcta aaacgacggc cagtgccaag acgggcaaca gc 42
<210> 487
   <211> 42
   <212> DNA
   <213> Artificial Sequence
<220>
   <223> Synthetic Polynucleotide
<400> 487
   cgcgtctggg cctcaggaag atcgcactag agttgcagca ag 42
<210> 488
   <211> 42
   <212> DNA
   <213> Artificial Sequence
<220>
   <223> Synthetic Polynucleotide
<400> 488
   ggagcaaact tttaaccaat aggaacgcga aaatcctgtt tg 42
<210> 489
   <211> 42
   <212> DNA
   <213> Artificial Sequence
<220>
   <223> Synthetic Polynucleotide
<400> 489
   gcaaggatat acaaaggcta tcaggtcatt ataaatcaaa ag 42
<210> 490
   <211> 42
   <212> DNA
   <213> Artificial Sequence
<220>
   <223> Synthetic Polynucleotide
<400> 490
   ctgtttagct aatacttttg cgggagaatc cagtttggaa ca 42
<210> 491
   <211> 42
   <212> DNA
   <213> Artificial Sequence
<220>
   <223> Synthetic Polynucleotide
<400> 491
   tacctttaaa ccattagata catttcgcca acgtcaaagg gc 42
<210> 492
   <211> 42
   <212> DNA
   <213> Artificial Sequence
<220>
   <223> Synthetic Polynucleotide
<400> 492
   atccccctcg gaagcaaact ccaacaggac tacgtgaacc at 42
<210> 493
   <211> 42
   <212> DNA
   <213> Artificial Sequence
<220>
   <223> Synthetic Polynucleotide
<400> 493
   accacattcc aatactgcgg aatcgtcagt gccgtaaagc ac 42
<210> 494
   <211> 42
   <212> DNA
   <213> Artificial Sequence
<220>
   <223> Synthetic Polynucleotide
<400> 494
   tgccctgacg gtagaaagat tcatcagtat ttagagcttg ac 42
<210> 495
   <211> 42
   <212> DNA
   <213> Artificial Sequence
<220>
   <223> Synthetic Polynucleotide
<400> 495
   cgcgacctgc gtaacaaagc tgctcattgg aagggaagaa ag 42
<210> 496
   <211> 42
   <212> DNA
   <213> Artificial Sequence
<220>
   <223> Synthetic Polynucleotide
<400> 496
   acagaggctt tgtatcatcg cctgataaaa gtgtagcggt ca 42
<210> 497
   <211> 42
   <212> DNA
   <213> Artificial Sequence
<220>
   <223> Synthetic Polynucleotide
<400> 497
   cctttaatta aagacagcat cggaacgagc ttaatgcgcc gc 42
<210> 498
   <211> 42
   <212> DNA
   <213> Artificial Sequence
<220>
   <223> Synthetic Polynucleotide
<400> 498
   tagcattcct gaaaatctcc aaaaaaaacg agcacgtata ac 42
<210> 499
   <211> 42
   <212> DNA
   <213> Artificial Sequence
<220>
   <223> Synthetic Polynucleotide
<400> 499
   gataagtgcg agtttcgtca ccagtacaag ctaaacagga gg 42
<210> 500
   <211> 42
   <212> DNA
   <213> Artificial Sequence
<220>
   <223> Synthetic Polynucleotide
<400> 500
   agtaagcgtt aggattagcg gggttttggt acgccagaat cc 42
<210> 501
   <211> 42
   <212> DNA
   <213> Artificial Sequence
<220>
   <223> Synthetic Polynucleotide
<400> 501
   caccggaaca atggaaagcg cagtctctca ccgagtaaaa ga 42
<210> 502
   <211> 42
   <212> DNA
   <213> Artificial Sequence
<220>
   <223> Synthetic Polynucleotide
<400> 502
   cagcaaaatt ttcataatca aaatcaccta gcaatacttc tt 42
<210> 503
   <211> 42
   <212> DNA
   <213> Artificial Sequence
<220>
   <223> Synthetic Polynucleotide
<400> 503
   taaaggtggc gtcaccgact tgagccatta gaagaactca aa 42
<210> 504
   <211> 42
   <212> DNA
   <213> Artificial Sequence
<220>
   <223> Synthetic Polynucleotide
<400> 504
   attgagttag cagtatgtta gcaaacgtca atattaccgc ca 42
<210> 505
   <211> 42
   <212> DNA
   <213> Artificial Sequence
<220>
   <223> Synthetic Polynucleotide
<400> 505
   atttgccagt gagcgctaat atcagagaaa atacctacat tt 42
<210> 506
   <211> 42
   <212> DNA
   <213> Artificial Sequence
<220>
   <223> Synthetic Polynucleotide
<400> 506
   tttcatcgtt accaacgcta acgagcgttt acattggcag at 42
<210> 507
   <211> 42
   <212> DNA
   <213> Artificial Sequence
<220>
   <223> Synthetic Polynucleotide
<400> 507
   ccagacgacc gcactcatcg agaacaaggg acattctggc ca 42
<210> 508
   <211> 42
   <212> DNA
   <213> Artificial Sequence
<220>
   <223> Synthetic Polynucleotide
<400> 508
   aataaacaca taaagtaccg acaaaagggc gtaagaatac gt 42
<210> 509
   <211> 42
   <212> DNA
   <213> Artificial Sequence
<220>
   <223> Synthetic Polynucleotide
<400> 509
   atttatcaaa ccgaccgtgt gataaatata gtctttaatg cg 42
<210> 510
   <211> 42
   <212> DNA
   <213> Artificial Sequence
<220>
   <223> Synthetic Polynucleotide
<400> 510
   agatgatgat tagattaaga cgctgagata aaaataccga ac 42
<210> 511
   <211> 42
   <212> DNA
   <213> Artificial Sequence
<220>
   <223> Synthetic Polynucleotide
<400> 511
   agggttagac gaattattca tttcaatttg aggcggtcag ta 42
<210> 512
   <211> 42
   <212> DNA
   <213> Artificial Sequence
<220>
   <223> Synthetic Polynucleotide
<400> 512
   agaagtattc tgattgtttg gattatacga gagccagcag ca 42
<210> 513
   <211> 42
   <212> DNA
   <213> Artificial Sequence
<220>
   <223> Synthetic Polynucleotide
<400> 513
   tcatggtcat agccgtgcct gttcttcgcg agatgccggg tt 42
<210> 514
   <211> 42
   <212> DNA
   <213> Artificial Sequence
<220>
   <223> Synthetic Polynucleotide
<400> 514
   acctgcagcc agctctttgc tcgtcataaa gtcggtggtg cc 42
<210> 515
   <211> 42
   <212> DNA
   <213> Artificial Sequence
<220>
   <223> Synthetic Polynucleotide
<400> 515
   atcccacgca accaacgtca gcgtggtgct aaaaaaagcc gc 42
<210> 516
   <211> 42
   <212> DNA
   <213> Artificial Sequence
<220>
   <223> Synthetic Polynucleotide
<400> 516
   acaggcggcc ttttctgctc atttgccgcc cgggaacgga ta 42
<210> 517
   <211> 42
   <212> DNA
   <213> Artificial Sequence
<220>
   <223> Synthetic Polynucleotide
<400> 517
   acctcaccgg aaacccagtc acgacgttgt tctggtgccg ga 42
<210> 518
   <211> 42
   <212> DNA
   <213> Artificial Sequence
<220>
   <223> Synthetic Polynucleotide
<400> 518
   aaccaggcaa agcggacgac gacagtatcg ccttcctgta gc 42
<210> 519
   <211> 42
   <212> DNA
   <213> Artificial Sequence
<220>
   <223> Synthetic Polynucleotide
<400> 519
   cagctttcat caatgttaaa tcagctcatt aagagaatcg at 42
<210> 520
   <211> 42
   <212> DNA
   <213> Artificial Sequence
<220>
   <223> Synthetic Polynucleotide
<400> 520
   gaacggtaat cgtgctattt ttgagagatc aaaattttta ga 42
<210> 521
   <211> 42
   <212> DNA
   <213> Artificial Sequence
<220>
   <223> Synthetic Polynucleotide
<400> 521
   accctcatat attaaaacat tatgaccctg tatattttca tt 42
<210> 522
   <211> 42
   <212> DNA
   <213> Artificial Sequence
<220>
   <223> Synthetic Polynucleotide
<400> 522
   tggggcgcga gctcgagtag atttagtttg ttgctccttt tg 42
<210> 523
   <211> 42
   <212> DNA
   <213> Artificial Sequence
<220>
   <223> Synthetic Polynucleotide
<400> 523
   ataagaggtc atttcaaagc gaaccagacc aaatgcttta aa 42
<210> 524
   <211> 42
   <212> DNA
   <213> Artificial Sequence
<220>
   <223> Synthetic Polynucleotide
<400> 524
   cagttcagaa aacttagact ggatagcgtc aactaatgca ga 42
<210> 525
   <211> 42
   <212> DNA
   <213> Artificial Sequence
<220>
   <223> Synthetic Polynucleotide
<400> 525
   tacataacgc caaggaacaa cattattaca gagaaacacc ag 42
<210> 526
   <211> 42
   <212> DNA
   <213> Artificial Sequence
<220>
   <223> Synthetic Polynucleotide
<400> 526
   aacgagtagt aaattcatta cccaaatcaa ctccatgtta ct 42
<210> 527
   <211> 42
   <212> DNA
   <213> Artificial Sequence
<220>
   <223> Synthetic Polynucleotide
<400> 527
   tagccggaac gagcaaagta caacggagat ttgaggacta aa 42
<210> 528
   <211> 42
   <212> DNA
   <213> Artificial Sequence
<220>
   <223> Synthetic Polynucleotide
<400> 528
   gactttttca tgacgtcacc ctcagcagcg gtatcggttt at 42
<210> 529
   <211> 42
   <212> DNA
   <213> Artificial Sequence
<220>
   <223> Synthetic Polynucleotide
<400> 529
   cagcttgctt tcgtaataat tttttcacgt acagacagcc ct 42
<210> 530
   <211> 42
   <212> DNA
   <213> Artificial Sequence
<220>
   <223> Synthetic Polynucleotide
<400> 530
   catagttagc gtaccatgta ccgtaacact cgtcgagagg gt 42
<210> 531
   <211> 42
   <212> DNA
   <213> Artificial Sequence
<220>
   <223> Synthetic Polynucleotide
<400> 531
   tgatataagt atactcctca agagaaggat catacatggc tt 42
<210> 532
   <211> 42
   <212> DNA
   <213> Artificial Sequence
<220>
   <223> Synthetic Polynucleotide
<400> 532
   ttgatgatac aggatcctca ttaaagccag cgcctccctc ag 42
<210> 533
   <211> 42
   <212> DNA
   <213> Artificial Sequence
<220>
   <223> Synthetic Polynucleotide
<400> 533
   agccgccacc ctcattagcg tttgccatct caccagtagc ac 42
<210> 534
   <211> 42
   <212> DNA
   <213> Artificial Sequence
<220>
   <223> Synthetic Polynucleotide
<400> 534
   cattaccatt agctaaaggt gaattatcac caacatataa aa 42
<210> 535
   <211> 42
   <212> DNA
   <213> Artificial Sequence
<220>
   <223> Synthetic Polynucleotide
<400> 535
   gaaacgcaaa gacttaagac tccttattac agcccaataa ta 42
<210> 536
   <211> 42
   <212> DNA
   <213> Artificial Sequence
<220>
   <223> Synthetic Polynucleotide
<400> 536
   agagcaagaa acacaaagtc agagggtaat ttacaaaata aa 42
<210> 537
   <211> 42
   <212> DNA
   <213> Artificial Sequence
<220>
   <223> Synthetic Polynucleotide
<400> 537
   cagccatatt attaatttta tcctgaatct aggaatcatt ac 42
<210> 538
   <211> 42
   <212> DNA
   <213> Artificial Sequence
<220>
   <223> Synthetic Polynucleotide
<400> 538
   cgcgcccaat agcggtatta aaccaagtac gacaataaac aa 42
<210> 539
   <211> 42
   <212> DNA
   <213> Artificial Sequence
<220>
   <223> Synthetic Polynucleotide
<400> 539
   catgttcagc taagccagta ataagagaat cggaatcata at 42
<210> 540
   <211> 42
   <212> DNA
   <213> Artificial Sequence
<220>
   <223> Synthetic Polynucleotide
<400> 540
   tactagaaaa agcatttaat ggtttgaaat aatcataggt ct 42
<210> 541
   <211> 42
   <212> DNA
   <213> Artificial Sequence
<220>
   <223> Synthetic Polynucleotide
<400> 541
   gagagactac cttgaaaaca tagcgatagc aacaaacatc aa 42
<210> 542
   <211> 42
   <212> DNA
   <213> Artificial Sequence
<220>
   <223> Synthetic Polynucleotide
<400> 542
   gaaaacaaaa ttaacaaaat cgcgcagagg acctaccata tc 42
<210> 543
   <211> 46
   <212> DNA
   <213> Artificial Sequence
<220>
   <223> Synthetic Polynucleotide
<400> 543
   aaaattattt gcaaattcat caatataatc agactttaca aacaat 46
<210> 544
   <211> 49
   <212> DNA
   <213> Artificial Sequence
<220>
   <223> Synthetic Polynucleotide
<400> 544
   tcgacaactc taacaactaa tcgtcaatag ataatgaacc tcaaatatc 49
<210> 545
   <211> 33
   <212> DNA
   <213> Artificial Sequence
<220>
   <223> Synthetic Polynucleotide
<400> 545
   tctgccagca cgtgtttcct gtgtgccgct cac 33
<210> 546
   <211> 42
   <212> DNA
   <213> Artificial Sequence
<220>
   <223> Synthetic Polynucleotide
<400> 546
   tgggtaaagg ttggtgccgg tgccccctgc ataccggggg tt 42
<210> 547
   <211> 42
   <212> DNA
   <213> Artificial Sequence
<220>
   <223> Synthetic Polynucleotide
<400> 547
   ccggacttgt agagcttacg gctggaggtg tgcggctggt aa 42
<210> 548
   <211> 42
   <212> DNA
   <213> Artificial Sequence
<220>
   <223> Synthetic Polynucleotide
<400> 548
   caaacttaaa ttagtgatga agggtaaagt taacggaacg tg 42
<210> 549
   <211> 42
   <212> DNA
   <213> Artificial Sequence
<220>
   <223> Synthetic Polynucleotide
<400> 549
   cgccagggtt ttcaatcggc gaaacgtaca gaaacagcgg at 42
<210> 550
   <211> 42
   <212> DNA
   <213> Artificial Sequence
<220>
   <223> Synthetic Polynucleotide
<400> 550
   gccagtttga gggccattcg ccattcaggc taagttgggt aa 42
<210> 551
   <211> 42
   <212> DNA
   <213> Artificial Sequence
<220>
   <223> Synthetic Polynucleotide
<400> 551
   gcattaaatt ttcattaaat gtgagcgagt aaccgtgcat ct 42
<210> 552
   <211> 42
   <212> DNA
   <213> Artificial Sequence
<220>
   <223> Synthetic Polynucleotide
<400> 552
   ccggagaggg taaaaactag catgtcaatc ttgttaaaat tc 42
<210> 553
   <211> 42
   <212> DNA
   <213> Artificial Sequence
<220>
   <223> Synthetic Polynucleotide
<400> 553
   tcggttgtac cattaaatgc aatgcctgag gataaattaa tg 42
<210> 554
   <211> 42
   <212> DNA
   <213> Artificial Sequence
<220>
   <223> Synthetic Polynucleotide
<400> 554
   caattctgcg aagaaaaggt ggcatcaatt cataaagcta aa 42
<210> 555
   <211> 42
   <212> DNA
   <213> Artificial Sequence
<220>
   <223> Synthetic Polynucleotide
<400> 555
   ttaattcgag cttttgcgga tggcttagag acagttgatt cc 42
<210> 556
   <211> 42
   <212> DNA
   <213> Artificial Sequence
<220>
   <223> Synthetic Polynucleotide
<400> 556
   atagtaaaat gtgagaatga ccataaatca aaatatcgcg tt 42
<210> 557
   <211> 42
   <212> DNA
   <213> Artificial Sequence
<220>
   <223> Synthetic Polynucleotide
<400> 557
   aaacgaacta acaaggaatt acgaggcata ccagaggggg ta 42
<210> 558
   <211> 42
   <212> DNA
   <213> Artificial Sequence
<220>
   <223> Synthetic Polynucleotide
<400> 558
   aagaaccgga tattgggctt gagatggttt tctacgttaa ta 42
<210> 559
   <211> 42
   <212> DNA
   <213> Artificial Sequence
<220>
   <223> Synthetic Polynucleotide
<400> 559
   ccaagcgcga aagcgcagac ggtcaatcat agtaatcttg ac 42
<210> 560
   <211> 42
   <212> DNA
   <213> Artificial Sequence
<220>
   <223> Synthetic Polynucleotide
<400> 560
   cttttgcggg atggaagttt ccattaaacg ccagcgatta ta 42
<210> 561
   <211> 42
   <212> DNA
   <213> Artificial Sequence
<220>
   <223> Synthetic Polynucleotide
<400> 561
   aggaattgcg aaaggtgaat ttcttaaaca agttaaaggc cg 42
<210> 562
   <211> 42
   <212> DNA
   <213> Artificial Sequence
<220>
   <223> Synthetic Polynucleotide
<400> 562
   cccaatagga acacgatcta aagttttgtc aggaacaact aa 42
<210> 563
   <211> 42
   <212> DNA
   <213> Artificial Sequence
<220>
   <223> Synthetic Polynucleotide
<400> 563
   aagaggctga gagcccggaa taggtgtatc agggatagca ag 42
<210> 564
   <211> 42
   <212> DNA
   <213> Artificial Sequence
<220>
   <223> Synthetic Polynucleotide
<400> 564
   acaaacaaat aaagtgtact ggtaataagt catgaaagta tt 42
<210> 565
   <211> 42
   <212> DNA
   <213> Artificial Sequence
<220>
   <223> Synthetic Polynucleotide
<400> 565
   catagccccc ttagaaccgc caccctcaga gccttgatat tc 42
<210> 566
   <211> 42
   <212> DNA
   <213> Artificial Sequence
<220>
   <223> Synthetic Polynucleotide
<400> 566
   gaaattattc ataaggccgg aaacgtcacc ggcattttcg gt 42
<210> 567
   <211> 42
   <212> DNA
   <213> Artificial Sequence
<220>
   <223> Synthetic Polynucleotide
<400> 567
   gaactggcat gaaccacgga ataagtttat taaatattga cg 42
<210> 568
   <211> 42
   <212> DNA
   <213> Artificial Sequence
<220>
   <223> Synthetic Polynucleotide
<400> 568
   gaacaccctg aaatgaaata gcaatagcta gaatacccaa aa 42
<210> 569
   <211> 42
   <212> DNA
   <213> Artificial Sequence
<220>
   <223> Synthetic Polynucleotide
<400> 569
   gcacccagct actatcccaa tccaaataag ggagaattaa ct 42
<210> 570
   <211> 42
   <212> DNA
   <213> Artificial Sequence
<220>
   <223> Synthetic Polynucleotide
<400> 570
   attccaagaa cgaagcaaat cagatataga agttgctatt tt 42
<210> 571
   <211> 42
   <212> DNA
   <213> Artificial Sequence
<220>
   <223> Synthetic Polynucleotide
<400> 571
   aggcattttc gatgcagaac gcgcctgttt tctttcctta tc 42
<210> 572
   <211> 42
   <212> DNA
   <213> Artificial Sequence
<220>
   <223> Synthetic Polynucleotide
<400> 572
   cttctgacct aactgtttag tatcatatgc taatttaggc ag 42
<210> 573
   <211> 42
   <212> DNA
   <213> Artificial Sequence
<220>
   <223> Synthetic Polynucleotide
<400> 573
   cttagaatcc tttttaacct ccggcttagg agttaatttc at 42
<210> 574
   <211> 42
   <212> DNA
   <213> Artificial Sequence
<220>
   <223> Synthetic Polynucleotide
<400> 574
   gaataccaag ttattacatt taacaatttc aattaatttt cc 42
<210> 575
   <211> 42
   <212> DNA
   <213> Artificial Sequence
<220>
   <223> Synthetic Polynucleotide
<400> 575
   tcagatgatg gccgtaaaac agaaataaag cctgattgct tt 42
<210> 576
   <211> 42
   <212> DNA
   <213> Artificial Sequence
<220>
   <223> Synthetic Polynucleotide
<400> 576
   tctttaggag cacgtattaa atcctttgcc tattcctgat ta 42
<210> 577
   <211> 42
   <212> DNA
   <213> Artificial Sequence
<220>
   <223> Synthetic Polynucleotide
<400> 577
   aattccacac aagggccgtt ttcacggtca tcagacgatc ca 42
<210> 578
   <211> 42
   <212> DNA
   <213> Artificial Sequence
<220>
   <223> Synthetic Polynucleotide
<400> 578
   gcgcagtgtc acccgggtca ctgttgccct ccagcatcag cg 42
<210> 579
   <211> 42
   <212> DNA
   <213> Artificial Sequence
<220>
   <223> Synthetic Polynucleotide
<400> 579
   gggtcattgc aggccagagc acatcctcat aaacgatgct ga 42
<210> 580
   <211> 42
   <212> DNA
   <213> Artificial Sequence
<220>
   <223> Synthetic Polynucleotide
<400> 580
   ttgccgttcc ggacggaaaa agagacgcag cgccatgttt ac 42
<210> 581
   <211> 42
   <212> DNA
   <213> Artificial Sequence
<220>
   <223> Synthetic Polynucleotide
<400> 581
   cagtcccgga atatgtgctg caaggcgatt gcgcaactgt tg 42
<210> 582
   <211> 42
   <212> DNA
   <213> Artificial Sequence
<220>
   <223> Synthetic Polynucleotide
<400> 582
   ggaagggcga tcgtagatgg gcgcatcgta acaacccgtc gg 42
<210> 583
   <211> 42
   <212> DNA
   <213> Artificial Sequence
<220>
   <223> Synthetic Polynucleotide
<400> 583
   attctccgtg ggttgtaaac gttaatatta tatgtacccc gg 42
<210> 584
   <211> 42
   <212> DNA
   <213> Artificial Sequence
<220>
   <223> Synthetic Polynucleotide
<400> 584
   ttgataatca gaattcaacc gttctagctt aatgtgtagg ta 42
<210> 585
   <211> 42
   <212> DNA
   <213> Artificial Sequence
<220>
   <223> Synthetic Polynucleotide
<400> 585
   aagattcaaa aggcaataaa gcctcagagc tactaatagt ag 42
<210> 586
   <211> 42
   <212> DNA
   <213> Artificial Sequence
<220>
   <223> Synthetic Polynucleotide
<400> 586
   tagcattaac ataagtttca ttccatatac ttaattgctg aa 42
<210> 587
   <211> 42
   <212> DNA
   <213> Artificial Sequence
<220>
   <223> Synthetic Polynucleotide
<400> 587
   tataatgctg tagaagcccg aaagacttca aaatcaggtc tt 42
<210> 588
   <211> 42
   <212> DNA
   <213> Artificial Sequence
<220>
   <223> Synthetic Polynucleotide
<400> 588
   taccctgact atttgcaaaa gaagttttgg taagagcaac ac 42
<210> 589
   <211> 42
   <212> DNA
   <213> Artificial Sequence
<220>
   <223> Synthetic Polynucleotide
<400> 589
   tatcataacc ctgacgttgg gaagaaaaaa atttcaactt ta 42
<210> 590
   <211> 42
   <212> DNA
   <213> Artificial Sequence
<220>
   <223> Synthetic Polynucleotide
<400> 590
   atcattgtga atggctgacc ttcatcaaga agggaaccga ac 42
<210> 591
   <211> 42
   <212> DNA
   <213> Artificial Sequence
<220>
   <223> Synthetic Polynucleotide
<400> 591
   tgaccaactt tgacactcat ctttgacccg gtaaaatacg ta 42
<210> 592
   <211> 42
   <212> DNA
   <213> Artificial Sequence
<220>
   <223> Synthetic Polynucleotide
<400> 592
   atgccactac gacgctgagg cttgcagggg cttgataccg at 42
<210> 593
   <211> 42
   <212> DNA
   <213> Artificial Sequence
<220>
   <223> Synthetic Polynucleotide
<400> 593
   agttgcgccg acgcggagtg agaatagaag tctttccaga cg 42
<210> 594
   <211> 42
   <212> DNA
   <213> Artificial Sequence
<220>
   <223> Synthetic Polynucleotide
<400> 594
   ttagtaaatg aaccaccacc ctcattttca ccgtactcag ga 42
<210> 595
   <211> 42
   <212> DNA
   <213> Artificial Sequence
<220>
   <223> Synthetic Polynucleotide
<400> 595
   ggtttagtac cgaacctatt attctgaaat ttaacggggt ca 42
<210> 596
   <211> 42
   <212> DNA
   <213> Artificial Sequence
<220>
   <223> Synthetic Polynucleotide
<400> 596
   gtgccttgag taggcaggtc agacgattgg ccaccaccct ca 42
<210> 597
   <211> 42
   <212> DNA
   <213> Artificial Sequence
<220>
   <223> Synthetic Polynucleotide
<400> 597
   gagccgccac catgtagcgc gttttcatca atgaaaccat cg 42
<210> 598
   <211> 42
   <212> DNA
   <213> Artificial Sequence
<220>
   <223> Synthetic Polynucleotide
<400> 598
   atagcagcac cggattgagg gagggaaggt ttgtcacaat ca 42
<210> 599
   <211> 42
   <212> DNA
   <213> Artificial Sequence
<220>
   <223> Synthetic Polynucleotide
<400> 599
   atagaaaatt cagaaacgca ataataacgt cttaccgaag cc 42
<210> 600
   <211> 42
   <212> DNA
   <213> Artificial Sequence
<220>
   <223> Synthetic Polynucleotide
<400> 600
   ctttttaaga aagggaagcg cattagacga aacgattttt tg 42
<210> 601
   <211> 42
   <212> DNA
   <213> Artificial Sequence
<220>
   <223> Synthetic Polynucleotide
<400> 601
   tttaacgtca aaagccttaa atcaagatta ggcttatccg gt 42
<210> 602
   <211> 42
   <212> DNA
   <213> Artificial Sequence
<220>
   <223> Synthetic Polynucleotide
<400> 602
   attctaagaa cgcaatcaat aatcggctga tcaacaatag at 42
<210> 603
   <211> 42
   <212> DNA
   <213> Artificial Sequence
<220>
   <223> Synthetic Polynucleotide
<400> 603
   aagtcctgaa cattaacaac gccaacatgg ttatacaaat tc 42
<210> 604
   <211> 42
   <212> DNA
   <213> Artificial Sequence
<220>
   <223> Synthetic Polynucleotide
<400> 604
   ttaccagtat aatttttcaa atatattttt tgggttatat aa 42
<210> 605
   <211> 42
   <212> DNA
   <213> Artificial Sequence
<220>
   <223> Synthetic Polynucleotide
<400> 605
   ctatatgtaa attgtaaatc gtcgctatta tttgaattac ct 42
<210> 606
   <211> 42
   <212> DNA
   <213> Artificial Sequence
<220>
   <223> Synthetic Polynucleotide
<400> 606
   tttttaatgg aaaaacaata acggattcga aattgcgtag at 42
<210> 607
   <211> 42
   <212> DNA
   <213> Artificial Sequence
<220>
   <223> Synthetic Polynucleotide
<400> 607
   tttcaggttt aagagcggaa ttatcatcac gaacgttatt aa 42
<210> 608
   <211> 31
   <212> DNA
   <213> Artificial Sequence
<220>
   <223> Synthetic Polynucleotide
<400> 608
   ttttaaaagt ttaaaggaat tgagtaaaat a 31
<210> 609
   <211> 32
   <212> DNA
   <213> Artificial Sequence
<220>
   <223> Synthetic Polynucleotide
<400> 609
   cggaagcata aagtgtaatt gaggatcccc gg 32
<210> 610
   <211> 42
   <212> DNA
   <213> Artificial Sequence
<220>
   <223> Synthetic Polynucleotide
<400> 610
   gtgcactctg tggtctcaca ttaattgctt cagcaaatcg tt 42
<210> 611
   <211> 42
   <212> DNA
   <213> Artificial Sequence
<220>
   <223> Synthetic Polynucleotide
<400> 611
   cactcaatcc gccgggaaac ctgtcgtggc aagaatgcca ac 42
<210> 612
   <211> 42
   <212> DNA
   <213> Artificial Sequence
<220>
   <223> Synthetic Polynucleotide
<400> 612
   tccgtttttt cgtcgcgggg agaggcggac atcgacataa aa 42
<210> 613
   <211> 42
   <212> DNA
   <213> Artificial Sequence
<220>
   <223> Synthetic Polynucleotide
<400> 613
   atagactttc tccgtctttt caccagtgag ctttcagagg tg 42
<210> 614
   <211> 42
   <212> DNA
   <213> Artificial Sequence
<220>
   <223> Synthetic Polynucleotide
<400> 614
   ctcttcgcta ttaccgcctg gccctgagcc agccagcttt cc 42
<210> 615
   <211> 42
   <212> DNA
   <213> Artificial Sequence
<220>
   <223> Synthetic Polynucleotide
<400> 615
   cggattgacc gtaattgccc cagcaggcca tcaaaaataa tt 42
<210> 616
   <211> 42
   <212> DNA
   <213> Artificial Sequence
<220>
   <223> Synthetic Polynucleotide
<400> 616
   aaaacaggaa gattatcggc aaaatccctt gcctgagagt ct 42
<210> 617
   <211> 42
   <212> DNA
   <213> Artificial Sequence
<220>
   <223> Synthetic Polynucleotide
<400> 617
   ggccggagac agtcgggttg agtgttgtgc ctttatttca ac 42
<210> 618
   <211> 42
   <212> DNA
   <213> Artificial Sequence
<220>
   <223> Synthetic Polynucleotide
<400> 618
   catacaggca aggcaagaac gtggactcaa atggtcaata ac 42
<210> 619
   <211> 42
   <212> DNA
   <213> Artificial Sequence
<220>
   <223> Synthetic Polynucleotide
<400> 619
   gttttaaata tgcacagggc gatggccctc aggattagag ag 42
<210> 620
   <211> 42
   <212> DNA
   <213> Artificial Sequence
<220>
   <223> Synthetic Polynucleotide
<400> 620
   aagcaaagcg gatttttttg gggtcgagta aatattcatt ga 42
<210> 621
   <211> 42
   <212> DNA
   <213> Artificial Sequence
<220>
   <223> Synthetic Polynucleotide
<400> 621
   gacgacgata aaaaaaaggg agcccccgtg agatttagga at 42
<210> 622
   <211> 42
   <212> DNA
   <213> Artificial Sequence
<220>
   <223> Synthetic Polynucleotide
<400> 622
   cgattttaag aactaacgtg gcgagaaaca gtgaataagg ct 42
<210> 623
   <211> 42
   <212> DNA
   <213> Artificial Sequence
<220>
   <223> Synthetic Polynucleotide
<400> 623
   agatgaacgg tgtagctagg gcgctggcat tgtgtcgaaa tc 42
<210> 624
   <211> 42
   <212> DNA
   <213> Artificial Sequence
<220>
   <223> Synthetic Polynucleotide
<400> 624
   cctaaaacga aagaaccaca cccgccgcgg gtagcaacgg ct 42
<210> 625
   <211> 42
   <212> DNA
   <213> Artificial Sequence
<220>
   <223> Synthetic Polynucleotide
<400> 625
   aaccatcgcc cacgtatggt tgctttgagg ctccaaaagg ag 42
<210> 626
   <211> 42
   <212> DNA
   <213> Artificial Sequence
<220>
   <223> Synthetic Polynucleotide
<400> 626
   tgggattttg ctaaagaatc agagcgggaa ctacaacgcc tg 42
<210> 627
   <211> 42
   <212> DNA
   <213> Artificial Sequence
<220>
   <223> Synthetic Polynucleotide
<400> 627
   gaaccgccac cctctttaga caggaacgct cagtaccagg cg 42
<210> 628
   <211> 42
   <212> DNA
   <213> Artificial Sequence
<220>
   <223> Synthetic Polynucleotide
<400> 628
   gtataaacag ttaaataatc agtgaggcga atttaccgtt cc 42
<210> 629
   <211> 42
   <212> DNA
   <213> Artificial Sequence
<220>
   <223> Synthetic Polynucleotide
<400> 629
   ccagagccgc cgcccaaatt aaccgttggg aaccagagcc ac 42
<210> 630
   <211> 42
   <212> DNA
   <213> Artificial Sequence
<220>
   <223> Synthetic Polynucleotide
<400> 630
   gcgacagaat caagatcact tgcctgagtt gggaattaga gc 42
<210> 631
   <211> 42
   <212> DNA
   <213> Artificial Sequence
<220>
   <223> Synthetic Polynucleotide
<400> 631
   ccagcgccaa agacggtaat atccagaaag aaaatacata ca 42
<210> 632
   <211> 42
   <212> DNA
   <213> Artificial Sequence
<220>
   <223> Synthetic Polynucleotide
<400> 632
   atagccgaac aaagaaaaac gctcatggga taacccacaa ga 42
<210> 633
   <211> 42
   <212> DNA
   <213> Artificial Sequence
<220>
   <223> Synthetic Polynucleotide
<400> 633
   agcagccttt acagctgaaa tggattatct ttccagagcc ta 42
<210> 634
   <211> 42
   <212> DNA
   <213> Artificial Sequence
<220>
   <223> Synthetic Polynucleotide
<400> 634
   ttagcgaacc tcccaccagt aataaaagca agccgttttt at 42
<210> 635
   <211> 42
   <212> DNA
   <213> Artificial Sequence
<220>
   <223> Synthetic Polynucleotide
<400> 635
   atatcccatc ctaacttctg acctgaaata aagtaattct gt 42
<210> 636
   <211> 42
   <212> DNA
   <213> Artificial Sequence
<220>
   <223> Synthetic Polynucleotide
<400> 636
   tcaacagtag ggctttttga atggctatag gcgttaaata ag 42
<210> 637
   <211> 42
   <212> DNA
   <213> Artificial Sequence
<220>
   <223> Synthetic Polynucleotide
<400> 637
   aatccaatcg caagtaaaac atcgccatag agtcaatagt ga 42
<210> 638
   <211> 42
   <212> DNA
   <213> Artificial Sequence
<220>
   <223> Synthetic Polynucleotide
<400> 638
   aaatcaatat atgtagataa aacagaggac ctgagcaaaa ga 42
<210> 639
   <211> 42
   <212> DNA
   <213> Artificial Sequence
<220>
   <223> Synthetic Polynucleotide
<400> 639
   aatatacagt aacaaacagt gccacgcttt ctgaataatg ga 42
<210> 640
   <211> 42
   <212> DNA
   <213> Artificial Sequence
<220>
   <223> Synthetic Polynucleotide
<400> 640
   tatcattttg cggaagcatc accttgctac atttgaggat tt 42
<210> 641
   <211> 42
   <212> DNA
   <213> Artificial Sequence
<220>
   <223> Synthetic Polynucleotide
<400> 641
   aatgagtgag ctaagctgcg gccagaatgc ggccatacga gc 42
<210> 642
   <211> 42
   <212> DNA
   <213> Artificial Sequence
<220>
   <223> Synthetic Polynucleotide
<400> 642
   ccgctttcca gtcgggcgcg gttgcggtat gagtgcgcgc ct 42
<210> 643
   <211> 42
   <212> DNA
   <213> Artificial Sequence
<220>
   <223> Synthetic Polynucleotide
<400> 643
   gaatcggcca acgctcgtcg ctggcagcct ccggcgcttt cg 42
<210> 644
   <211> 42
   <212> DNA
   <213> Artificial Sequence
<220>
   <223> Synthetic Polynucleotide
<400> 644
   gccagggtgg tttttggtga agggatagct ctccaaacgc gg 42
<210> 645
   <211> 42
   <212> DNA
   <213> Artificial Sequence
<220>
   <223> Synthetic Polynucleotide
<400> 645
   tgattgccct tcacgccagc tggcgaaagg gggttgtgag ag 42
<210> 646
   <211> 42
   <212> DNA
   <213> Artificial Sequence
<220>
   <223> Synthetic Polynucleotide
<400> 646
   cggtccacgc tggttgggat aggtcacgtt ggtggtgcgg gc 42
<210> 647
   <211> 42
   <212> DNA
   <213> Artificial Sequence
<220>
   <223> Synthetic Polynucleotide
<400> 647
   atggtggttc cgaagtataa gcaaatattt aaaaacaaac gg 42
<210> 648
   <211> 42
   <212> DNA
   <213> Artificial Sequence
<220>
   <223> Synthetic Polynucleotide
<400> 648
   aatagcccga gataaaatca ccatcaatat gataaagccc ca 42
<210> 649
   <211> 42
   <212> DNA
   <213> Artificial Sequence
<220>
   <223> Synthetic Polynucleotide
<400> 649
   agagtccact attaaaagaa ttagcaaaat taaggtgaga aa 42
<210> 650
   <211> 42
   <212> DNA
   <213> Artificial Sequence
<220>
   <223> Synthetic Polynucleotide
<400> 650
   gaaaaaccgt ctatactaaa gtacggtgtc tggccaataa at 42
<210> 651
   <211> 42
   <212> DNA
   <213> Artificial Sequence
<220>
   <223> Synthetic Polynucleotide
<400> 651
   cacccaaatc aagtgcatca aaaagattaa gaggctcaac at 42
<210> 652
   <211> 42
   <212> DNA
   <213> Artificial Sequence
<220>
   <223> Synthetic Polynucleotide
<400> 652
   taaatcggaa ccctccaaaa tagcgagagg ctttatagtc ag 42
<210> 653
   <211> 42
   <212> DNA
   <213> Artificial Sequence
<220>
   <223> Synthetic Polynucleotide
<400> 653
   ggggaaagcc ggcgggctca ttataccagt cagcgtttac ca 42
<210> 654
   <211> 42
   <212> DNA
   <213> Artificial Sequence
<220>
   <223> Synthetic Polynucleotide
<400> 654
   cgaaaggagc gggccagacc aggcgcatag gcttacctta tg 42
<210> 655
   <211> 42
   <212> DNA
   <213> Artificial Sequence
<220>
   <223> Synthetic Polynucleotide
<400> 655
   cgctgcgcgt aaccggcaaa agaatacact aaaaaagagg ac 42
<210> 656
   <211> 42
   <212> DNA
   <213> Artificial Sequence
<220>
   <223> Synthetic Polynucleotide
<400> 656
   tacagggcgc gtaccataac cgatatattc ggtaggcacc aa 42
<210> 657
   <211> 42
   <212> DNA
   <213> Artificial Sequence
<220>
   <223> Synthetic Polynucleotide
<400> 657
   gtgctttcct cgttacaact ttcaacagtt tcaaatgaca ac 42
<210> 658
   <211> 42
   <212> DNA
   <213> Artificial Sequence
<220>
   <223> Synthetic Polynucleotide
<400> 658
   ccgattaaag ggatagaacc gccaccctca gagttttctg ta 42
<210> 659
   <211> 42
   <212> DNA
   <213> Artificial Sequence
<220>
   <223> Synthetic Polynucleotide
<400> 659
   tgagaagtgt tttttgcccc ctgcctattt cggccaccct ca 42
<210> 660
   <211> 42
   <212> DNA
   <213> Artificial Sequence
<220>
   <223> Synthetic Polynucleotide
<400> 660
   gtctgtccat cacgagcatt gacaggaggt tgaacagtgc cc 42
<210> 661
   <211> 42
   <212> DNA
   <213> Artificial Sequence
<220>
   <223> Synthetic Polynucleotide
<400> 661
   tgattagtaa taactttgcc tttagcgtca gacgaaccac ca 42
<210> 662
   <211> 42
   <212> DNA
   <213> Artificial Sequence
<220>
   <223> Synthetic Polynucleotide
<400> 662
   ctatcggcct tgctaaaagg gcgacattca acctaatcag ta 42
<210> 663
   <211> 42
   <212> DNA
   <213> Artificial Sequence
<220>
   <223> Synthetic Polynucleotide
<400> 663
   gccattgcaa caggttacca gaaggaaacc gagtatggtt ta 42
<210> 664
   <211> 42
   <212> DNA
   <213> Artificial Sequence
<220>
   <223> Synthetic Polynucleotide
<400> 664
   tgacgctcaa tcgtagagaa taacataaaa acaagtaagc ag 42
<210> 665
   <211> 42
   <212> DNA
   <213> Artificial Sequence
<220>
   <223> Synthetic Polynucleotide
<400> 665
   tcaccagtca cacggacttg cgggaggttt tgaaatgaaa at 42
<210> 666
   <211> 42
   <212> DNA
   <213> Artificial Sequence
<220>
   <223> Synthetic Polynucleotide
<400> 666
   acagagatag aacctttacg agcatgtaga aaccgaggcg tt 42
<210> 667
   <211> 42
   <212> DNA
   <213> Artificial Sequence
<220>
   <223> Synthetic Polynucleotide
<400> 667
   ggcacagaca atattaattg agaatcgcca tatagaaaaa ta 42
<210> 668
   <211> 42
   <212> DNA
   <213> Artificial Sequence
<220>
   <223> Synthetic Polynucleotide
<400> 668
   cgaactgata gcccacaaag aacgcgagaa aacagccaac gc 42
<210> 669
   <211> 42
   <212> DNA
   <213> Artificial Sequence
<220>
   <223> Synthetic Polynucleotide
<400> 669
   gaaccaccag cagagagtga ataaccttgc ttcgctgatg ca 42
<210> 670
   <211> 42
   <212> DNA
   <213> Artificial Sequence
<220>
   <223> Synthetic Polynucleotide
<400> 670
   ttaacaccgc ctgcgtacct tttacatcgg gagacagtac at 42
<210> 671
   <211> 42
   <212> DNA
   <213> Artificial Sequence
<220>
   <223> Synthetic Polynucleotide
<400> 671
   aatgaaaaat ctaaacaaag aaaccaccag aagcgtcaga tg 42
<210> 672
   <211> 42
   <212> DNA
   <213> Artificial Sequence
<220>
   <223> Synthetic Polynucleotide
<400> 672
   aaaccctcaa tcaagttggc aaatcaacag ttggagtaac at 42
<210> 673
   <211> 35
   <212> DNA
   <213> Artificial Sequence
<220>
   <223> Synthetic Polynucleotide
<400> 673
   cgctggttgg gataggtcac gttggtggtg cgggc 35
<210> 674
   <211> 35
   <212> DNA
   <213> Artificial Sequence
<220>
   <223> Synthetic Polynucleotide
<400> 674
   ttccgaagta taagcaaata tttaaaaaca aacgg 35
<210> 675
   <211> 35
   <212> DNA
   <213> Artificial Sequence
<220>
   <223> Synthetic Polynucleotide
<400> 675
   cgagataaaa tcaccatcaa tatgataaag cccca 35
<210> 676
   <211> 35
   <212> DNA
   <213> Artificial Sequence
<220>
   <223> Synthetic Polynucleotide
<400> 676
   actattaaaa gaattagcaa aattaaggtg agaaa 35
<210> 677
   <211> 35
   <212> DNA
   <213> Artificial Sequence
<220>
   <223> Synthetic Polynucleotide
<400> 677
   cgtctatact aaagtacggt gtctggccaa taaat 35
<210> 678
   <211> 32
   <212> DNA
   <213> Artificial Sequence
<220>
   <223> Synthetic Polynucleotide
<400> 678
   tggttgggat aggtcacgtt ggtggtgcgg gc 32
<210> 679
   <211> 32
   <212> DNA
   <213> Artificial Sequence
<220>
   <223> Synthetic Polynucleotide
<400> 679
   cgaagtataa gcaaatattt aaaaacaaac gg 32
<210> 680
   <211> 32
   <212> DNA
   <213> Artificial Sequence
<220>
   <223> Synthetic Polynucleotide
<400> 680
   gataaaatca ccatcaatat gataaagccc ca 32
<210> 681
   <211> 32
   <212> DNA
   <213> Artificial Sequence
<220>
   <223> Synthetic Polynucleotide
<400> 681
   attaaaagaa ttagcaaaat taaggtgaga aa 32
<210> 682
   <211> 32
   <212> DNA
   <213> Artificial Sequence
<220>
   <223> Synthetic Polynucleotide
<400> 682
   ctatactaaa gtacggtgtc tggccaataa at 32
<210> 683
   <211> 62
   <212> DNA
   <213> Artificial Sequence
<220>
   <223> Synthetic Polynucleotide
<400> 683
   tgcaatttaa ttcttttagc atttcaatat ttgtagattt gagaatttcg tttttttatt 60
   ca 62
<210> 684
   <211> 84
   <212> DNA
   <213> Artificial Sequence
<220>
   <223> Synthetic Polynucleotide
<400> 684
   tggttctgga gttttactcg ggacacttca gcgtaatatc ggaagcaggc actttgaaac 60
   ctataagtcc tgactattaa taac 84
<210> 685
   <211> 40
   <212> DNA
   <213> Artificial Sequence
<220>
   <223> Synthetic Polynucleotide
<400> 685
   ggctcccttt agggttccga tgatcctcaa ctgtgaggag 40
<210> 686
   <211> 40
   <212> DNA
   <213> Artificial Sequence
<220>
   <223> Synthetic Polynucleotide
<400> 686
   ctcctcacag ttgaggatca tcggaaccct aaagggagcc 40
<210> 687
   <211> 26
   <212> DNA
   <213> Artificial Sequence
<220>
   <223> Synthetic Polynucleotide
<220>
   <221> misc_feature
   <222> (9)..(17)
   <223> n is a, c, g, or t
<400> 687
   gcctggggnn nnnnnnntga gtgagc 26
<210> 688
   <211> 27
   <212> DNA
   <213> Artificial Sequence
<220>
   <223> Synthetic Polynucleotide
<220>
   <221> misc_feature
   <222> (11)..(19)
   <223> n is a, c, g, or t
<400> 688
   agctcactca nnnnnnnnnc cccaggc 27

## Description

### BACKGROUND

In nature, biomolecules assemble into hierarchical structures through intermolecular interactions. In synthetic biology, it is possible to rationally design biosynthetic building blocks, with hierarchical structures arising from built-in functionality at the molecular level controlling intermolecular interactions. Such biosynthetic self-assembling structures have useful applications in the field of nanotechnology, for example.

Han, D. et al., Science (2013), 339(6126):1412-1415, WO 2014/018675, Simmel, S.S. et al., Acc Chem Res (2014), 47(6):1691-1699, US 2007/117109 and Schlichthaerle, T. et al., Curr Opin Biotechnol (2016), 39:41-47 disclose self-assembling nucleic acid nanostructures.

### SUMMARY

Provided herein, in some embodiments, is a technology (including, for example, methods, compositions and kits) for controlling nucleation and hierarchical assembly (programmable self-assembly) of molecular structures, such as nucleic acid *(e.g.,* DNA) and/or protein nanostructures, microstructures, and macrostructures. This technology, referred to herein as 'crisscross cooperative assembly' can be used to program and rapidly assemble structures that only originate from provided macromolecular 'seeds,' thus may be considered a 'zero-background' assembly method. Through the design of cooperative binding sites on individual biomolecular subunits that require simultaneous engagement with a large number of other subunits to achieve stable attachment, the system imposes an intrinsically high energetic barrier against spontaneous nucleation of structures, even in the presence of high concentrations of each individual component. Nucleation can only be triggered by providing a macromolecular 'seed' that resembles a pre-existing structural interface (presents multiple weak binding sites for stable capture of the next subunit). Addition of a seed that can stably capture individual subunits effectively bypasses the activation energy barrier against spontaneous nucleation to drive higher-order assembly of a microscale structure. Components can be continually added to the structures such that their growth in one-dimension, two-dimensions or three-dimensions is potentially as large as for other polymerization or crystallization processes.

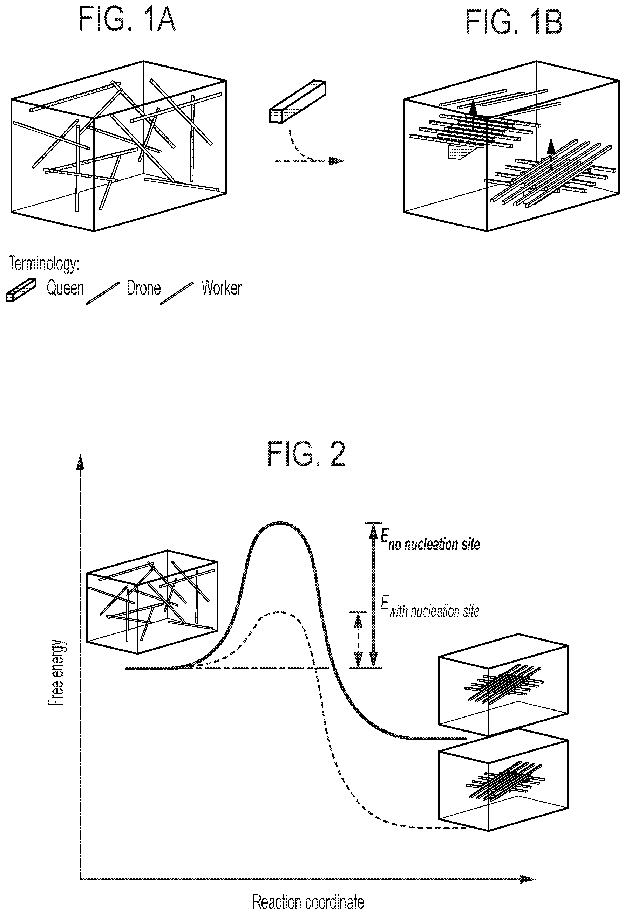

Crisscross cooperative assembly, as provided herein, uses molecular (*e.g.*, nucleic acid or protein) building blocks (FIG. 1A) that are programmed to self-assemble into crisscrossed layers (FIG. 1B). A building block, in some embodiments, may be a rod-shaped structure assembled from programmable nucleic acid hybridization interactions. As indicated above, this crisscross cooperative assembly technology uses a 'seed' structure from which programmable nucleic acid self-assembly begins. This seed structure is formed through irreversible interactions between a nucleating structure (FIG. 1A; 'queen') and a subset of building blocks (FIG. 1A; 'drones') that are aligned to form an initial seed layer along the nucleating structure. In the presence of a seed structure, another set of building blocks (FIG. 1A; 'workers') are added to the pre-existing seed layer (FIG. 1B). Binding between a sufficient number of building blocks (drones) and a nucleating structure (queen) to form a seed can trigger the addition of many additional layers of building blocks (workers), with each layer rotated by some degree *(e.g.,* 90°) relative to adjacent layers (above and/or below).

The nucleating structure and the building blocks are engineered to interact with (*e.g.,* bind to) each other based on a set of kinetic/nucleation energy parameters, as follows. An initial subset of building blocks (drones) should bind strongly (irreversibly/stably) to and form an aligned layer along the nucleating structure (queen). The building blocks (drones) of the initial subset should not interact with (bind to) each other. Likewise, building blocks (workers) of a subsequent subset should not interact with (bind to) each other. Further, in the absence of a nucleating structure (queen), any building block (drone) from the initial subset should have only one weak (reversible) interaction with any other building block (worker) from another subset. In the presence of a nucleating structure (queen), a single building block (drone) from an initial subset may interact with more than one building block (worker) from a subsequent subset, and a single building block (worker) from a subsequent subset may interact with more than one building block (drone) from the initial subset or another subset ('workers' of another subset). For example, with reference to FIG. 1B, a single building block *(e.g.,* DNA nanorod) may bind to eight other building blocks (*e.g.,* DNA nanorods), although the single building block binds to each of the eight building blocks only once to form two layers having a 'crisscross' pattern.

The single interaction between a building block (drone) from the initial subset and a building block from a subsequent subset (worker) should be weak enough such that there is an arbitrarily large entropy penalty against nucleation in the absence of a seed structure (a large number of individual workers would have to come together simultaneously). With these parameters, zero-background and minimal defects can be achieved, even at high concentrations of interacting building blocks, thereby enabling rapid nucleation and assembly of nucleic acid nanostructures.

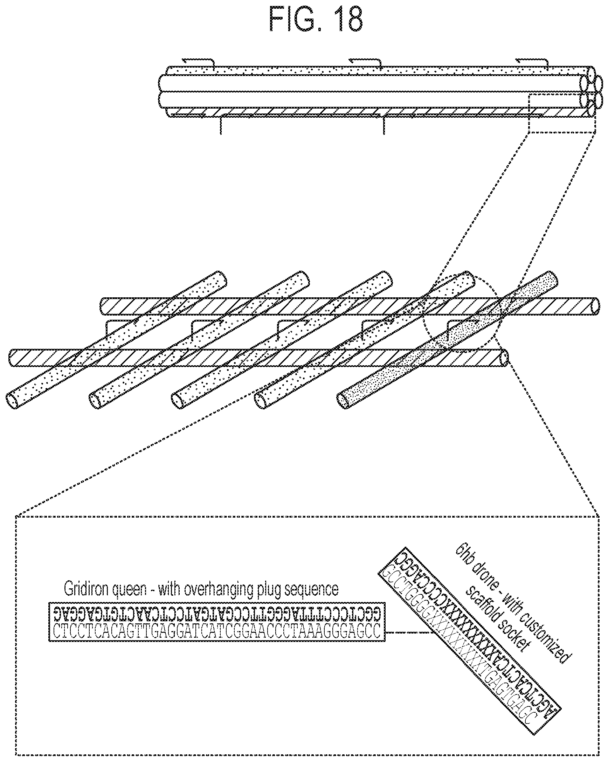

Provided are crisscross nucleic acid nanostructures, comprising a first nanorod comprising a first plug strand and a second plug strand; a second nanorod comprising a third plug strand and a fourth plug strand, wherein the second nanorod is parallel to the first nanorod; a third nanorod comprising a fifth plug strand complementary to and bound to the first plug strand and a sixth plug strand complementary to and bound to the second plug strand; a fourth nanorod comprising a seventh plug strand complementary to and bound to the third plug strand and an eighth plug strand complementary to and bound to the fourth plug strand, wherein the third nanorod is parallel to the fourth nanorod. See, e.g., FIG. 18. A crisscross nanostructure is not limited to 4 nanorods and, in many embodiments, includes at least 4 (e.g., at least 5, 10, 15, 20, 25, 50, 100 or more) nanorods arranged in a crisscross pattern as described herein.

Thus, in some embodiments, a crisscross nucleic acid nanostructure, comprises a first plurality of nanorods parallel to each other, and a second plurality of nanorods parallel to each other, wherein the nanorods of the first plurality are bound to and perpendicular to (or are non-parallel to) the nanorods of the second plurality. See, e.g., FIG. 18.

In some embodiments, each nanorod is comprised of DNA. For example, a nanorod may be comprised of a 6-helix DNA bundle (see, e.g., Douglas SM1, Chou JJ, Shih WM. DNA-nanotube-induced alignment of membrane proteins for NMR structure determination. Proc Natl Acad Sci U S A. 104, 6644-6648, 2007).

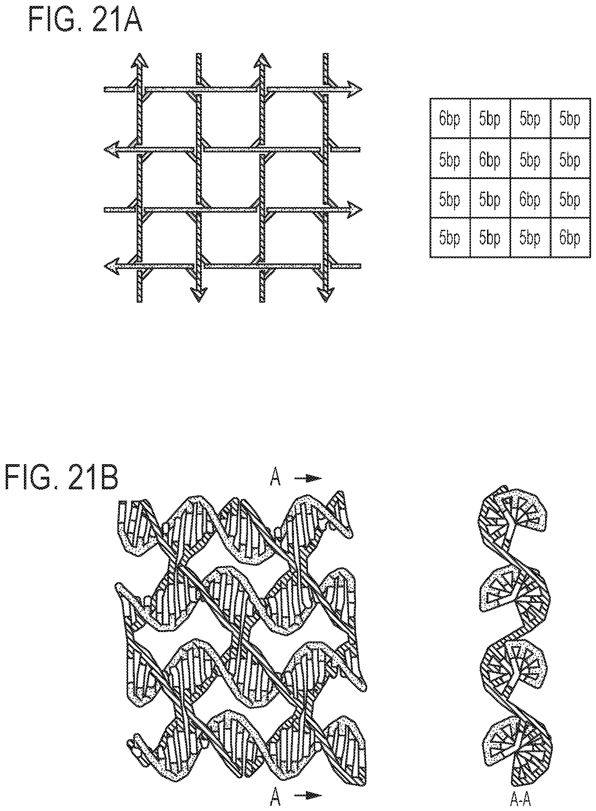

Also provided herein, in some aspects, are crisscross nucleic acid slats, comprising: a first plurality of at least four nucleic acid strands parallel to each other, each strand of the first plurality having a length of 20-100 nucleotides (e.g., 20-30, 20-40 or 20-50 nucleotides); and a second plurality of at least four nucleic acid strands parallel to each, each strand of the second plurality having a length of 20-100 nucleotides (e.g., 20-30, 20-40 or 20-50 nucleotides), wherein the at least four nucleic acid strands of the first plurality are bound to and perpendicular to the at least four nucleic acid strands of the second plurality. See, e.g., FIGS. 21A-21B.

Also provided herein, in some aspects are crisscross nucleic acid slats, comprising: a first plurality of at least four nucleic acid strands parallel to each other, each strand of the first plurality having a length of at least 21 nucleotides; and a second plurality of at least four nucleic acid strands parallel to each, each strand of the second plurality having a length of at least 21 nucleotides, wherein the at least four nucleic acid strands of the first plurality are bound to and perpendicular to the at least four nucleic acid strands of the second plurality. See, e.g., FIGS. 21A-21B.

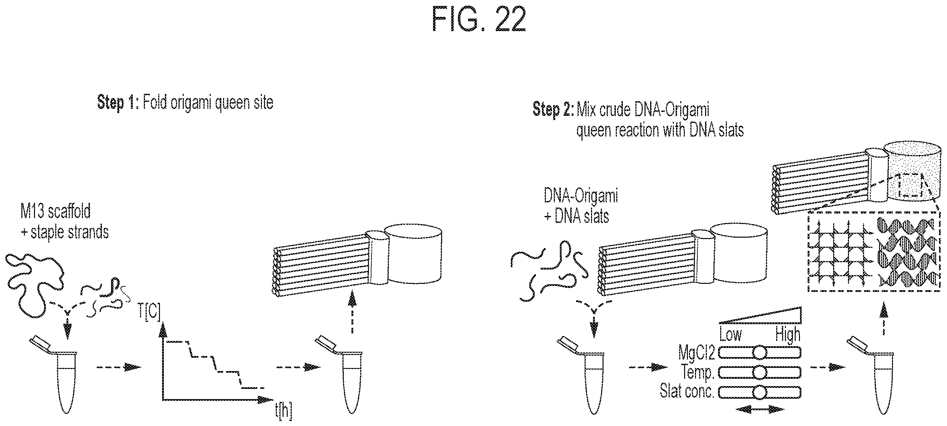

Further provided herein, in some aspects, are nucleic acid nanostructures comprising a nucleic acid scaffold strand folded (e.g., M13 or M13-derived) into repeating loop-like shapes (e.g., 5-15 loops, or 5, 6, 7, 8, 9 or 10 loops) secured by shorter nucleic acid staple strands, wherein the repeating loop structures are bound to at least one (e.g., at least 2, 3, 4,, 5, 10, 20, 25, 30, 35, 40, 45, 50, 60, 70, 80, 90, 100 or more) crisscross nucleic acid slat. See, e.g., FIGS. 22, 24A, 25 and 27B.

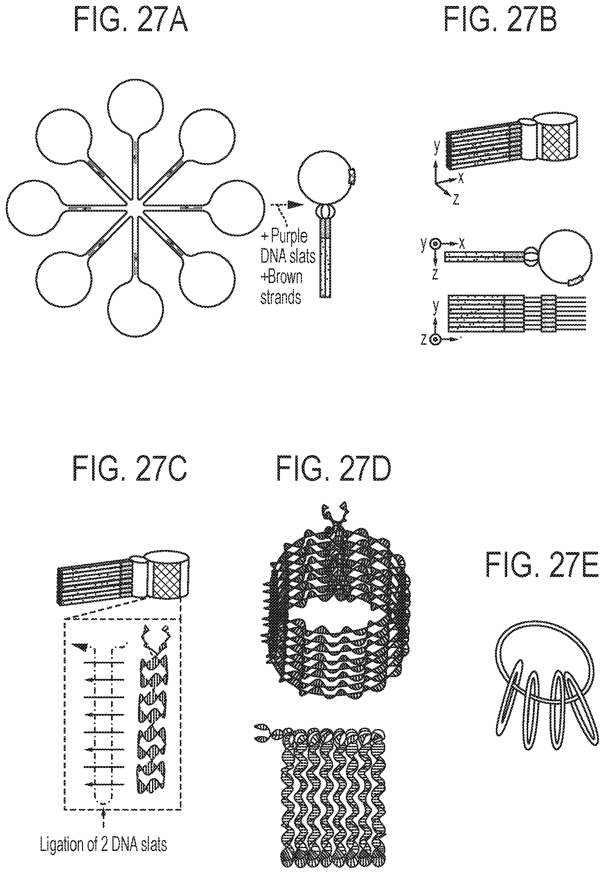

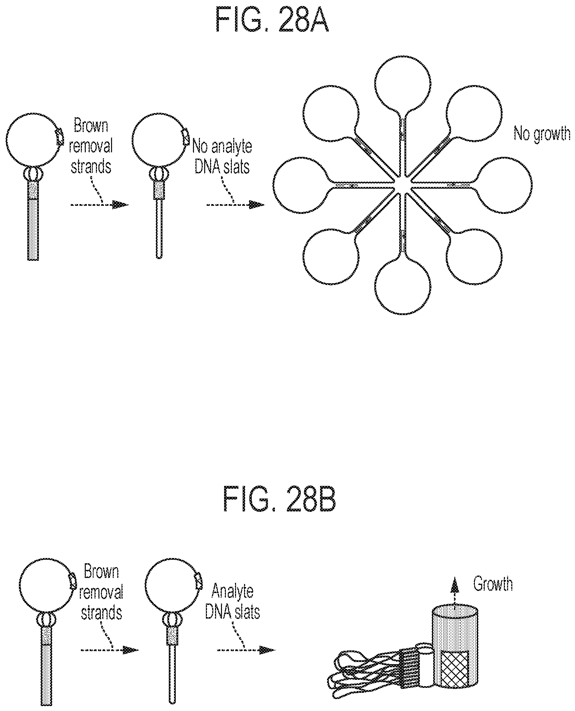

Further still, provided herein, in some aspects, are nucleic acid nanostructures, comprising a nucleic acid scaffold strand folded into repeating loop-like shapes secured by at least two crisscross nucleic acid slats. See, e.g., FIGS. 27B and 28B.

The present disclosure also provides, in some aspects, methods of producing a crisscross nucleic acid nanostructures, comprising: combining in a reaction mixture (a) a first nanorod comprising a first plug strand and a second plug strand, (b) a second nanorod comprising a third plug strand and a fourth plug strand, wherein the second nanorod is parallel to the first nanorod, (c) a third nanorod comprising a fifth plug strand complementary to and bound to the first plug strand and a sixth plug strand complementary to and bound to the second plug strand, and (d) a fourth nanorod comprising a seventh plug strand complementary to and bound to the third plug strand and an eighth plug strand complementary to and bound to the fourth plug strand, wherein the third nanorod is parallel to the fourth nanorod; and incubating the reaction mixture under conditions (e.g., nucleic acid hybridization conditions) that result in assembly of a crisscross nucleic acid nanostructure. See, e.g., FIG. 22.

Biomolecule (analyte) detection methods are also provided herein, in some aspects. In some embodiments, a method, comprises (a) combining in a reaction mixture (i) a sample comprising a biomolecule; (ii) a nucleic acid strand capable of self-assembling into a nanostructure that comprise vertically-stacked parallel strands; (iii) a plurality of oligonucleotides, shorter than the nucleic acid strand of (ii), wherein the oligonucleotides of (iii) bind to the strand of (ii) to assemble the vertically-stacked parallel strands; (iv) two crisscross nucleic acid slats, wherein the two slats bind to the strand of (ii), and wherein each of the slats is linked to a biomolecule binding partner that specifically binds to the biomolecule in the sample; (b) incubating the reaction mixture under conditions that permit binding of the biomolecule binding partners to the biomolecule and assembly of the nanostructure into vertically-stacked parallel strands; (c) removing the plurality of oligonucleotides of (iii) from the reaction mixture of (b); (d) incubating the reaction mixture of (c) in the presence of a plurality of crisscross nucleic acid slats of claim 27, wherein the crisscross nucleic acid slats bind to the vertically-stacked parallel strands to form a three-dimensional barrel structure. In some embodiments, the methods further comprise imaging the three-dimensional barrel structure. See, e.g., FIGS. 28A-28B.

In some embodiments, the methods may comprise combining in a reaction mixture (e.g., with hybridization buffer) (a) a sample comprising a biomolecule and (b) a nucleic acid nanostructure comprising (i) a nucleic acid scaffold strand capable of folding into repeating loop-like shapes (e.g., 2-15 vertically-stacked loops) and (ii) two crisscross nucleic acid slats, wherein a biomolecule binding partner (e.g., an antibody) that specifically binds to the biomolecule is linked to each of the crisscross nucleic acid slats such that in the presence of the cognate biomolecule the biomolecule binding partner binds to the biomolecule and the nucleic acid nanostructure folds into repeating loop-like shapes. See, e.g., FIGS. 28A-28B.

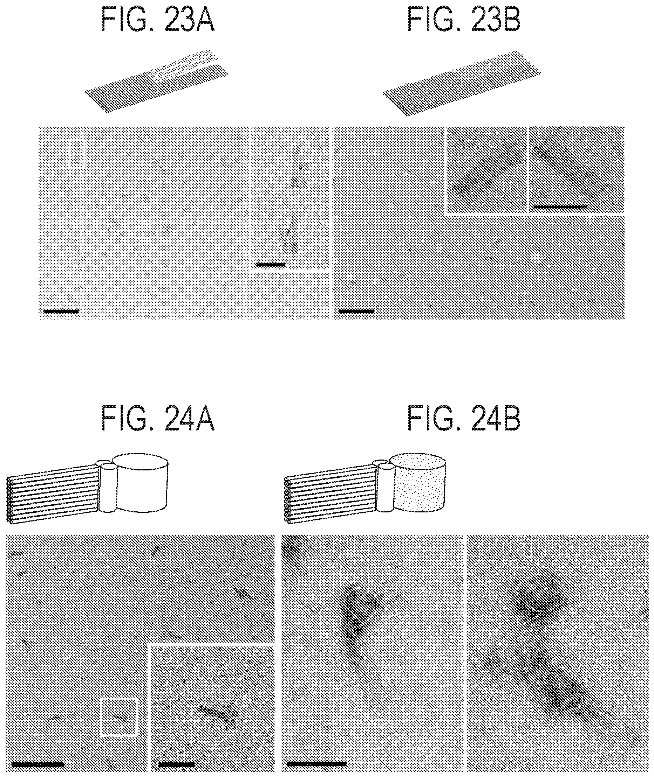

In some embodiments, the methods further comprise combining the reaction mixture with a plurality (e.g., 2-50 or 2-100) of crisscross nucleic acid slats to form a three-dimensional barrel-like structure. See, e.g., FIG. 24B.

It should be understood that the nucleic acid nanostructures as described herein (e.g., nanorods, slats, barrels, etc.) and variants thereof, as provided herein, may be designed, for example, using the following publicly-available tool described by Douglas SM, Marblestone AH, Teerapittayanon S, Vazquez A, Church GM, Shih WM. Rapid prototyping of 3D DNA-origami shapes with caDNAno. Nucleic Acids Res. 37, 5001-5006, 2009.

See, also, Douglas et al. Nature, 459(7245): 414-418, 2009. For example, and as described elsewhere herein, it is known in the art that custom shape (e.g., megadalton-scale) DNA nanostructures may be produced using a long 'scaffold' strand to template the assembly of hundreds of oligonucleotide 'staple' strands into a planar antiparallel array of cross-linked helices. This 'scaffolded DNA origami' method has also been adapted to produce 3D shapes formed as pleated layers of double helices constrained to a honeycomb lattice. caDNAno, an open-source software package with a graphical user interface, may be used to aid in the design of DNA sequences for folding 3D DNA (or other nucleic acid) nanostructures. The caDNAno software is available at cadnano.org, along with example designs and video tutorials demonstrating their construction.

### BRIEF DESCRIPTION OF THE DRAWINGS

FIGS. 1A-1B show an abstraction of an example of crisscross cooperative assembly system. FIG. 1A indicates the system without a nucleation site and no self-assembly. FIG. 1B indicates the system after the addition of the nucleation site and triggered spontaneous self-assembly. Growth direction is indicated by the grey arrows and shows a 1D growth in this example. Individual components are referred to as 'queen,' 'drone' and 'worker.'
FIG. 2 shows a graph depicting the principle by which the nucleation site (queen) structure initiates higher-order structures with drones and workers by lowering the activation energy for assembly.

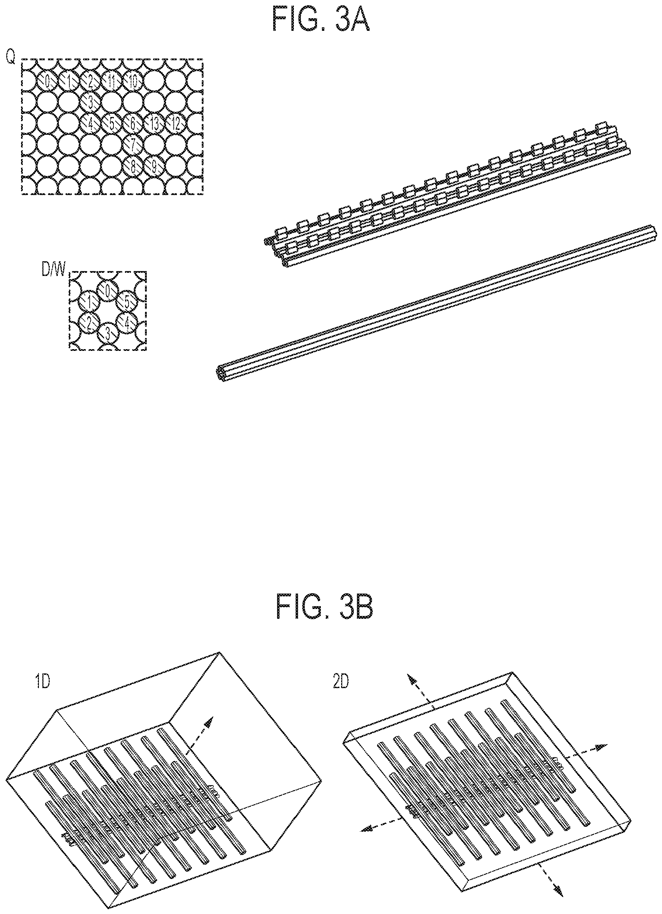

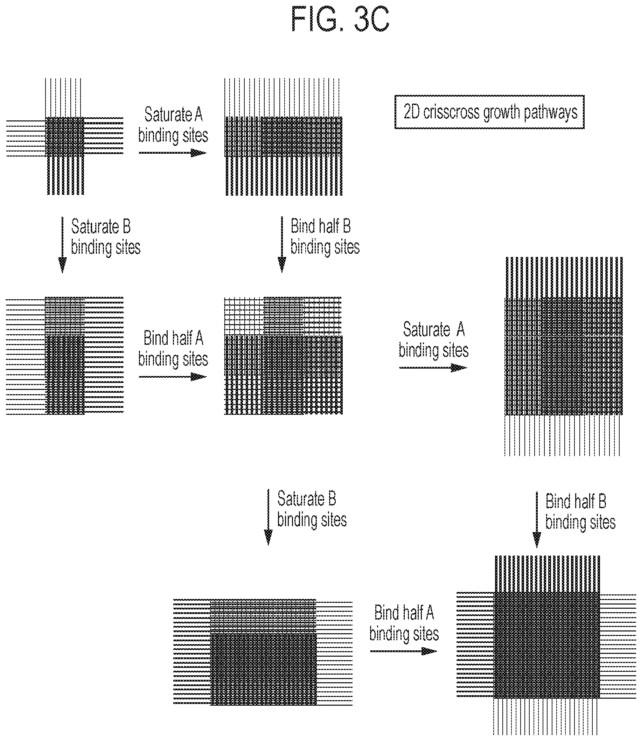

FIGS. 3A-3C show examples of DNA-origami crisscross assembly. FIG. 3A shows queen (Q) and drone/worker (D/W) architecture shown in cross-section (caDNAno software downloaded from cadnano.org) and in 3D representation. Each individual cylinder represents a double stranded DNA helix. FIG. 3B is a representation of 1D and 2D growth with the crisscross DNA-origami cooperative assembly. 3D growth can be achieved by creating a design that merges 1D and 2D growth. FIG. 3C shows different pathways for 2D growth.

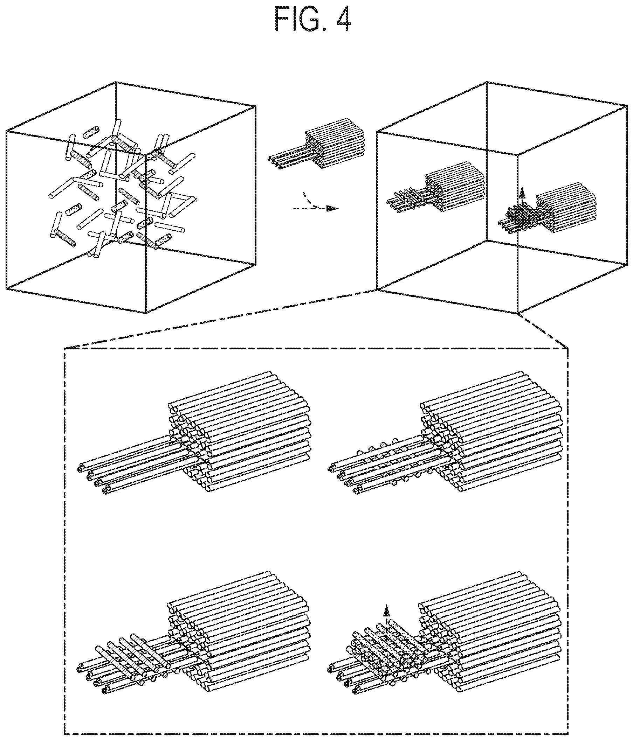

FIG. 4 shows an example of single-stranded DNA crisscross cooperative assembly. Oligonucleotides comprising the workers and drones of the system (shown as cylinders) are nucleated by the addition of a cubic DNA-Origami queen structure with a nucleation site. Stepwise assembly is shown in illustrated magnification.

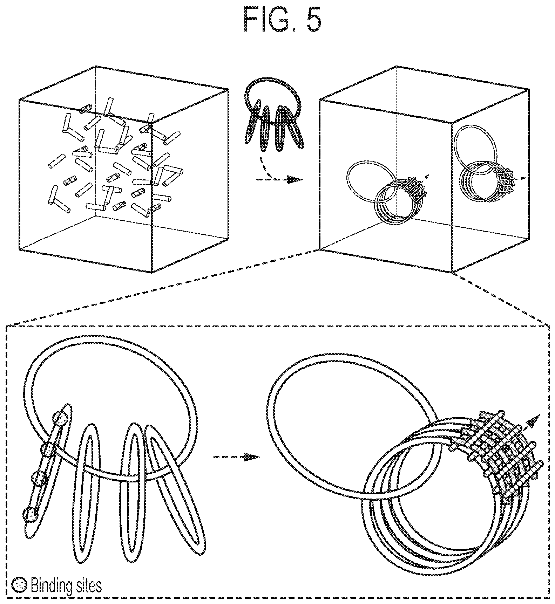

FIG. 5 shows an example of catenane crisscross cooperative assembly queen (catenane queen), useful for ultrasensitive detection. Oligonucleotides comprising the workers and drones of the system (shown as cylinders) are nucleated by the addition of a single-stranded catenane queen structure with a nucleation site. Binding sites on the structure shown to the left of the illustrated magnification indicate the nucleation for the workers/drones. Each host ring has multiple binding sites, collectively functioning as a cooperative binding site.

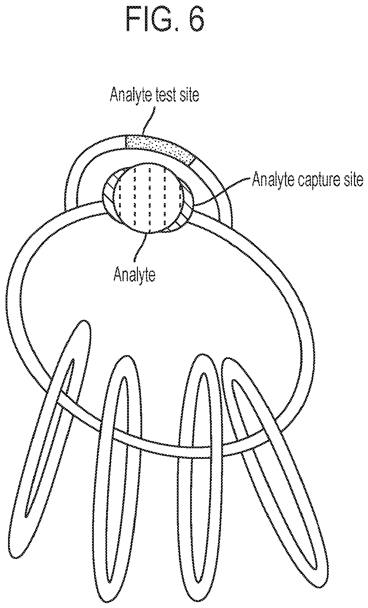

FIG. 6 shows a catenane queen from FIG. 5 that has been modified to serve as a biosensor. The large DNA ring has been split to incorporate and biomolecule capture site to bind a biomolecule (e.g., macromolecule) in biological samples. The presence of the biomolecule, in some embodiments may be detected in mixtures as follows: (1) A biological sample is mixed with a high concentration of the catenane queen and a biomolecule of interest binds the biomolecule capture site. (2) A chemical reaction is used to reversibly cleave the biomolecule capture site. (3) Catenane queens not bound to the target biomolecule fall apart more quickly compared to those held together by the target biomolecule. (4) Remaining catenane queens in the test mixture are re-ligated at the biomolecule capture site. (5) Drones and workers are added to the test mixture to amplify remaining queens using readily observable micrometer-scale DNA structures. This system is modular, as the biomolecule capture site may be customized to bind disease markers, including proteins or nucleic acid sequences.

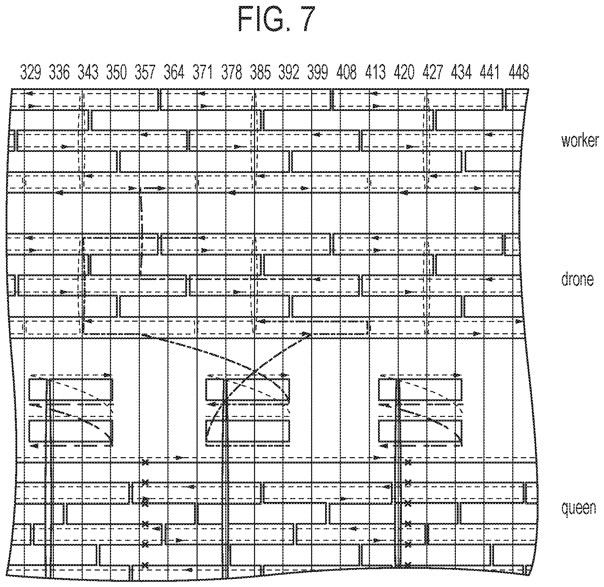

FIG. 7 shows a CAD schematic of an example of base-pairing linkages between a 6 helix bundle worker and a 6 helix bundle drone to queen. A plug socket linkage design may also be used, as shown in FIGS. 18-20. The following CAD tool was used to design the structures: Douglas SM, Marblestone AH, Teerapittayanon S, Vazquez A, Church GM, Shih WM. Rapid prototyping of 3D DNA-origami shapes with caDNAno. Nucleic Acids Res. 37, 5001-5006, 2009.

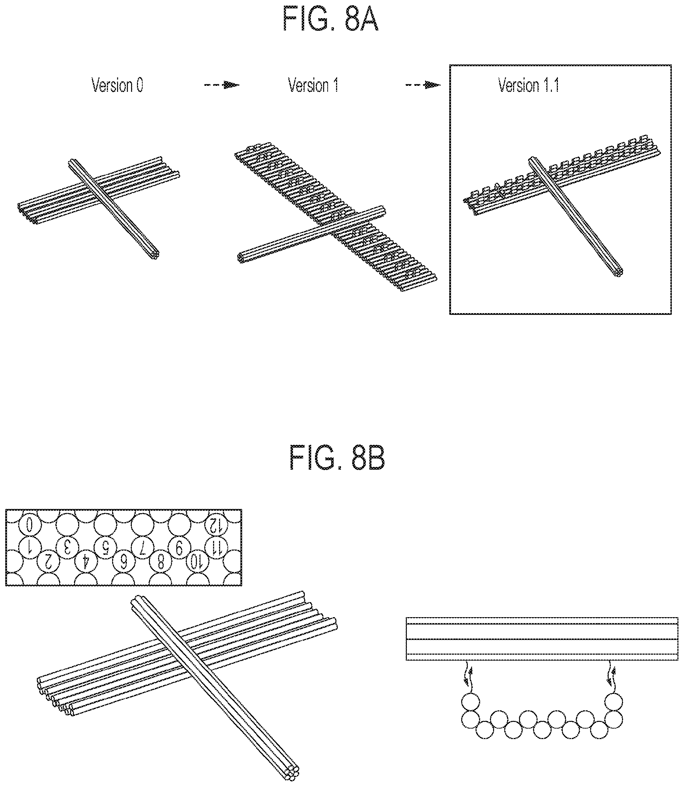

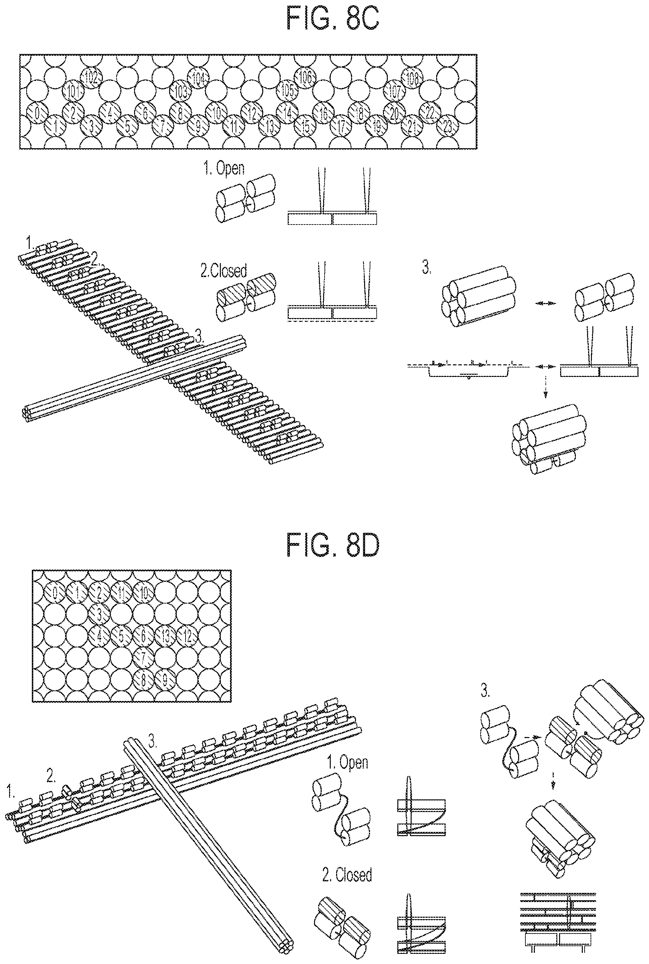

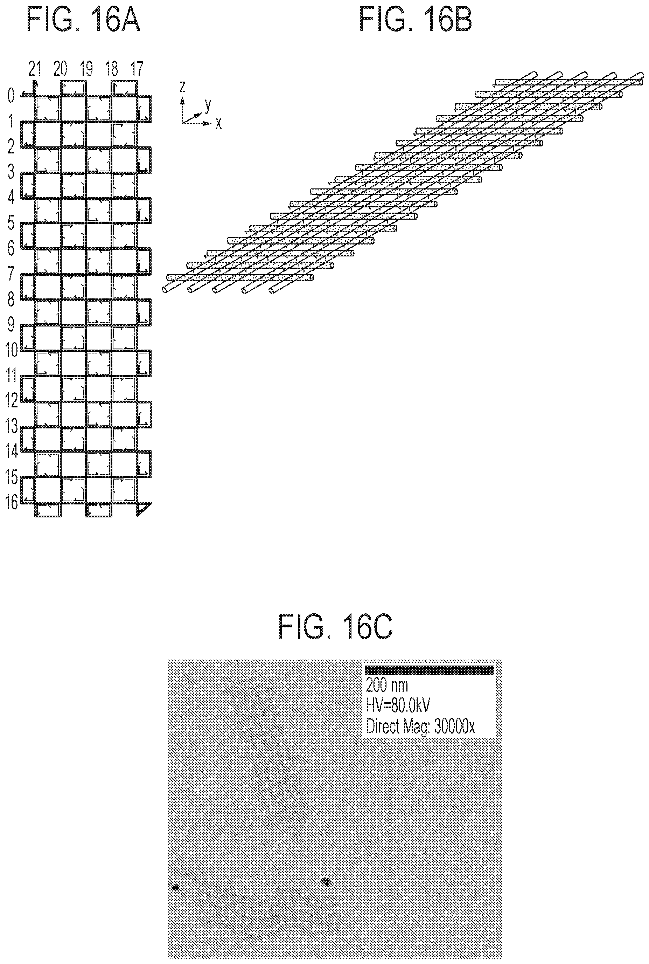

FIGS. 8A-8D are schematics depicting different example 'seed' designs (a 6-helix bundle nanorod bound to a nucleating nanostructure) with different cooperative bind site configurations. Additional example 'seed' designs are shown in FIGS. 16A-16C.

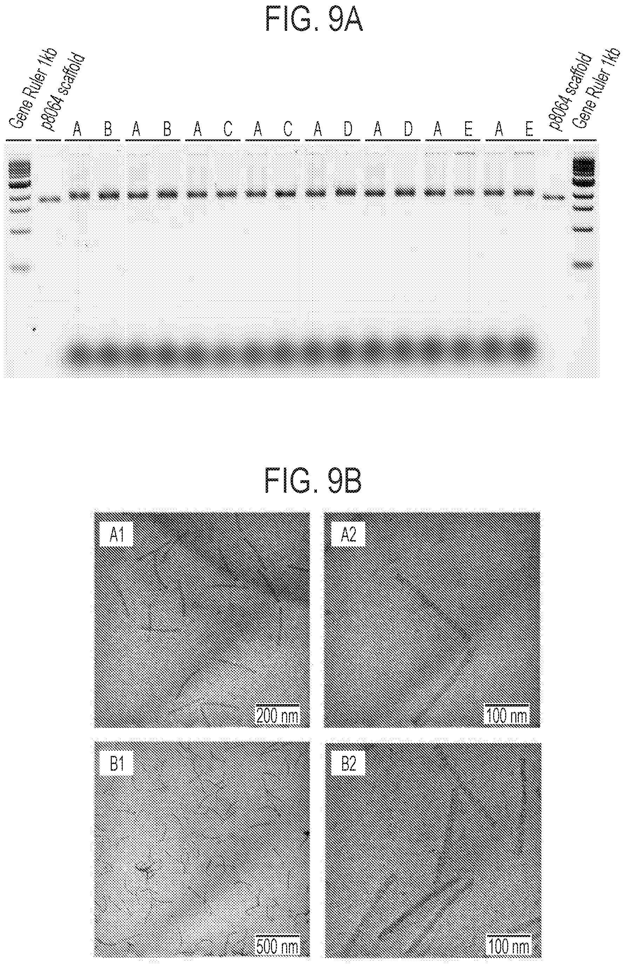

FIG. 9A-9B show results from a seesaw experiment with an example of a nucleating nanostructure (queen) folded at different temperatures (A: 65-60 °C; B: 60-55 °C; C: 65-55 °C; D: 60-50 °C) at a MgCl₂ concentration of 6 mM.

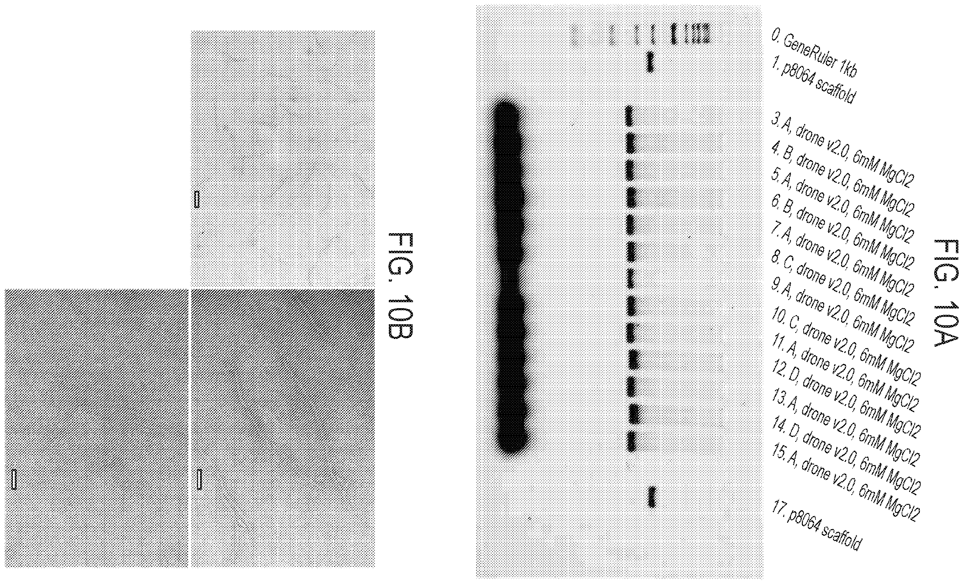

FIGS. 10A-10B show results from a seesaw experiment with an example of a nanostructure (drone) folded at different temperatures (A: 70-60 °C; B: 65-55 °C; C: 65-60 °C; D: 60-55 °C) at a MgCl₂ concentration of 6 mM.

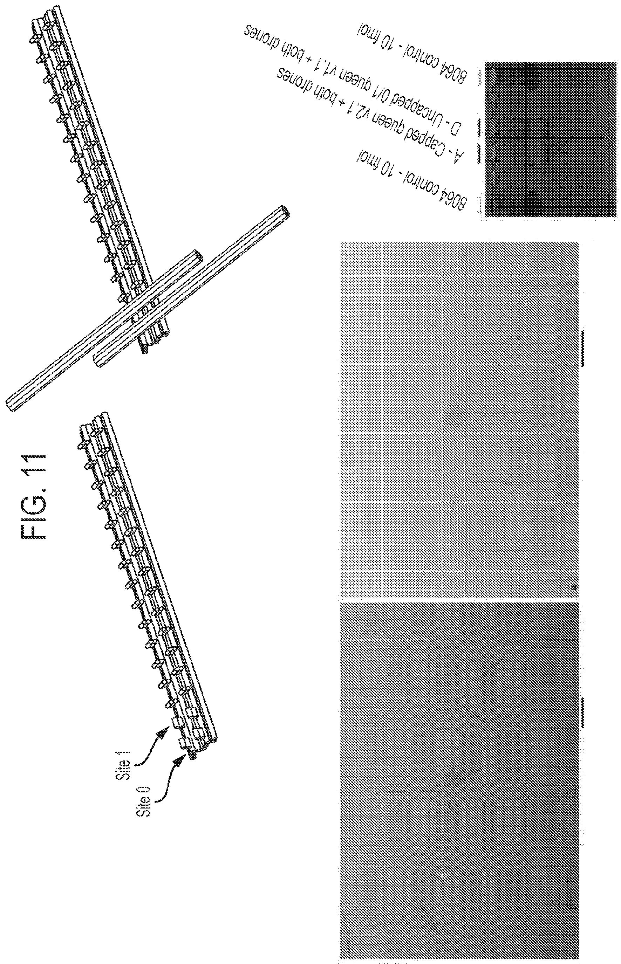

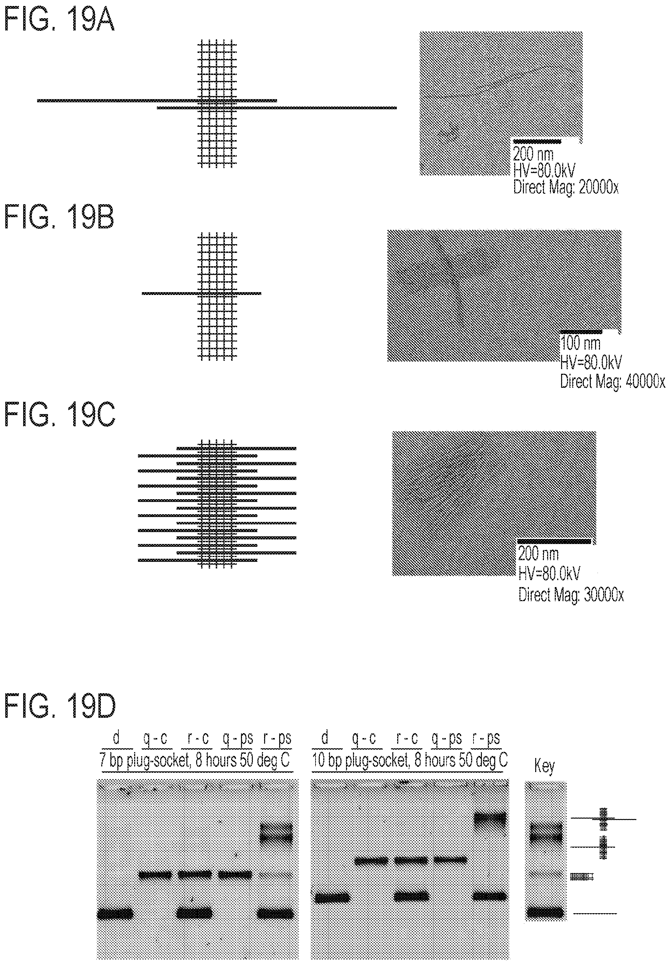

FIG. 11 shows a schematic depicting the assembly of an example seed structure (queen + drones). Images of the structures are also shown. Nanostructure assembly may also be carried out as shown in FIGS. 19A-19D.

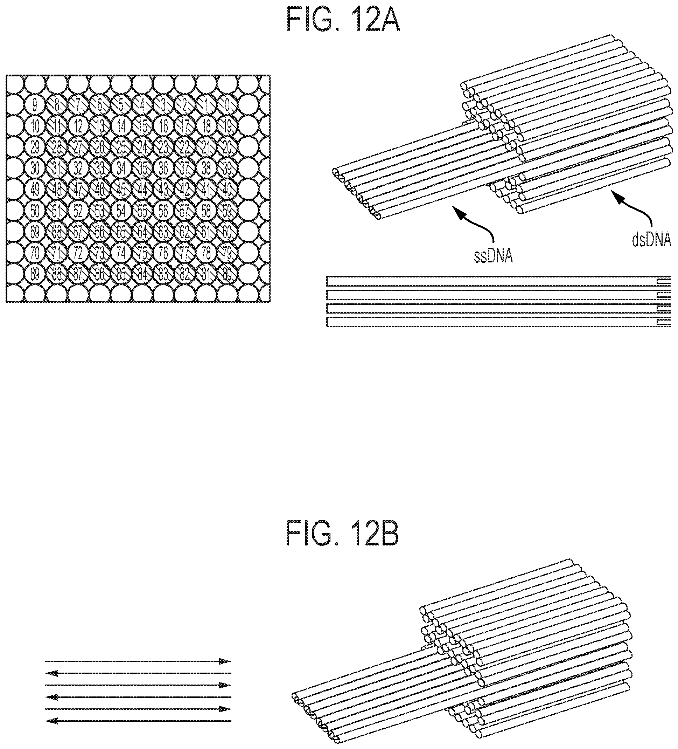

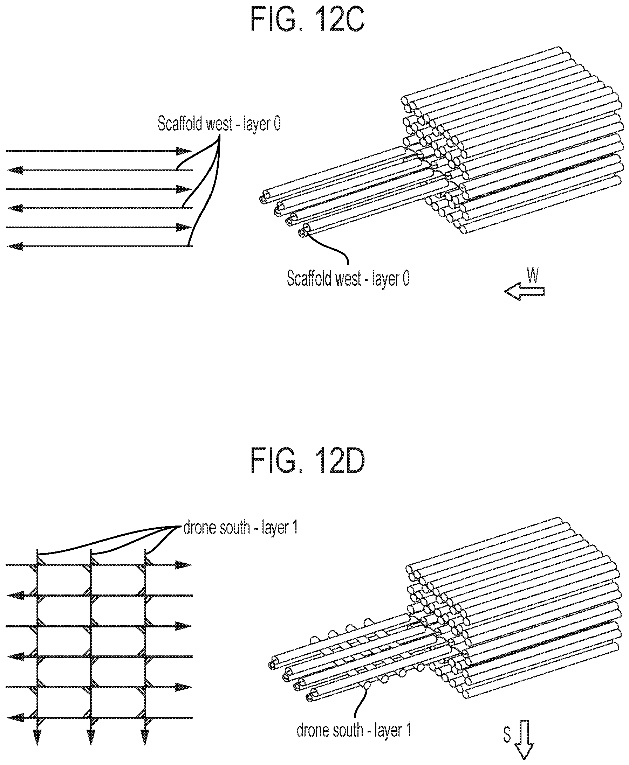

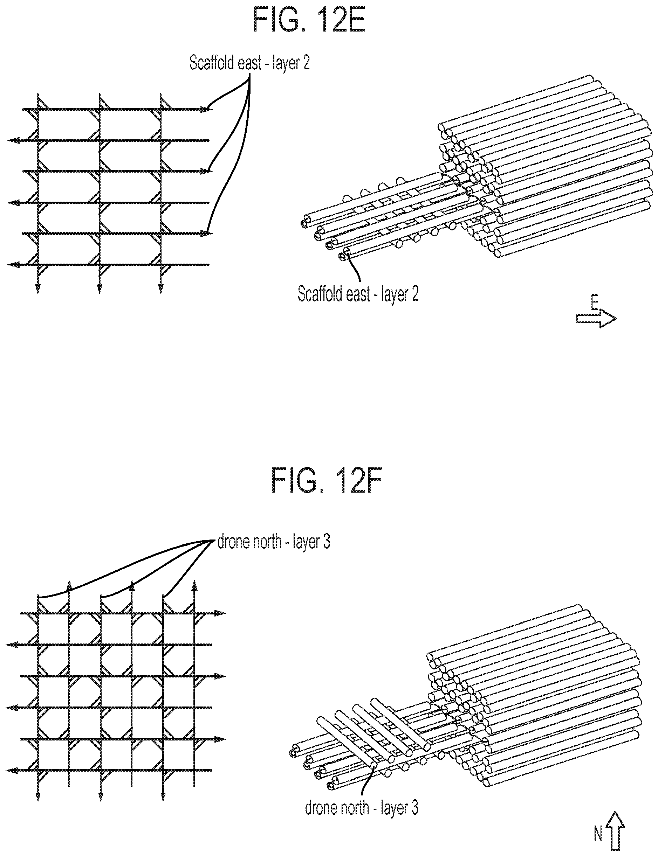

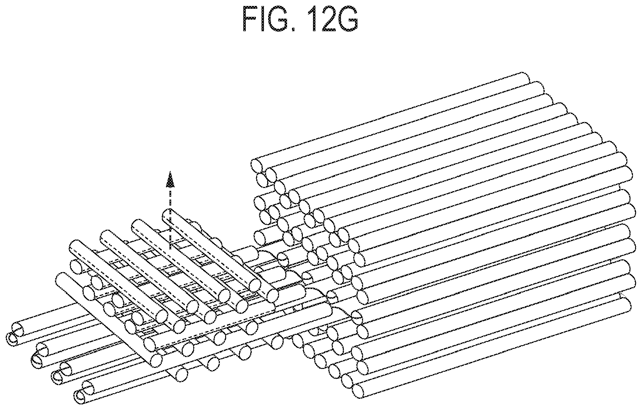

FIGS. 12A-12G show a seed structure forming from the assembly of a single-stranded DNA and additional nanostructures (drones).

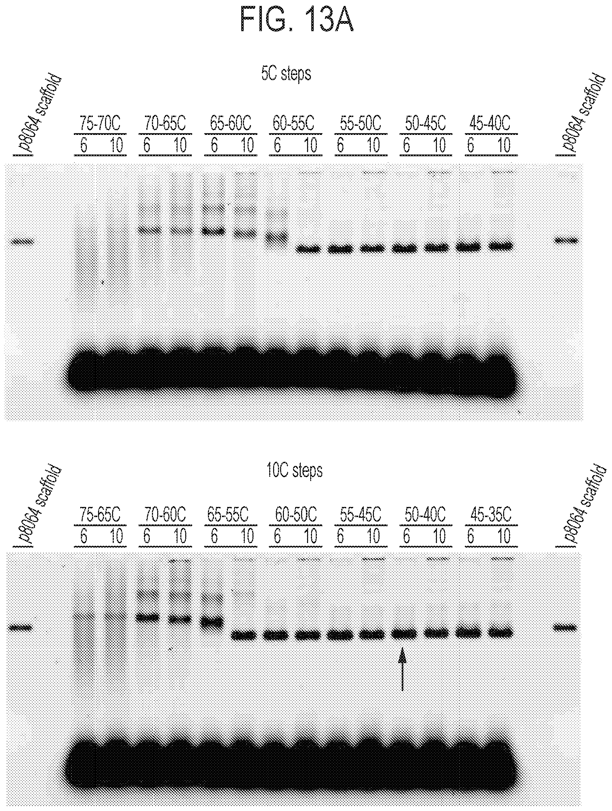

FIGS. 13A-13B show results from seesaw experiments with a single-stranded nucleating nanostructure (queen) at various temperatures and steps.

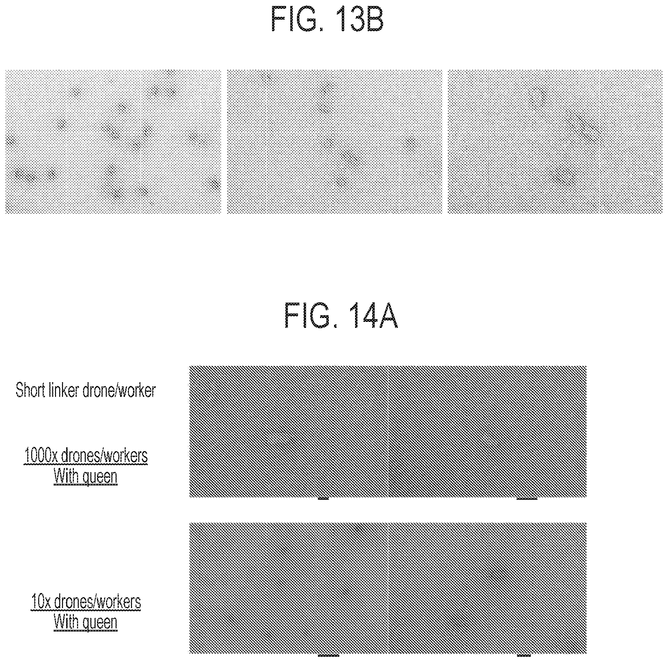

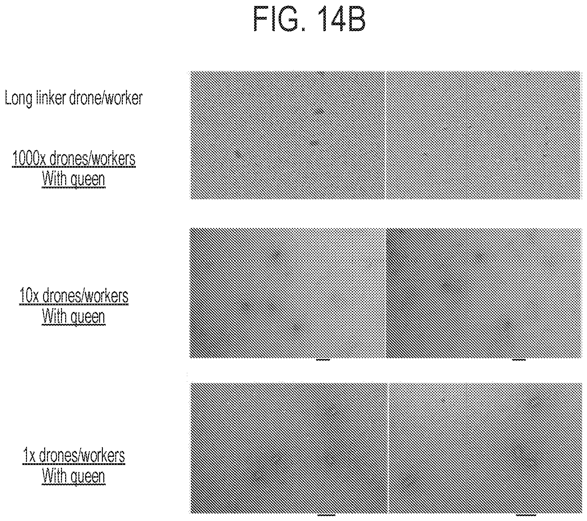

FIGS. 14A-14C show results demonstrating that nanostructures (workers) assemble in the presence of a nucleating nanostructure (queen) but not in the absence of a nucleating nanostructure.

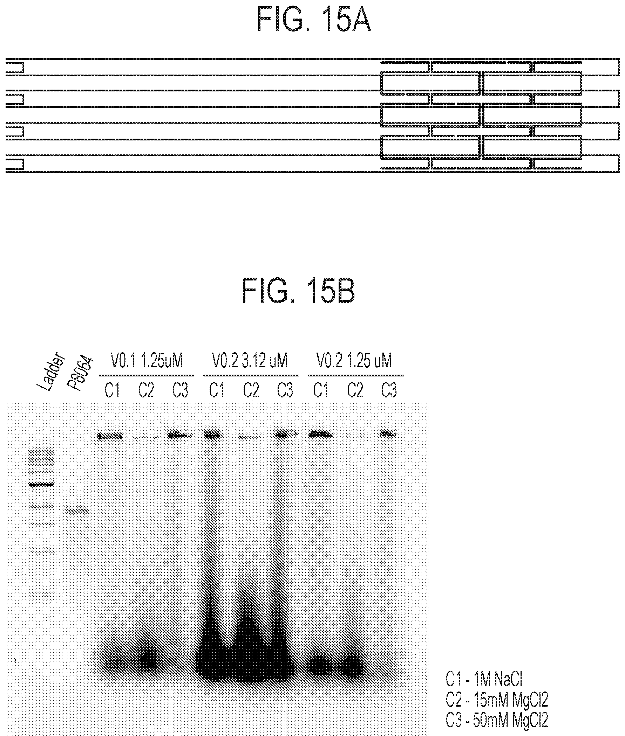

FIGS. 15A-15B show results an example of nanostructures not assembling in the absence of a nucleating nanostructure (queen).
FIG. 16A shows a two-dimensional view of the gridiron queen that can bind 16 drones simultaneously in a horizontal (coordinate x in FIG. 16B) across the queen. Staples necessary to fold the scaffold into the queen are shown, and the 3' ends of the staples may be appended with overhanging single-stranded sequences to bind drones. A three-dimensional view (FIG. 16B) shows the queen with binding sites in each drone-docking cell. A transmission electron microscope (TEM) image of the queen is shown in FIG. 16C. Lateral dimensions of the structure are approximately 72 nm x 240 nm.

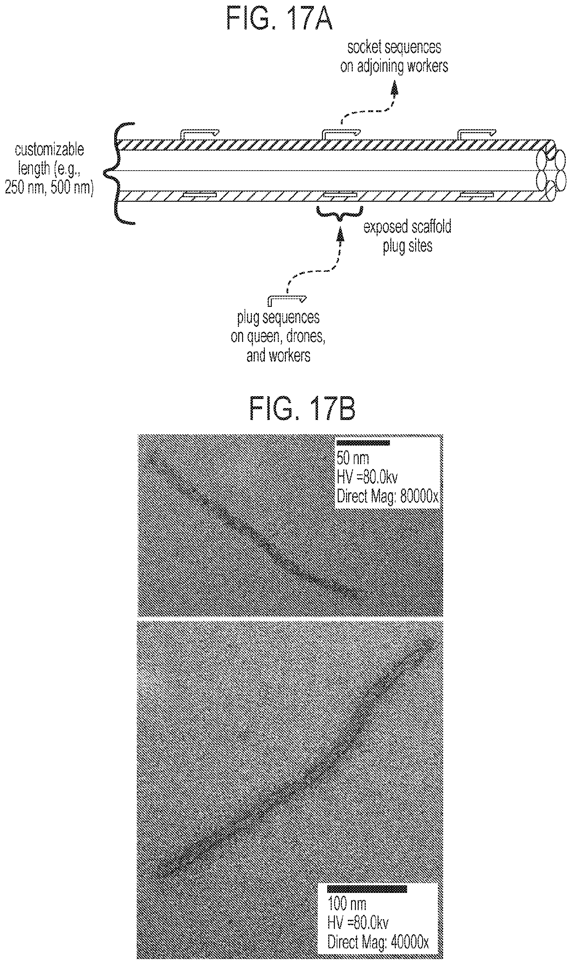

FIGS. 17A-17B depict drone and worker subcomponents. FIG. 17A shows drone and worker subcomponents constructed from 6-helix bundle scaffolded DNA origami to form rods that are customizable in length. The 3' ends of staples contain overhanging single strand DNA sequences that act as plug binding handles to interact with other components. Similarly, the lowermost helix contains socket sequences (i.e. single strand DNA scaffold not complemented by a folding staple) that accept plug sequences from other components. The plugs and socket respectively are periodic and can be situated, for example, every 42 bp (~ 14 nm) along the length of the component. FIG. 17B shows TEM images of test drones folded from two different scaffold sequences. The drone in the top image is -250 nm in length, versus the drone in the bottom image, which is -440 nm in length.
FIG. 18 shows a detailed view of the plug-socket binding system. The case shown in the upper panel shows the full set of 5 single-stranded plug sequences extending from the queen (the small arrow) with matching socket sites in a six-helix bundle drone. In the lower pane, the binding sequence is drawn as a series of 'X' to indicate that both the length and sequence of the plug and socket may be varied. The scaffold sequences are drawn in black. Note that this design (shown for a drone-queen assembly) is also used to bind drones to workers. The gridiron queen sequences, from top to bottom, correspond to SEQ ID NOs: 685 and 686. The 6 helix bundle (hb) drone sequences, from left to right, correspond to SEQ ID NOs: 687 and 688.
FIGS. 19A-19D shows how the plug-socket binding system can be used to program drones to bind to desired sites on the queen. FIG. 19A shows two 440 nm drones placed in the middle two queen cells, FIG. 19B shows one 250 nm drone placed in the middle queen cell, and FIG. 19C shows 250 nm located in every cell of the queen. The desired design is shown to the left, versus a TEM image of the assembled structure to the right. FIG. 19D shows bulk analysis of the design from FIG. 19A using agarose gel electrophoresis for one design using a 7 bp plug-socket, and another with a 10 bp plug-socket.

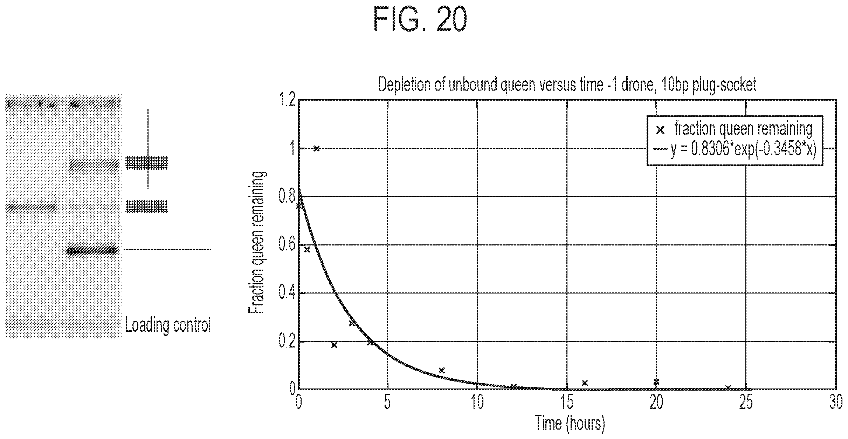

FIG. 20 shows the extent of free queen remaining over time as it becomes bound to a single 440 nm drone.
FIGS. 21A-21B show an example of a crisscross DNA slat-based architecture. FIG.21A is an abstraction of the crisscross DNA slats motif (right). Light and dark strands weave and are complementary to each other at each junction. The length of each binding site is shown on the right. Each row and column amount to 21 base pairs (bp). The matrix shows the number of base pairs (bp) per binding site at each position of the abstraction and 3D rendering. FIG. 21B is a 3D rendering of the DNA slats. On the left, the top down view shows the weaving of each strand. A cross section (A-A) is shown on the right.
FIG. 22 shows the steps to DNA slats assembly. Step 1 shows DNA-origami folding of an arbitrary DNA-origami queen (a barrel queen shown as an example). Step 2 is the mixing of the crude DNA-origami queen reaction (from step 1) with DNA slats at various salt concentrations, temperatures, and DNA slat concentration.
FIG. 23A shows a flat DNA-origami queen without any DNA slats added. FIG. 23B shows a flat DNA-origami queen with the addition of DNA slats and the correct formation of a sheet, by tiling the ssDNA scaffold of the queen with DNA slats. DNA slat tiled region is indicated in light gray. Scale bars on images are 600 nm and on enlarged view 100 nm.
FIG. 24A shows a barrel DNA-origami queen without any DNA slats added. Scale bar on image is 400 nm and on enlarged view 100nm. FIG. 24B shows a barrel DNA-origami queen with the addition of DNA slats and the correct formation of a barrel, by tiling the ssDNA scaffold of the queen with DNA slats. Scale bar indicates 50nm. DNA slat tiled region is indicated in light gray.

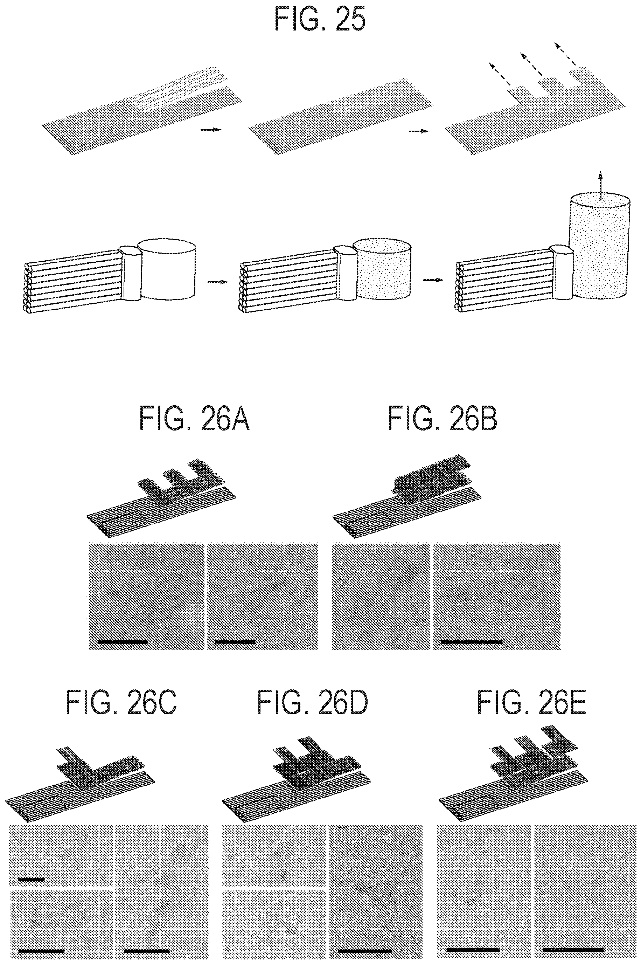

FIG. 25 depicts a growth mechanism of DNA slats seeded on a queen structure. The DNA-origami queen is mixed with DNA slats to tile the ssDNA scaffold of the queen and then later extend and polymerize the growth of micron-sized structures solely through DNA slats (DNA slats may be joined end-to-end, within the same plane, through nucleotide base pairing of adjacent slats). The upper design shows a flat DNA-origami queen with the growth of three linear sheets in the horizontal direction. The lower design shows a barrel DNA-origami queen with tubular growth in the vertical direction.
FIGS. 26A-26E show three extensions of the first generation of DNA slats binding to the flat DNA-origami queen. FIG. 26A shows first generation extensions tiled with a short second generation of DNA slats, resulting in three tooth-like extensions on the queen. FIG.26B shows first generation extensions tiled with a long second generation of DNA slats, which are terminally tiled with short third generation DNA slats. FIGS. 26C-26E show first, second, and third generations of DNA slats which are complementary to one another, resulting in extensions of linear sheet structures. FIG. 26C contains one extended first generation, FIG. 26D contains two extended first generations, and FIG. 26E contains three extended first generations. Scale bars for FIGS. 26A-26C are 100 nm and for FIGS. 26D-26E, 20 0nm.
FIGS. 27A-27E show formation of multi-host-ring catenane systems with DNA slats in a one-pot reaction. (FIG. 27A) Formation of eight loops with M13 scaffold through staple strands. Staple ("brown") strands fold stable DNA-Origami base and DNA slats catenate the eight loops. (FIG. 27B) 3D view of barrel queen additionally serving as multi-host-ring catenane system with high yield typical for DNA-Origami. (FIG. 27C) Abstract and 3D view of DNA slats weaving through the ssDNA M13 scaffold loops on the barrel queen. Through ligation of one side a single DNA slat catenates all eight loops. (FIG. 27D) 3D rendering of DNA slat weaving and catenating eight separate ssDNA loops. Top shows a tilted bottom view and bottom a side view. (FIG. 27E) Former technique to achieve a maximum of four-host-ring catenane system with low yield.
FIGS. 28A-28B show a barrel queen used for ultrasensitive detection. FIG. 28A shows that the biomolecule presence is connected to the DNA slats (black) holding the eight loops together. Without the biomolecule, the queen falls apart and no growth can occur, even with DNA slats present in solution. FIG. 28B shows that biomolecule presence in the reaction holds the DNA slats (black) together and provides the close proximity of ssDNA scaffold for the tube structure to nucleate and grow.

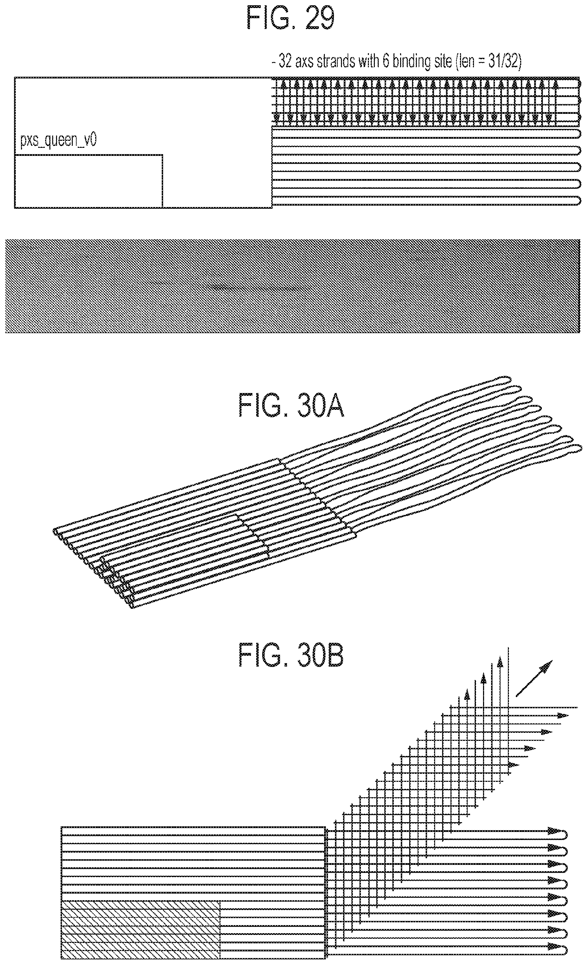

FIG. 29 shows a schematic (upper panel) of a queen with six binding sites per slat and a transmission electron microscope (TEM) image (lower panel) of the queen.
FIGS. 30A-30B show a flat DNA-origami queen nucleating a staggered DNA slats ribbon. FIG. 30A depicts a flat DNA-origami queen without the bottom right hand sheet shown in FIG. 23A. FIG. 30B is a schematic explaining how the DNA slats (moving in a diagonal direction) assemble on the ssDNA scaffold on the flat queen.

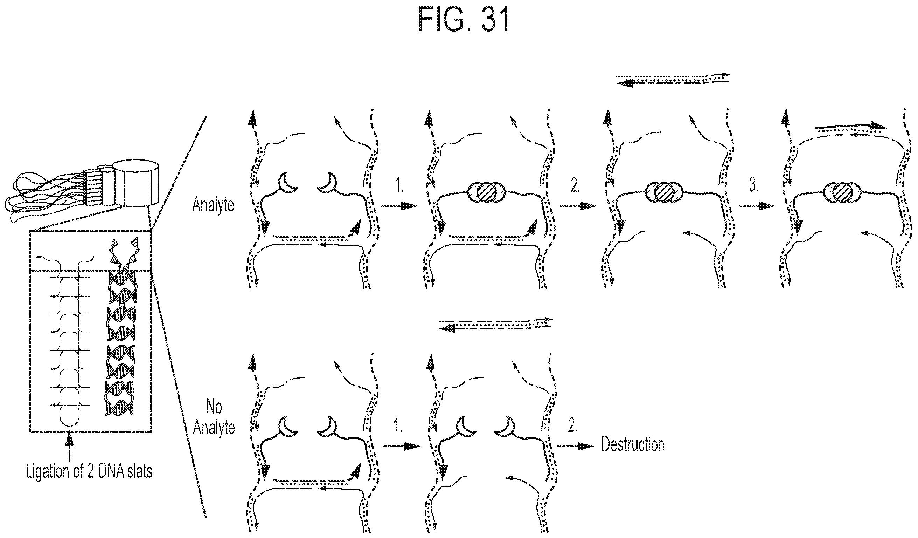

FIG. 31 shows an example of a biomolecule sensing and proofreading mechanism on DNA-Origami barrel queen. Top: Biomolecule present. (1) Biomolecule binds to antibody bridge. (2) Medium gray strand is displaced via toehold-mediated strand displacement. (3) Light gray strand binds to dark gray strands, sealing bridge. Bottom: No biomolecule present. (1) No biomolecule binds to the antibody bridge. (2) Medium gray strand is displaced via toehold-mediated strand displacement, leading to no bridge being intact and the subsequent falling apart of the barrel queen (shown in FIG. 28A).

### DETAILED DESCRIPTION

Nature achieves rapid and nucleation-limited growth of cytoskeletal filaments such as actin and microtubules. This is achieved by securing each additional subunit by weak interactions to 2-3 already attached subunits at the growing end of the filament. This means that if any two monomers bind to each other in solution, they will rapidly (e.g., within milliseconds) dissociate from each other, because the single interaction is so weak. It is only after four subunits come together simultaneously - a rare event - that a stable nucleus will be formed. Therefore, untriggered spontaneous nucleation will be rare. Conversely, nucleation can be triggered by providing a macromolecular "seed" that mimics a fully formed filament end.

Rapid and nucleation-limited growth are very useful features for programmable self-assembly, however technological modification of natural filaments such as actin or microtubules has many current drawbacks: (1) there is a limited understanding of how to tune the interaction strength between subunits; (2) the level of cooperativity is relatively low (the weak interactions upon binding are spread only over 2-3 subunits), therefore the suppression of spontaneous nucleation is not as robust as it could be; and (3) growth is limited to one-dimension (filament formation).

Rapid, reversible, zero-background, triggered nucleation and growth, as provided herein, can have useful applications in nanotechnology and biotechnology, such as ultrasensitive detection, and templates for miniaturized materials.

### Crisscross Cooperative Assembly

The crisscross cooperative assembly technology as provided herein is based on a concept that may apply to many self-assembling molecules, including nucleic acids and proteins. For simplicity and ease of understanding, however, reference herein primarily will address crisscross cooperative assembly in the context of nucleic acids, such as deoxyribonucleic acid (DNA). A crisscross cooperative assembly system uses three basic components: a nucleating nanostructure, an initial (first) subset of nanostructures programmed to bind to the nucleating nanostructure, and another (second) subset of nanostructures programmed to bind to the nanostructures of the initial. An example of a crisscross cooperative assembly is provided in FIGS. 1A-1B, wherein the nucleating structure is referred to as a 'queen,' nanostructures of the first subset are referred to as 'drones,' and nanostructures of the second (and any subsequent) subset are referred to as 'workers.' The final structure, in this example, includes layers of aligned molecular rods, where each layer is rotated by some amount *(e.g.,* 90 degrees) relative to the layer below and above. For example, one layer may be perpendicular to another adjacent (directly above or below) layer. In some embodiments, one layer is rotated 20, 30, 40, 50, 60, 70, 80 or 90 degrees relative to an adjacent layer (measured alone the length of a drone and/or worker nanorod, for example). Each intersection between rods on adjacent layers adds a small binding energy; any given rod intersects with a large number of rods below and above, and the net binding energy can be tuned *(e.g.,* by adjusting the design of the binding interface, for example, the number of base pairs, or by adjusting subunit concentration, temperature, or salt concentration) to be large enough to achieve stable (irreversible) or slightly favorable (reversible) attachment as desired. Before assembly initiates, any spontaneous crossing between two rods in solution is short-lived, as the net energy is very low because there is only one interaction. Thus, a rod can be stably (or else slightly favorably (reversibly)) added to a pre-existing crisscross structure (many attachment points can immediately be realized), but a structure will not spontaneously assemble in the absence of a pre-existing one. There should be no growth unless a structural mimic of a pre-existing crisscross structure-a seed-is added to the solution.

An example protocol for a crisscross cooperative assembly system is as follows: (1) Design constitutive building blocks (queen, drones and workers) using DNA CAD tools. See, e.g., Douglas SM, Marblestone AH, Teerapittayanon S, Vazquez A, Church GM, Shih WM. Rapid prototyping of 3D DNA-origami shapes with caDNAno. Nucleic Acids Res. 37, 5001-5006, 2009.

Cooperative binding site sequences and number of sites on queens are tailored to modulate the activation energy of nucleation as required. (2) Construct and purify constitutive building blocks using techniques in DNA synthesis and DNA origami. (3) Mix drones and workers in solution, and add queens to initiate growth of higher order DNA structures.

Nanostructures bind to each other through cooperative binding sites. A "cooperative binding site" is the location at which two nanostructures interact (hybridize/bind). For example, a nucleating nanostructure may be programmed with multiple nucleotide base sequences, each of which is complementary to a nucleotide base sequence of one of the nanostructures of the initial subset of nanostructures. A cooperative binding site may include plug and socket sites that include plug and socket strands. A plug strand is a nucleic acid strand (single-stranded nucleic acid) attached to a nucleic acid nanostructure, such as a nanorod. A plug strand contains a nucleotide sequence that is complementary to (and this binds to) a nucleotide sequence within a cognate socket strand. Thus, a pair of plug and socket strands include nucleotide sequences that are complementary to each other such that the plug and socket strand bind (hybridize) to each other to anchor, for example, a drone to a queen or a worker to a drone (see, e.g., FIG. 17B). In some embodiments, a queen includes multiple plug strands that direct and anchor a drone that includes multiple complementary (cognate) socket strands. Likewise, a drone may include multiple plug strands that direct and anchor a worker that includes multiple complementary socket strands.

Cooperative binding sites, e.g., plug and socket strands, may also be used to assemble nucleic acid (e.g., DNA) slats onto another nucleic acid scaffold structure in a similar manner. For example, as shown in FIG. 22, DNA slats may be appended to a nucleic acid scaffold (queen) to secure the two- or three-dimensional shape of the scaffold structure. In the example, shown in FIG. 22, DNA slats are used to secure (hold together) the barrel shape of a larger scaffold nanostructure. "Growth" of these slats along the scaffold through cooperative binding sites results in a barrel-like shape that may be visualized by microscopy, for example.

Cooperative binding sites (e.g., plug and socket sequences) are arranged on a nucleating nanostructure in a spatial configuration that facilitates binding and alignment of the initial e.g.,. scaffold) nanostructures. The length of a cooperative binding site may vary, depending in part on the desired strength (strong v. weak) of the intended interaction between two molecules having complementary sites. In some embodiments, a cooperative binding site has a length of 5-50 nucleotides. For example, a cooperative binding site may have a length of 5-40, 5-30, 5-20, 5-10, 5-15, 10-50, 10-40, 10-30, 10-20, 30-50, 30-40, or 40-50 nucleotides. In some embodiments, a cooperative binding site has a length of 5, 10, 15, 20, 25, 30, 35, 40, 45, or 50 nucleotides. A single plug strand and/or socket strand may have a length of 5-20 (e.g., 5, 6, 7, 8, 9, 10, 11, 12, 13, 14, 15, 16, 17, 18, 19 or 20) nucleotides, for example.

The number of cooperative binding sites on a nanostructure may also vary. In some embodiments, the number of cooperative binding sites on a nanostructure is 3-1000. For example, the number of cooperative binding sites on a nanostructure may be 3-900, 3-800, 3-700, 3-600, 3-500, 3-400, 3-300, 3-200, or 3-100. In some embodiments, the number of cooperative binding sites on a nanostructure is 3-10, 3-15, 3-20, 3-25, 3-30, 3-35, 3-40, 3-45 or 3-50. In some embodiments, the number of cooperative binding sites on a nanostructure is 3-15, 3-20, 3-25, 3-30, 3-35, 3-40, 3-45 or 3-50. In some embodiments, the number of cooperative binding sites on a nanostructure is 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100.

The distance between cooperative binding sites may also vary. In some embodiments, the distance between two cooperative binding sites on the same nanostructure is 20-1000 angstroms. For example, the distance between two cooperative binding sites on a nanostructures may be 20-900, 20-800, 20-700, 20-600, 20-500, 20-400, 20-300, 20-200, 20-100, 50-1000, 50-900, 50-800, 50-700, 50-600, 50-500, 50-400, 50-300, 50-200, or 50-100 angstroms. In some embodiments, the distance between two cooperative binding sites on a nanostructures is 20, 25, 30, 35, 40, 45, 50, 55, 60, 65, 70, 75, 80, 85, 90, 95, 100, 125, 150, 175, 200, 250, 300, 350, 400, 450 or 500 angstroms.

In some embodiments, the distance between cooperative binding sites, for example, the distance between plug strands (and/or between socket strands) may be 5 to 100 nucleotides (or nucleotide base pairs (bp)). In some embodiments, the distance between plug strands (and/or between socket strands) is 5-20, 5-25, 5-50 or 5-100 nucleotides. In some embodiments, the distance between plug strands (and/or between socket strands) is 5, 10, 15, 20, 25, 30, 35, 40, 45, 50, 55, 60, 65, 70, 75, 80, 85, 90, 95 or 100 nucleotides. In some embodiments, the distance between plug strands (and/or between socket strands) is 5, 6, 7, 8, 9, 10, 11, 12, 13, 14, 15, 16, 17, 18, 19, 20 or 21 nucleotides. In some embodiments, the distance between plug strands (and/or between socket strands) is 42 +/- 21 nucleotides. For example, the distance between plug strands (and/or between socket strands) may be 21, 42 or 63 nucleotides. In some embodiments, the distance between plug strands (and/or between socket strands) is 42 nucleotides.

One nucleotide unit measures 0.33 nm. Thus, in some embodiments, the distance between cooperative binding sites, for example, the distance between plug strands (and/or between socket strands) may be 5 to 35 nanometers (nm). In some embodiments, the distance between plug strands (and/or between socket strands) is 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 nm. In some embodiments, the distance between plug strands (and/or between socket strands) is 14 +/- 7 nm. For example, the distance between plug strands (and/or between socket strands) may be 7, 14 or 21 nm. In some embodiments, the distance between plug strands (and/or between socket strands) is 14 nucleotides.

In some embodiments, the distance between two cooperative binding sites on a nanostructure is evenly spaced, while in other embodiments, the distances may vary. For example, the distance between a first cooperative binding site and a second cooperative binding site may be 30 angstroms, while the distance between the second cooperative binding site and a third may be 30 angstroms, 40 angstroms or 50 angstroms.

Two or more nanostructures are considered "aligned" if they are oriented in the same direction relative to one another. For example, the 5' ends (or 3' ends) of the nanostructures maybe facing the same direction along its y axis. The top layer of the structure shown in FIG. 3B shows aligned nanorods bound to a nucleating nanostructure. The nanorods, in this example, are perpendicular to the nucleating nanostructure.

A nucleating nanostructure is required to initiate assembly of the first (initial) and second (and, thus, subsequent, *e.g.,* third, fourth, fifth, *etc.)* subsets of nanostructures, and binding of the nanostructures in the first subset to the nucleating structure is required to initiate assembly of the nanostructures of the second subset. A "nucleating nanostructure" is any nanostructure programmed with binding sites that interacts strongly (irreversibly) with binding sites on each member of drone nanostructures of the initial subset, and aligns them for recruitment of subsequent subsets of worker nanostructures. That is, the binding sites between a nucleating nanostructure and nanostructures of the initial subset should be strong enough that the initial nanostructures bind to and align along the nucleating nanostructures and do not dissociate from the nucleating nanostructure under reaction conditions (*e.g.*, isothermal, physiological conditions). A nucleating nanostructure may have a two-dimensional or a three-dimensional shape, for example.

Additional subsets of nanostructures may be added to the crisscross cooperative assembly system to propagate growth of the end structure (*e.g.,* nanostructure, microstructure or macrostructure). For example, third, fourth and fifth subsets of nanostructures may be added. Binding of the nanostructures of the second subset to the first subset is required to initiate assembly of the nanostructures of the third subset; binding of the nanostructures of the third subset to the second subset is required to initiate assembly of the nanostructures of the fourth subset; and so on. The user-defined end structure may be assembled in one dimension, two dimensions *(see, e.g.,* FIG. 3B) or three-dimensions.

Each subset of nanostructures (*e.g.*, nanorods) should follow a specific set of binding energy parameters. More specifically, the initial subset of nanostructures (*e.g.*, nanorods) should bind strongly (irreversibly) to and form an aligned layer (where each nanostructure is oriented in the same direction relative to one another) along the nucleating nanostructure. The nanostructures (*e.g.,* nanorods) of the initial subset should not interact with (bind to) each other. Likewise, nanostructures (*e.g.*, nanorods) of a subsequent subset should not interact with (bind to) each other. Further, in the absence of a nucleating structure, any nanostructure (*e.g.*, nanorod) from the initial subset should have only one weak (reversible) interaction with any other nanostructure (*e.g.*, nanorod) from a subsequent subset. In the presence of a nucleating structure, a single nanostructure (*e.g.*, nanorod) from an initial subset may interact with more than one nanostructure *(e.g.,* nanorod) from a subsequent subset, and a single nanostructure *(e.g.,* nanorod) from a subsequent subset may interact with more than one nanostructure (*e.g.*, nanorod) from the initial subset. For example, with reference to FIG. 1B, a single nanostructure (*e.g.*, nanorod) may bind to eight other nanostructure (*e.g.*, nanorod), although the single nanostructure *(e.g.,* nanorod) binds to each of the eight nanostructure *(e.g.,* nanorod) only once to form two layers having a 'crisscross' pattern.

A "strong interaction" refers to binding that is engaged more than 50% *(e.g.,* more than 60%, 70%, 80% or 90%) of the time that the binding nanostructures are in a reaction together (the dissociation constant is lower than the concentration of the species/nanostructures in excess).

A "weak interaction" - refers to binding that is engaged less than 1% of the time that the binding nanostructures are in a reaction together (the dissociation constant is at least 100 times higher than the concentration of the species/nanostructures in excess).

A nucleating nanostructure may bind to two or more other nanostructures. In some embodiments, a nucleating nanostructure binds to 5-1000 nanostructures (*e.g.*, DNA nanorods). For example, a nucleating nanostructure may bind to 3-900, 3-800, 3-700, 3-600, 3-500, 3-400, 3-300, 3-200, or 3-100 nanostructures. In some embodiments, a nucleating nanostructure binds to 3-10, 3-15, 3-20, 3-25, 3-30, 3-35, 3-40, 3-45 or 3-50 nanostructures. In some embodiments, a nucleating nanostructure binds to 10-15, 10-20, 10-25, 10-30, 10-35, 10-40, 10-45 or 10-50 nanostructures. In some embodiments, a nucleating nanostructure binds to 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100 nanostructures (*e.g.*, DNA nanorods).

Thus, a single subset of nanostructures (nanostructures programmed to interact with a single nucleating nanostructure) may comprise 3-900, 3-800, 3-700, 3-600, 3-500, 3-400, 3-300, 3-200, or 3-100 nanostructures. In some embodiments, a single subset of nanostructures comprises 3-10, 3-15, 3-20, 3-25, 3-30, 3-35, 3-40, 3-45 or 3-50 nanostructures. In some embodiments, a single subset of nanostructures comprises 10-15, 10-20, 10-25, 10-30, 10-35, 10-40, 10-45 or 10-50 nanostructures. In some embodiments, a single subset of nanostructures comprises 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100 nanostructures (*e.g.*, DNA nanorods).

A "subset of nanostructures" refers to a specific group of nanostructures that are similar in size (have similar dimensions) and structure/shape and are programmed to bind to either the nucleating nanostructure (the initial subset) or to a pre-existing layer formed by alignment and binding of other nanostructures that have already aligned and bound to the nucleating structure or nanostructures of another pre-existing layer.

Nanostructures within a defined subset are programmed not bind to each other. Thus, in some embodiments, less than 10% of the nanostructures of a subset bind to another nanostructure of the same subset. In some embodiments, less than 9%, less than 8%, less than 7%, less than 6%, less than 5%, less than 4%, less than 3%, less than 2%, less than 1%, less than 0.5%, less than 0.2%, or less than 0.1% of the nanostructures of a subset bind to another nanostructure of the same subset. In some embodiments, none of the nanostructures of a subset bind to another nanostructure of the same subset.

With crisscross cooperative assembly, nanostructures are aligned to form multiple layers, each layer rotated by some degree relative to adjacent layers (above and below). An example of two layers rotated relative to one another is shown in FIG. 1B. The top layer of aligned nanorods is rotated 90 degrees relative to the bottom layer of aligned nanorods. The degree of rotation between two adjacent layers may vary. In some embodiments, one layer is rotated 10-90 degrees, 20-90 degrees, 30-90 degrees, 40-90 degrees, 50-90 degrees, 60-90 degrees, 70-90 degrees, or 80-90 degrees relative to an adjacent layer. In some embodiments, one layer is rotated 10, 15, 20, 25, 30, 35, 40, 45, 50, 55, 60, 65, 70, 75, 80, 85, or 90 degrees relative to an adjacent layer.

### Nucleic Acid Nanostructures

A "nucleic acid nanostructure," including a "DNA nanostructure," refers to a nanostructure *(e.g.,* a structure that is between 0.1 nm and 1 µm *(e.g.,* 0.1 nm and 100 nm) in each spatial dimension, *e.g.*, 1D, 2D or 3D) that is rationally designed to self-assemble (is programmed) into a pre-determined, defined shape that would not otherwise assemble in nature. The use of nucleic acids to build nanostructures is enabled by strict nucleotide base pairing rules *(e.g.,* A binds to T, G binds to C, A does not bind to G or C, T does not bind to G or C), which result in portions of strands with complementary base sequences binding together to form strong, rigid structures. This allows for the rational design of nucleotide base sequences that will selectively assemble (self-assemble) to form nanostructures.

Examples of nucleic acid *(e.g.,* DNA) nanostructures include, but are not limited to, DNA origami structures, in which a long scaffold strand *(e.g.,* at least 500 nucleotides in length) is folded by hundreds *(e.g.,* 100, 200, 200, 400, 500 or more) of short *(e.g.,* less than 200, less than 100 nucleotides in length) auxiliary strands into a complex shape (Rothemund, P. W. K. Nature 440, 297-302 (2006); Douglas, S. M. et al. Nature 459, 414-418 (2009); Andersen, E. S. et al. Nature 459, 73-76 (2009); Dietz, H. et al. Science 325, 725-730 (2009); Han, D. et al. Science 332, 342-346 (2011); Liu, W et al. Angew. Chem. Int. Ed. 50, 264-267 (2011); Zhao, Z. et al. Nano Lett. 11, 2997-3002 (2011); Woo, S. & Rothemund, P. Nat. Chem. 3, 620-627 (2011); Tørring, T. et al. Chem. Soc. Rev. 40, 5636-5646 (2011)). Other more modular strategies have also been used to assemble DNA tiles (Fu, T. J. & Seeman, N. C. Biochemistry 32, 3211-3220 (1993); Winfree, E. et al. Nature 394, 539-544 (1998); Yan, H. et al. Science 301, 1882-1884 (2003); Rothemund, P. W. K. et al. PLoS Biol. 2, e424 (2004); Park, S. H. et al. Angew. Chem. Int. Ed. 45, 735-739 (2006); Schulman, R. & Winfree, E. Proc. Natl Acad. Sci. USA 104, 15236-15241 (2007); He, Y. et al. Nature 452, 198-201 (2008); Yin, P. et al. Science 321, 824-826 (2008); Sharma, J. et al. Science 323, 112-116 (2009); Zheng, J. P. et al. Nature 461, 74-77 (2009); Lin, C. et al. ChemPhysChem 7, 1641-1647 (2006)) or RNA tiles (Chworos, A. et al. Science 306, 2068-2072 (2004); Delebecque, C. J. et al. Science 333, 470-474 (2011)) into periodic (Winfree, E. et al., Nature 394, 539-544 (1998); Yan, H. et al. Science 301, 1882-1884 (2003); Chworos, A. et al. Science 306, 2068-2072 (2004); Delebecque, C. J. et al. Science 333, 470-474 (2011)) and algorithmic (Rothemund, P. W. K. et al. PLoS Biol. 2, e424 (2004)) two-dimensional lattices (Seeman, N. C. J. Theor. Biol. 99, 237-247 (1982); Park, S. H. et al. Angew. Chem. Int. Ed. 45, 735-739 (2006)), extended ribbons (Schulman, R. & Winfree, E. Proc. Natl Acad. Sci. USA 104, 15236-15241 (2007); Yin, P. et al. Science 321, 824-826 (2008)) and tubes (Yan, H. et al. Science 301, 1882-1884 (2003); Yin, P. et al. Science 321, 824-826 (2008); Sharma, J. et al. Science 323, 112-116 (2009)), three-dimensional crystals (Zheng, J. P. et al. Nature 461, 74-77 (2009)), polyhedral (He, Y. et al. Nature 452, 198-201 (2008)) and simple finite two-dimensional shapes (Chworos, A. et al. Science 306, 2068-2072 (2004); Park, S. H. et al. Angew. Chem. Int. Ed. 45, 735-739 (2006)).

Thus, crisscross cooperative assembly building blocks (*e.g.*, nucleating nanostructures and subsets of nanostructures) may be one of a number of nucleic acid nanostructure shapes, including, but not limited to, rods/tubes, sheets, ribbons, lattices, cubes, spheres, polyhedral, or another two-dimensional or three-dimensional shape. In some embodiments, a nanostructure has junction(s), branch(es), crossovers, and/or double-crossovers formed by nucleotide base pairing of two or more nucleic acid strands *(see, e.g.,* Mao, C. PLoS Biology, 2(12), 2036-2038, 2004).

In some embodiments, a nucleic acid nanostructure has a handle and barrel shape, similar to that depicted in FIG. 22.

The versatile and stable nature of DNA origami enables the construction of various individual architectures that can be designed in a particular way, to facilitate to cooperative assembly of larger structures. In one example, each component is a separately folded DNA-origami structure. FIG. 3A shows an example of a DNA origami queen, drone and worker, whereby the drone and worker are of identical architecture (six helix bundle DNA nanotubes). Queen, drones and workers can then assemble in a cooperative manner to form higher order 1D, 2D and 3D structures (FIG. 3B). 3D structures are contemplated by merging 1D and 2D design principles. For example, FIG. 22 depicts a 3D queen nanostructure assembling with 2D drone/worker slats to form a barrel shape.

A nucleic acid (e.g., DNA) slat is a slat-shaped nanostructure that is composed of DNA. A slat may be an antiparallel-crossover single-stranded slat (AXSSS) comprising single strands that cross a partnering single strand only once. Also provided herein are paranemic crossover slats that include a pair of strands that cross another pair of strands.

Similar to the larger scale DNA-origami crisscross cooperative assembly, single-stranded DNA can be used to achieve cooperative assembly of higher order structures. In order to achieve this, drones and workers are replaced with oligonucleotides of various lengths (depending on the proposed architecture) that can assemble onto a DNA-origami queen nucleation site (shown in FIG. 4) or onto a single stranded DNA catenane structure shown in FIG. 5, FIG. 22 and FGIS. 24A-24B. The ring structures depicted in FIG. 5 are comprised of single-stranded DNA that has exposed binding sites for drone and worker oligonucleotides. In another example, the components are folded into a DNA origami barrel queen (FIGS. 24A-24B). The scaffold can be tiled with extended DNA slats (slats) capable of seeding further DNA slats, leading to growth of the structure. Generally, the DNA slats work in two steps: first, folding the origami queen site (for example, mixing M13 scaffold and staple strands), and second, mixing the crude DNA origami queen reaction with DNA slats, leading to growth of the structure. Varying salt concentrations, temperatures, and DNA slat concentration can alter the binding energy of the various sub-components, leading to reversible or irreversible binding, for example.

Typically, nucleic acid nanostructures do not contain coding sequences (sequences that code for a full length mRNA or protein), thus, nucleic acid nanostructures do not contain a promoter or other genetic elements that control gene/protein expression. An individual single-stranded nucleic acid *(e.g.,* DNA strand or RNA strand without secondary structure), or an individual double-stranded nucleic acid (*e.g.*, without secondary structure), for example, double helices found in nature or produced synthetically or recombinantly (*e.g.*, such as a plasmid or other expression vector), are specifically excluded from the definition of a nucleic acid nanostructure.

Nanostructures, in some embodiments, have a void volume, which is the combine volume of space between nucleic acids that form a nanostructures. It should be understood that "space" includes fluid-filled space. Thus, a nanostructure in solution, have a void volume of 25% may include 75% nucleic acids and 25% reaction buffer (filling the 25% void volume of the nanostructure). In some embodiments, a nanostructure in solution, *e.g.*, in reaction buffer, may have a void volume of at least 10% *(e.g.,* 10-90%, 10-80%, 10-70%, 10-60%, 10-50%, 10-40%, or 10-30%), at least 20% *(e.g.,* 20-90%, 20-80%, 20-70%, 20-60%, 20-50%, 20-40%, or 20-30%), at least 30%, *(e.g.,* 30-90%, 30-80%, 30-70%, 30-60%, 30-50%, or 30-40%), at least 40% *(e.g.,* 40-90%, 40-80%, 40-70%, 40-60%, or 40-50%), at least 50% *(e.g.,* 50-90%, 50-80%, 50-70%, or 50-60%), at least 60% *(e.g.,* 60-90%, 60-80%, or 60-70%), at least 70% *(e.g.,* 70-90% or 70-80%), or at least 80% *(e.g.,* 80-90%). In some embodiments, a nanostructure has a void volume of 10%, 15%, 20%, 25%, 30%, 35%, 40%, 45%, 50%, 55%, 60%, 65%, 70%, 75%, 80%, 85%, or 90%.

A "nucleic acid nanorod," including a "DNA nanorod" is a nucleic acid *(e.g.,* DNA) nanostructure in the shape of a rod. A nanorod is a three-dimensional cylindrical shape having a length longer than its diameter. Examples of nanorods are depicted in FIGS. 1A-1B and FIGS. 3A-3B. In some embodiments, a nucleic acid nanorod comprises six helix bundles. For example, six DNA double helices may be connected to each other at two crossover sites. DNA double helices with 10.5 nucleotide pairs per turn facilitate the programming of DNA double crossover molecules to form hexagonally symmetric arrangements when the crossover points are separated by seven or fourteen nucleotide pairs *(see, e.g.,* Mathieu F. et al. Nano Lett. 5(4), 661-664 (2005)). Other methods of assembling nucleic acid nanorods (also referred to as nanotubes) may be used *(see, e.g.,* Feldkamp, U. et al. Angew. Chem. Int. Ed. 45(12), 1856-1876 (2006); Hariri A. et al. Nature Chemistry, 7, 295-300 (2015)).

The length and diameter of a nanorod (or other nanostructure) may vary. In some embodiments, a nanorod (or other nanostructure) has a length of 10-100 nm, or 10-500 nm. For example, a nanorod may have a length of 10-500 nm, 10-400 nm, 10-300 nm, 10-200 nm, 10-100 nm, 10-90 nm, 10-80 nm, 10-70 nm, 10-60 nm, 10-50 nm, 10-30 nm, or 10-20 nm. In some embodiments, a nanorod has a length of 100-500 nm, 200-500 nm, or 300-500 nm. In some embodiments, a nanorod has a length of 100 nm, 150 nm, 200 nm, 250 nm, 300 nm, 350 nm, 400 nm, 450 nm or 500 nm. In some embodiments, a nanorod has a length of 10, 15, 20, 25, 30, 35, 40, 45, 50, 55, 60, 65, 70, 75, 80, 85, 90, 95 or 100 nm. In some embodiments, the length of a nanorod (or other nanostructure) is longer than 100 nm *(e.g.,* 100-1000 nm), or shorter than 10 nm *(e.g.,* 1-10 nm). In some embodiments, a nanorod (or other nanostructure) has a diameter of 5-90 nm. For example, a nanorod may have a diameter of 5-80 nm, 5-70 nm, 5-60 nm, 5-50 nm, 5-30 nm, 5-20 or 5-10 nm. In some embodiments, a nanorod has a diameter of 5, 10, 15, 20, 25, 30, 35, 40, 45, 50, 55, 60, 65, 70, 75, 80, 85 or 90 nm. In some embodiments, the diameter of a nanorod is longer than 9 nm, or shorter than 5 nm. Thus, in some embodiments, a nanorod (or other nanostructure) has a circumference of 15-300 nm (C ≈ 3.14 x d).

A nucleic acid nanostructure, such as a nanorod, is considered "elongated," if the length of the nanostructure is longer than its width/diameter (*e.g.*, by at least 10%, 20%, 25%, 50%, 100%, or 200%).

Nucleic acid nanostructures are typically nanometer-scale structures (*e.g.*, having lengths of 1 to 1000 nanometers). In some embodiments, however, the term "nanostructure" herein may include micrometer-scale structures (*e.g.*, assembled from more than one nanometer-scale or micrometer-scale structure). In some embodiments, a nanostructure has a dimension (*e.g.*, length or width/diameter) of greater than 500 nm or greater than 1000 nm. In some embodiments, a nanostructure has a dimension of 1 micrometer to 2 micrometers. In some embodiments, a nanostructure has a dimension of 10 to 500 nm, 10 to 450 nm, 10 to 400 nm, 10 to 350 nm, 10 to 300 nm, 10 to 250 nm, 10 to 200 nm, 10 to 150 nm, 10 to 100 nm, 10 to 50 nm, or 10 to 25 nm. In some embodiments, the nanostructure has a dimension of 500 to 450 nm, 500 to 400 nm, 500 to 350 nm, 500 to 300 nm, 500 to 250 nm, 500 to 200 nm, 500 to 150 nm, 500 to 100 nm, 500 to 50 nm, or 500 to 25nm. In some embodiments, the nanostructure has a dimension of 10, 25, 50, 75, 100, 150, 200, 250, 300, 350, 400, 450, or 500 nm.

A nucleic acid nanostructure is considered to "self-assemble." Bottom up, self-assembly refers to the process by which molecules adopt a defined arrangement without guidance or management from an outside source. Although, it should be understood that with synthetic nucleic acid self-assembly, as provided herein, the nucleotide base sequences that guide assembly of nucleic acids are artificially designed, and the corresponding nucleic acids are accordingly synthesized by an outside source, such as one of skill in the art (using, for example, standard nucleic acid synthesis techniques). That is, one of ordinary skill in the art can 'program' nucleotide base sequences within a single nucleic acid strand or between two difference nucleic acid strands to selectively bind to each other in solution based on a strict set of nucleotide base pairing rules *(e.g.,* A binds to T, G binds to C, A does not bind to G or C, T does not bind to G or C). Self-assembly may be intramolecular (folding) or intermolecular.

The nanostructures and, thus, nanostructures, microstructures and macrostructures assembled from smaller nanostructures, are "rationally designed." A nanostructure, as discussed above, does not assemble in nature. Nucleic acid strands for use in crisscross cooperative assembly are 'programmed' such that among a specific population of strands, complementary nucleotide base sequences within the same strand or between two different strands bind selectively to each other to form a complex, user-defined structure, such as a rod/tube, ribbon, lattice, sheet, polyhedral, cube, sphere, or other two-dimensional or three-dimensional shape. A nanostructure may have a regular shape (sides that are all equal and interior angles that are all equal) or an irregular shape (sides and angles of any length and degree).

### Methods of Crisscross Cooperative Assembly

Self-assembly of a nucleating nanostructure and subsets of nanostructures occurs, in some embodiments, in a 'one-pot' reaction, whereby all nucleic acid nanostructures of a crisscross cooperative assembly system are combined in a reaction buffer, and then the reaction buffer is incubated under conditions that result in self-assembly of all of the nucleic acid nanostructures.

Conditions that result in self-assembly of nucleic acid nanostructures of a crisscross cooperative assembly reaction may vary depending on the size, shape, composition and number of nucleic acid nanostructures in a particular reaction. Such conditions may be determined by one of ordinary skill in the art, for example, one who rationally designs/programs the nanostructures to self-assemble.

A crisscross cooperative assembly method may be performed at a variety of temperatures. In some embodiments, a crisscross cooperative assembly method is performed at room temperature (~25 °C) or 37 °C. A crisscross cooperative assembly method may be performed at a temperature lower than 25 °C or higher than 37 °C.

The salt concentration of the reaction buffer in which a crisscross cooperative assembly reaction is performed may also vary. In some embodiments, the reaction buffer comprises MgCl₂ salt at a concentration of 1 mM-10 mM *(e.g.,* 1 mM, 2 mM, 3 mM, 4 mM, 5 mM, 6 mM, 7 mM, 8 mM, 9 mM or 10 mM). In some embodiments, the reaction buffer comprises NaCl at a concentration of 100 mM-500 mM (e.g., 100 mM, 200 mM, 300 mM, 400 mM or 500 mM). In some embodiments, a crisscross cooperative assembly method is performed under high-salt conditions. Thus, in some embodiments, the reaction buffer comprises MgCl₂ salt at a concentration of at least 20 mM *(e.g.,* 20-500 mM, or 20-200 mM). In some embodiments, the reaction buffer comprises NaCl at a concentration of at least 1 M *(e.g.,* 1-2 M, 1-3 M, 1-4 M, or 1-5 M).

In any given reaction, the number of initial nanostructures (drones) exceeds the number of nucleating nanostructures (queens). Thus, in some embodiments, the ratio of nucleating nanostructure to non-nucleating nanostructure (e.g., a drone from an initial subset, or a worker from a subsequent subset) is 1:10 - 1:10¹² (trillion). For example, the ratio of nucleating nanostructure to non-nucleating nanostructure may be 1:10 - 1:1000, 1:10 - 1:500, 1:10 - 1:100, 1:10 - 1:75, 1:10 - 1:50, or 1:10 - 1:25. In some embodiments, the ratio of nucleating nanostructure to non-nucleating nanostructure is 1:1000, 1:500, 1:100, 1:90, 1:80, 1:70, 1:60, 1:50, 1:40, 1:30, 1:20 or 1:10.

In some embodiments, a crisscross cooperative assembly reaction is incubated for 2-96 hours. For example, a crisscross cooperative assembly reaction may be incubated for 2-24 hours, 2-30 hours, 2-36 hours, 2-42 hours, 2-48 hours, 2-54 hours, 2-60 hours, 2-66 hours, 2-72 hours, 2-78 hours, 2-84 hours, 2-90 hours, or 2-96 hours. In some embodiments, a crisscross cooperative assembly reaction is incubated for 3, 6, 9, 12, 15, 18, 21, 24, 27, 30, 33, 36, 39, 42, 45, 48, 51, 54, 57, 60, 63, 66, 69, or 72 hours. In some embodiments, a crisscross cooperative assembly reaction is incubated for 2, 4, 6, 8, 10, 12, 14, 16, 18, 20, 22, 24, 26, 28, 30, 32, 34, 36, 38, 40, 42, 44, 46, 48, 50, 52, 54, 56, 58, 60, 62, 64, 66, 68, 70 or 72 hours.

### Biosensors

In some embodiments, the crisscross assembly products may be used as biosensors that are capable of detecting a selected biomolecule (analyte) using a variety of different mechanisms and the systems described herein. For example, in such systems, the presence of a biomolecule can be used to trigger crisscross assembly, which can then be detected (visualized), indicating the presence of the biomolecule.

The biomolecule may be detected using a ring system. As depicted in FIG. 6, the large DNA ring ("host ring"), single-stranded DNA, may be split to incorporate a biomolecule capture site (analyte test site) to bind macromolecules in biological samples. The DNA ring loops through and encloses a number of discrete, separate "guest" rings, which are single-stranded DNA and function as catenane queens, so that the guest rings are catenated on the host ring, similar to individual beads on a bracelet. In some embodiments, the guest rings are independently formed from separate single-stranded nucleic acids (see, e.g., FIGS. 5 and 6), while in other embodiments, the guest rings are formed from a long single nucleic acid strand assembled into multiple (e.g., vertically stacked) rings (see, e.g., FIGS. 27A and 27B). The number of guest rings can be 2, 3, 4, or 5 or more. In embodiments, each guest ring (catenane queen) comprises binding sites for drone and worker oligonucleotides and is therefore capable of crisscross assembly. In embodiments, the plurality of catenated guest rings when in close proximity forms a catenane queen comprising binding sites (e.g., plug strands) for drone and worker nucleic acids and/or structures and is thus capable of crisscross assembly. The number of binding sites per guest ring can vary, and may be 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95 or 100. A biomolecule test site, located near the biomolecule capture site may also formed.

The presence of the biomolecule, in some embodiments may be detected in mixtures, such as biological samples, as follows. First, a biological sample is mixed with a high concentration of the catenane queen, allowing macromolecules of interest bind the biomolecule capture site. Then, a chemical reaction is used to reversibly cleave the biomolecule capture site. Catenane queens not bound to the target biomolecule will fall apart more quickly compared to those held together by the target biomolecule. The remaining catenane queens in the test mixture are re-ligated at the biomolecule test site. Subsequently, drones and workers are added to the test mixture to amplify remaining intact queens using readily observable micrometer-scale DNA structures. This system is modular, and the biomolecule capture site may be customized to bind disease markers, including proteins or nucleic acid sequences.

Ultraspecific biosensors can also be created by adding a biomolecule detection system to the multiple guest-ring (e.g., guest-loop) catenane systems with DNA slats, as depicted in FIGS. 27-28. In this example, a barrel queen is used; however, other 3-dimensional shapes are also possible (e.g., sheets, blocks and dendrimers). An example of the production of a barrel queen (a rolled sheet) is described above.

An example of a DNA slat is depicted in FIGS. 21A and 21B.

Using a scaffold for DNA origami, for example an M13 scaffold and staple strands, a multiple guest-ring catenane system can be formed. For example, in FIG. 27, an eight-loop system is formed in a one-pot reaction. The number of loops (rings) can be varied, depending on the design of the system, and may be 2, 3, 4, 5, 6, 7, 8, 9, 10, 11, 12, 13, 14, 15, 16, 17, 18, 19, or 20 loops. Additional loops may be used. Unlike the system described above, the handles (elongated structures) and loops (rings) are all part of the same single-stranded DNA (e.g., M13 DNA); the handle structures are programmed to link together by specific staple strands (slats). The system is designed around the specific staple strands/slats; in the presence of biomolecule, they hold the structure together and growth can occur from the parallel loops when drones and workers are added (FIG. 28B). In the absence of biomolecule, the staple strands/slats release the structure, and no growth can occur as the queen falls apart and the binding sites are not close enough for nucleation and growth even in the presence of drones and workers (FIG. 28A). The presence of the structures can be detected using any one of the methods described above, or with any method known in the art.

DNA slats or other nucleic acids of a biosensor may be modified with one or more switchable bridges. A "switchable bridge" is a link between functional groups that forms or breaks in the presence of a particular agent (e.g., reaction agent or dissociation agent). Examples of switchable bridges include bonds formed via a "click chemistry" reaction (e.g., a between an azide and an alkyne), protein-protein binding (e.g., one or more antibodies binding to a target protein/antigen), a disulfide bond (between two thiols).

Thus, some aspects of the present disclosure provide a biosensor comprising (i) a first DNA slat comprising a first functional group (e.g., an azide or alkyne), a first binding partner (e.g., an antibody, aptamer or nanobody), and a second functional group (e.g., a thiol or nucleic acid), and (ii) a second DNA slat comprising a third functional group (e.g., a thiol or nucleic acid), a second binding partner (e.g., an antibody, aptamer or nanobody), and a fourth functional group (e.g., an azide or alkyne), wherein the first and fourth functional groups react in the presence of a reaction agent to form a link (e.g., a covalent link), wherein the first and fourth binding partners bind specifically to a biomolecule of interest to form a link (e.g., non-covalent link), and wherein the second and third functional groups form a link (e.g., a covalent link) that breaks in the presence of a dissociation agent.

In some embodiments, a biosensor comprises a first DNA slat comprising an azide, an antibody, and a thiol group, and a second DNA slat comprising an alkyne, an antibody, and a thiol group, wherein antibody of (i) and the antibody of (ii) bind specifically to a biomolecule of interest.

A "first biomolecule binding partner" and a "second biomolecule binding partner" are any molecules that bind to the same target biomolecule to form a switchable bridge linking DNA slats to each other (via a non-covalent link). In some embodiments, the first and second biomolecule binding partners are proteins or peptides. For example, the first and second biomolecule binding partners may be antibodies that bind to different epitopes of the same antigen. Thus, in some embodiments, the first and second biomolecule binding partners are antibodies (e.g., monoclonal, polyclonal, human, humanized or chimeric). In some embodiments, the first and second biomolecule binding partners are antibody fragments (e.g., Fab, F(ab')2, Fc, scFv, or vhh). The biomolecule binding partners may also be nanobodies or aptamers. Other protein-protein binding partners may be used.

A "first functional group" and a "fourth functional group" are functional groups that react with each other to form a link (bond, such as a covalent bond or a non-covalent bond), which forms a switchable bridge linking the DNA slats to each other. In some embodiments, this bridge is formed through a click chemistry (azide-alkyne cycloaddition) reaction (e.g., V. V. Rostovtsev, et al., Angew. Chem. Int. Ed., 2002, 41, 2596-2599; and F. Himo, et al. J. Am. Chem. Soc., 2005, 127, 210-21 6) . Thus, in some embodiments, one of the first or fourth functional group is an azide, while the other of the first or fourth functional groups is an alkyne. For example, the first functional group may be azide, and the fourth functional group may be *trans*-cyclooctene (TCO). Other click chemistry functional groups may be used.

A "second functional group" and a "third functional group" are functional groups that react with each other to form a link (bond, such as a covalent bond or a non-covalent bond), which forms yet another switchable bridge linking the DNA slats to each other. This bridge breaks (dissociates) in the presence of a dissociation agent. A "dissociation agent" is an agent (e.g., chemical) that breaks the bond (e.g., covalent bond) between the second and third functional groups. In some embodiments, the second and third functional groups are thiol groups that react with each other to form a disulfide bridge. Thus, in some embodiments, the dissociation agent is dithiothreitol (DTT). In some embodiments, the concentration of DTT is 50 mM-200 mM. For example, the concentration of DTT may be 100 mM. Other functional groups may be used.

### EXAMPLES

### Example 1

The examples demonstrates assembly of a nucleating nanostructures (queen). The sharpest bands from a screen of nucleic acid self-assembly reactions were selected and subjected to a 2 minute incubation at 90 °C for denaturing and then an 18 hour ramp. The gel (FIG. 9A) was 2% agarose (10 µL ethidium bromide, c = 10 mg/mL), and run at 60V for 240 minutes in 0.5x TBE and 11 mM MgCl₂. A seesaw experiment was performed, whereby the temperature was varied between 65-60°C (A), 60-55°C (B), 65-55°C (C), and 60-50°C (D). The structures were purified using band excision of the gel, followed by 15 minutes at 16k x g FreezeNSqueeze and then stained with 2% UF following a 2 minute ddH₂O post-wash. The queen folded well, and no noticeable difference was observed between conditions A through D on the seesaw experiment (FIG. 9B).

The experiment was repeated with different nanostructures that function as 'drones'. The sharpest bands from a large screen were selected and subjected to 2 minute incubation at 90°C for denaturing and then an 18 hour ramp. The gel (FIG. 10A) was 2% agarose (10 µL ethidium bromide, c = 10 mg/mL), and run at 60V for 240 minutes in 0.5x TBE and 11mM MgCl₂. In this experiment, the temperature was varied between 70-60°C (A), 65-55°C (B), 65-60°C (C), and 60-55°C (D). The structures were purified using band excision of the gel, followed by 15 minutes at 16k x g FreezeNSqueeze and then stained with 1% UF. The drone folded well, and there was no noticeable difference between conditions A through D on the seesaw experiment (FIG. 10B).

Next, the assembly of a queen together with a drone was examined (FIG. 11). The following conditions were tested (with a 1:1:1 ratio): queen - all sites closed and both drones; queen - site 0 exposed and both drones; queen - site 1 exposed and both drones; queen - site 0/1 exposed and both drones; and queen - all sites exposed and both drones. Assembly was achieved through a 72 hour incubation period at 25 °C, and the structures were purified by band excision of the gel, followed by 15 minutes at 16k x g FreezeNSqueeze followed by 2% UF staining. The samples were run on a 2% agarose gel (10 µL ethidium bromide, c = 10 mg/mL), and run at 60V for 240 minutes in 0.5x TBE and 11mM MgCl₂. Approximately 10-15 ng of each structure were observed.

### Example 2

A similar system was built using single-stranded DNA instead of 6 helix bundles. A schematic of the nucleating nanostructure architecture is shown in FIG. 12A. The single strands each contain binding and linker regions, including a 5 bp binding region and 3 and 5 nucleotide linker (poly T) regions. An exemplary 5 bp, 2 nucleotide linker is shown below:

The workers stack on top of drones in layers (FIGS. 12B-12G). The queen was shown to fold in both 5C or 10C steps (FIG. 12A). Folding occurred after a 2 minute 90°C denaturing period and then an 18 hour ramp. The samples were run on a 2% agarose gel (10 µL ethidium bromide, c = 10 mg/mL), and run at 60V for 240 minutes in 0.5x TBE and 11mM MgCl₂. The queen was incubated under a 50-40°C thermal ramp, with 6mM MgCl₂. The resulting structure is shown in FIG. 13B.

### Example 3

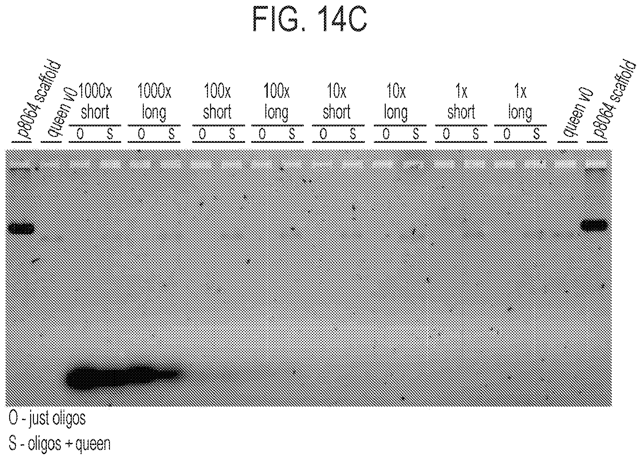

Assembly without the queen and only the workers (both short and long linkers) was examined. In this example, workers did not assemble under conditions with high salt concentration (1M NaCl, up to 15 mM MgCl₂), low temperature (4°C), and a high concentration of workers (3.125 µM). Other conditions, including 10-20 mM PEG and high salt and a high concentration of oligonucleotides were also tested. No assembly occurred. FIGS. 14A-14B show drone/workers at different concentrations successfully assembling with queens. The structures were purified with band excision, 15 minutes at 16k x g FreezeNSqueeze and 2% UF staining. Without the queens, there was no sign of assembly (FIG. 14C).

The duplex length was then increased to 8bp and linker regions of 2 nt (v0.1) and 3 nt (v0.2) were tested. Weaving was introduced into the structure and staple strands were added to constrain the end of the scaffold loops (FIG. 15A). As seen in FIG. 16B, there was no assembly without the queen in any of the groups and under any of the salt concentration conditions.

### Example 4

This Example demonstrates assembly of 6-helix bundle DNA nanorod drones into a crisscross structure via nucleation by a gridiron queen (FIGS. 19A-19D). The gridiron queen has 16 cells, each to which may bind a drone using 5 cooperative plug-socket binding sites. Shown here is site-specific binding of two 440 nm long drones (FIG. 19A), one 250 nm long drone (FIG. 19B), and sixteen 250 nm long drones (FIG. 19C). This example shows binding of drones to the gridiron queen using a 10 bp plug-socket. The agarose gel image in FIG. 19D shows that the queen in the reaction is completely bound by drones, when stoichiometric excess of drones is present. Additionally, functionality of the plug-socket binding system (FIG. 18) is shown with the TEM micrographs of these assemblies and kinetics data (FIGS. 19A-D and FIG. 20).

The gridiron queen and 6-helix bundle drones were conceptualized and then designed using the caDNAno design tool (see FIGS. 16A-16C (and data not shown) for the queen, FIGS. 17A-17B (and data not shown) for the drones). Staple sequences designed in caDNAno were ordered commercially and folded with M13 phage scaffold DNA over the following conditions: drones in 6mM MgCl₂, (90 °C/2mins, 60-50 °C/18 hrs); queen in 8mM MgCl₂, ({(94 °C - 86°C) in 4 °C / 5 min steps}; {(85 °C - 70 °C) in 1 °C / 5 minute steps}; {(70 °C to 40 °C) in 1 °C / 15 minutes steps}; {(40 °C to 25 °C) in 1 °C / 10 minute steps}). The scaffold for the gridiron queen was comprised of a 8634 base genome from M13 phage, and staple DNA sequences were determined by caDNAno. Binding sequences for drones were manually appended to the 3' ends of the staple DNA to bind drones in the desired orientation. The 250 nm and 440 nm 6hb drones were also designed in caDNAno. The scaffold DNA was comprised of either the 8064 base genome from M13 phage (for the 440 nm drone), or a custom 3825 base sequence derived from M13 phage (for the 250 nm drone). Staple DNA sequences were determined by caDNAno and purchased commercially. Scaffold sections for the sockets and plug sequences were customized to determine the orientation and final location of sub-components in assembled structures. The 5' ends of a subset of the staples are truncated to free scaffold so that it could act as a socket to bind plugs. The 3' ends of another subset of staples were appended with plug DNA sequences so that they could interact with other worker subcomponents.

The folded structures were separated from excess folding staples using agarose gel electrophoresis and bands containing the structure of interest were purified from the agarose gel matrix. The purified structures were placed onto carbon grids, stained with 2% uranyl formate, and analyzed by TEM to validate assembly of the correct structure (see FIG. 16C for the queen and FIG. 17B for the drones).

The purified sub-components were assembled into crisscross formation using the following conditions: 0.1 or 0.01 nM queen, 1 nM drones, 30 mM MgCl₂, 45 mM Tris-borate, 1 mM EDTA, and 0.01% Tween-20; incubation at 50 °Celsius for 8-24 hours. Assembly reactions were analyzed using gel electrophoresis and TEM, as shown in FIGS. 19A-19D. Kinetics of binding between the drone and queen are shown in FIG. 20.

### Example 5

A similar system was built using single-stranded DNA (ssDNA) instead of 6 helix bundles, referred to as "crisscross DNA slats" (short: "DNA slats"). A schematic of the base unit is shown in FIGS. 21A-21B. The 21 nucleotide (nt) long oligonucleotide per DNA slat shown in FIGS. 21A-21B allows the 4 by 4 DNA slats array to retains the correct 10.5 bases/turn. The length of the DNA slats can be expanded by repeats of 21 nt, for example, achieving larger structures. FIG. 21A shows an abstraction of the DNA slats architecture and a matrix with the number of base pairs (bp) per binding site at each position. The alternation of 6 bp and 5 bp is used to retain the correct helicity and approximately same binding energy per DNA slat. An exemplary DNA slat strand with 16 binding sites (84 nt) is shown below:

FIG. 21B shows a 3D rendering from the front and cross-section of the DNA slats architecture. The strands weave over and under each other. The DNA slats can reliably tile the ssDNA overhangs (from the M13 scaffold) of different DNA-Origami queens. FIGS. 23A-24B both show examples of ssDNA scaffold being tiled upon and the addition of DNA slats. The flat DNA-Origami queen shown in FIG. 23A is folded through a 2 minute 90°C denaturing period following an 18-hour ramp from 55°C - 50°C with 6 mM MgCl₂. The barrel DNA-Origami queen shown in FIG. 24A is folded by a 15 minute 80°C denaturing period following an 18-hour ramp from 60°C - 25°C with 8 mM MgCl₂. The assembly process of DNA slats with the queen is shown in FIG. 22. Once the queen is folded the crude reaction queen is mixed with the DNA slats, assembly conditions can be tuned by varying the concentrations of DNA slats (100 nM - 1000 nM), salt (5 mM - 30 mM MgCl₂ and 0 - 1 M NaCl), and the temperature of assembly (4 °C - 55 °C). By altering the assembly conditions such as MgCl₂ and the DNA slat concentration the kinetics of the assembly process can be influenced.

### Example 6

This Example shows how extending the DNA slats to create more binding sites facilitates polymerization seeded on the queen. FIG. 25 shows both flat and barrel queen first tiled (as shown in FIGS. 23A-23B and FIGS 24A-24B) and with extended DNA slats seeding the next generation of DNA slats to bind and eventually grow into micron sized structures. The queen nucleating site determines the shape of the subsequently grown structure. FIGS. 26A and 26B show the flat queen assembled in two types of terminal extensions. FIGS. 26C-26E show the flat queen with one, two, and three domain extensions. Samples shown in FIGS. 26A-26E were prepared using crude flat queen reaction (~1 nM), DNA slats (1000 nM/strand), and 15mM MgCh at 50 □ for 2 hours. FIGS. 30A-30B show a staggered design of DNA slats that bind to the flat queen, growing a ribbon like sheet. The subsequent formation of ribbons is shown in FIG. 31. Control reactions, without the flat queen, showed no assembly of the DNA slats after 18 hours of running the reaction. Samples shown in FIG. 31 were prepared using crude flat queen reaction (~9 nM), DNA slats (7500 nM/strand), and 14mM MgCl₂ at 50 □ for -66 hours.

### Example 7

Through the use of the barrel DNA-Origami queen multiple guest-ring catenane systems can be produced in a one-pot reaction. FIGS. 27A-27C show that by folding the barrel queen, a multiple guest-ring catenane system can be achieved through the addition of DNA slats. In order to catenate the loops two DNA slats are needed. A close up view of the DNA slats weaving and catenating the ssDNA M13 scaffold loops is shown in FIG. 27C. By ligating the two DNA slats on one end, a single DNA slat is created that captures all eight loops. A 3D rendering of the purple DNA slat capturing eight loops is shown in FIG. 27D.. FIG. 24 shows the addition of 64 slats, which can simply be reduced, depending on size of the size and number of guest rings. Using the barrel queen with the DNA slats achieves a high yield in a one-pot reaction. The barrel queen can subsequently be transformed into an ultrasensitive biosensor, by coupling a biomolecule detection system to the DNA slats (see, e.g., FIG. 31). Through the integration of proofreading steps, the analyte presence can be transferred into the open or closed state of the purple DNA slat. FIGS. 28A-28B show that without a biomolecule present, the queen falls apart (open DNA slats) and no nucleation of DNA slat mediated growth can occur. The presence of a biomolecule, however, keeps the DNA slats intact and holds the queen structure together, which can then trigger the growth of micron sized tubes, for example, that can subsequently be detected using low-cost optical instruments.

**Table 1. Exemplary Gridiron Queen Staple Sequences**

| **Sequence** | **Comment** | **SEQ ID NO:** |
|---|---|---|
| | 20170216_cc6hb_v3_queen, c0, h21, p0, control, cyan, start 0[94], end 21[94], 42mer | 1 |
| | 20170216_cc6hb_v3_queen, c0, h20, p1, control, cyan, start 1[95], end 20[95], 42mer | 2 |
| | 20170216_cc6hb_v3_queen, c0, h19, p2, control, cyan, start 0[178], end 19[94], 42mer | 3 |
| | 20170216_cc6hb_v3_queen, c0, h18, p3, control, cyan, start 1[179], end 18[95], 42mer | 4 |
| | 20170216_cc6hb_v3_queen, c0, h17, p4, control, cyan, start 0[262], end 17[94], 42mer | 5 |
| | 20170216_cc6hb_v3_queen, c1, h21, p4, control, cyan, start 1[53], end 21[136], 42mer | 6 |
| | 20170216_cc6hb_v3_queen, c1, h20, p3, control, cyan, start 2[136], end 20[137], 42mer | 7 |
| | 20170216_cc6hb_v3_queen, c1, h19, p2, control, cyan, start 1[137], end 19[136], 42mer | 8 |
| | 20170216_cc6hb_v3_queen, c1, hl8, p1, control, cyan, start 2[220], end 18[137], 42mer | 9 |
| | 20170216_cc6hb_v3_queen, c1, h17, p0, control, cyan, start 1[221], end 17[136], 42mer | 10 |
| | 20170216_cc6hb_v3_queen, c2, h21, p0, control, cyan, start 2[94], end 21[178], 42mer | 11 |
| | 20170216_cc6hb_v3_queen, c2, h20, p1, control, cyan, start 3[95], end 20[179], 42mer | 12 |
| | 20170216_cc6hb_v3_queen, c2, h19, p2, control, cyan, start 2[178], end 19[178], 42mer | 13 |
| | 20170216_cc6hb_v3_queen, c2, h18, p3, control, cyan, start 3[179], end 18[179], 42mer | 14 |
| | 20170216_cc6hb_v3_queen, c2, h17, p4, control, cyan, start 2[262], end 17[178], 42mer | 15 |
| | 20170216_cc6hb_v3_queen, c3, h21, p4, control, cyan, start 3[53], end 21[220], 42mer | 16 |
| | 20170216_cc6hb_v3_queen, c3, h20, p3, control, cyan, start 4[136], end 20[221], 42mer | 17 |
| | 20170216_cc6hb_v3_queen, c3, h19, p2, control, cyan, start 3[137], end 19[220], 42mer | 18 |
| | 20170216_cc6hb_v3_queen, c3, hl8, p1, control, cyan, start 4[220], end 18[221], 42mer | 19 |
| | 20170216_cc6hb_v3_queen, c3, h17, p0, control, cyan, start 3[221], end 17[220], 42mer | 20 |
| | 20170216_cc6hb_v3_queen, c4, h21, p0, control, cyan, start 4[94], end 21[262], 42mer | 21 |
| | 20170216_cc6hb_v3_queen, c4, h20, p1, control, cyan, start 5[95], end 20[263], 42mer | 22 |
| | 20170216_cc6hb_v3_queen, c4, h19, p2, control, cyan, start 4[178], end 19[262], 42mer | 23 |
| | 20170216_cc6hb_v3_queen, c4, h18, p3, control, cyan, start 5[179], end 18[263], 42mer | 24 |
| | 20170216_cc6hb_v3_queen, c4, h17, p4, control, cyan, start 4[262], end 17[262], 42mer | 25 |
| | 20170216_cc6hb_v3_queen, c5, h21, p4, control, cyan, start 5[53], end 21[304], 42mer | 26 |
| | 20170216_cc6hb_v3_queen, c5, h20, p3, control, cyan, start 6[136], end 20[305], 42mer | 27 |
| | 20170216_cc6hb_v3_queen, c5, h19, p2, control, cyan, start 5[137], end 19[304], 42mer | 28 |
| | 20170216_cc6hb_v3_queen, c5, hl8, p1, control, cyan, start 6[220], end 18[305], 42mer | 29 |
| | 20170216_cc6hb_v3_queen, c5, h17, p0, control, cyan, start 5[221], end 17[304], 42mer | 30 |
| | 20170216_cc6hb_v3_queen, c6, h21, p0, control, cyan, start 6[94], end 21[346], 42mer | 31 |
| | 20170216_cc6hb_v3_queen, c6, h20, p1, control, cyan, start 7[95], end 20[347], 42mer | 32 |
| | 20170216_cc6hb_v3_queen, c6, h19, p2, control, cyan, start 6[178], end 19[346], 42mer | 33 |
| | 20170216_cc6hb_v3_queen, c6, h18, p3, control, cyan, start 7[179], end 18[347], 42mer | 34 |
| | 20170216_cc6hb_v3_queen, c6, h17, p4, control, cyan, start 6[262], end 17[346], 42mer | 35 |
| | 20170216_cc6hb_v3_queen, c7, h21, p4, control, cyan, start 7[53], end 21[388], 42mer | 36 |
| | 20170216_cc6hb_v3_queen, c7, h20, p3, control, cyan, start 8[136], end 20[389], 42mer | 37 |
| | 20170216_cc6hb_v3_queen, c7, h19, p2, control, cyan, start 7[137], end 19[388], 42mer | 38 |
| | 20170216_cc6hb_v3_queen, c7, hl8, p1, control, cyan, start 8[220], end 18[389], 42mer | 39 |
| | 20170216_cc6hb_v3_queen, c7, h17, p0, control, cyan, start 7[221], end 17[388], 42mer | 40 |
| | 20170216_cc6hb_v3_queen, c8, h21, p0, control, cyan, start 8[94], end 21[430], 42mer | 41 |
| | 20170216_cc6hb_v3_queen, c8, h20, p1, control, cyan, start 9[95], end 20[431], 42mer | 42 |
| | 20170216_cc6hb_v3_queen, c8, h19, p2, control, cyan, start 8[178], end 19[430], 42mer | 43 |
| | 20170216_cc6hb_v3_queen, c8, h18, p3, control, cyan, start 9[179], end 18[431], 42mer | 44 |
| | 20170216_cc6hb_v3_queen, c8, h17, p4, control, cyan, start 8[262], end 17[430], 42mer | 45 |
| | 20170216_cc6hb_v3_queen, c9, h21, p4, control, cyan, start 9[53], end 21[472], 42mer | 46 |
| | 20170216_cc6hb_v3_queen, c9, h20, p3, control, cyan, start 10[136], end 20[473], 42mer | 47 |
| | 20170216_cc6hb_v3_queen, c9, h19, p2, control, cyan, start 9[137], end 19[472], 42mer | 48 |
| | 20170216_cc6hb_v3_queen, c9, h18, p1, control, cyan, start 10[220], end 18[473], 42mer | 49 |
| | 20170216_cc6hb_v3_queen, c9, h17, p0, control, cyan, start 9[221], end 17[472], 42mer | 50 |
| | 20170216_cc6hb_v3_queen, c10, h21, p0, control, cyan, start 10[94], end 21[514], 42mer | 51 |
| | 20170216_cc6hb_v3_queen, c10, h20, p1, control, cyan, start 11[95], end 20[515], 42mer | 52 |
| | 20170216_cc6hb_v3_queen, c10, h19, p2, control, cyan, start 10[178], end 19[514], 42mer | 53 |
| | 20170216_cc6hb_v3_queen, c10, h18, p3, control, cyan, start 11[179], end 18[515], 42mer | 54 |
| | 20170216_cc6hb_v3_queen, c10, h17, p4, control, cyan, start 10[262], end 17[514], 42mer | 55 |
| | 20170216_cc6hb_v3_queen, c11, h21, p4, control, cyan, start 11[53], end 21[556], 42mer | 56 |
| | 20170216_cc6hb_v3_queen, c11, h20, p3, control, cyan, start 12[136], end 20[557], 42mer | 57 |
| | 20170216_cc6hb_v3_queen, c11, h19, p2, control, cyan, start 11[137], end 19[556], 42mer | 58 |
| | 20170216_cc6hb_v3_queen, c11, h18, p1, control, cyan, start 12[220], end 18[557], 42mer | 59 |
| | 20170216_cc6hb_v3_queen, c11, h17, p0, control, cyan, start 11[221], end 17[556], 42mer | 60 |
| | 20170216_cc6hb_v3_queen, c12, h21, p0, control, cyan, start 12[94], end 21[598], 42mer | 61 |
| | 20170216_cc6hb_v3_queen, c12, h20, p1, control, cyan, start 13[95], end 20[599], 42mer | 62 |
| | 20170216_cc6hb_v3_queen, c12, h19, p2, control, cyan, start 12[178], end 19[598], 42mer | 63 |
| | 20170216_cc6hb_v3_queen, c12, h18, p3, control, cyan, start 13[179], end 18[599], 42mer | 64 |
| | 20170216_cc6hb_v3_queen, c12, h17, p4, control, cyan, start 12[262], end 17[598], 42mer | 65 |
| | 20170216_cc6hb_v3_queen, c13, h21, p4, control, cyan, start 13[53], end 21[640], 42mer | 66 |
| | 20170216_cc6hb_v3_queen, c13, h20, p3, control, cyan, start 14[136], end 20[641], 42mer | 67 |
| | 20170216_cc6hb_v3_queen, c13, h19, p2, control, cyan, start 13[137], end 19[640], 42mer | 68 |
| | 20170216_cc6hb_v3_queen, c13, h18, p1, control, cyan, start 14[220], end 18[641], 42mer | 69 |
| | 20170216_cc6hb_v3_queen, c13, h17, p0, control, cyan, start 13[221], end 17[640], 42mer | 70 |
| | 20170216_cc6hb_v3_queen, c14, h21, p0, control, cyan, start 14[94], end 21[682], 42mer | 71 |
| | 20170216_cc6hb_v3_queen, c14, h20, p1, control, cyan, start 15[95], end 20[683], 42mer | 72 |
| | 20170216_cc6hb_v3_queen, c14, h19, p2, control, cyan, start 14[178], end 19[682], 42mer | 73 |
| | 20170216_cc6hb_v3_queen, c14, h18, p3, control, cyan, start 15[179], end 18[683], 42mer | 74 |
| | 20170216_cc6hb_v3_queen, c14, h17, p4, control, cyan, start 14[262], end 17[682], 42mer | 75 |
| | 20170216_cc6hb_v3_queen, c15, h21, p4, control, cyan, start 15[53], end 21[724], 42mer | 76 |
| | 20170216_cc6hb_v3_queen, c15, h20, p3, control, cyan, start 16[136], end 20[725], 42mer | 77 |
| | 20170216_cc6hb_v3_queen, c15, h19, p2, control, cyan, start 15[137], end 19[724], 42mer | 78 |
| | 20170216_cc6hb_v3_queen, c15, h18, p1, control, cyan, start 16[220], end 18[725], 42mer | 79 |
| | 20170216_cc6hb_v3_queen, c15, h17, p0, control, cyan, start 15[221], end 17[724], 42mer | 80 |
| | 20170216_cc6hb_v3_queen, c7, h21, p4, 7bp plug, cyan, start 7[53], end 21[388], 49mer | 81 |
| | 20170216_cc6hb_v3_queen, c7, h20, p3, 7bp plug, cyan, start 8[136], end 20[389], 49mer | 82 |
| | 20170216_cc6hb_v3_queen, c7, h19, p2, 7bp plug, cyan, start 7[137], end 19[388], 49mer | 83 |
| | 20170216_cc6hb_v3_queen, c7, h18, p1, 7bp plug, cyan, start 8[220], end 18[389], 49mer | 84 |
| | 20170216_cc6hb_v3_queen, c7, h17, p0, 7bp plug, cyan, start 7[221], end 17[388], 49mer | 85 |
| | 20170216_cc6hb_v3_queen, c8, h21, p0, 7bp plug, cyan, start 8[94], end 21[430], 49mer | 86 |
| | 20170216_cc6hb_v3_queen, c8, h20, p1, 7bp plug, cyan, start 9[95], end 20[431], 49mer | 87 |
| | 20170216_cc6hb_v3_queen, c8, h19, p2, 7bp plug, cyan, start 8[178], end 19[430], 49mer | 88 |
| | 20170216_cc6hb_v3_queen, c8, h18, p3, 7bp plug, cyan, start 9[179], end 18[431], 49mer | 89 |
| | 20170216_cc6hb_v3_queen, c8, h17, p4, 7bp plug, cyan, start 8[262], end 17[430], 49mer | 90 |
| | 20170216_cc6hb_v3_queen, c7, h21, p4, 10bp plug, cyan, start 7[53], end 21[388], 52mer | 91 |
| | 20170216_cc6hb_v3_queen, c7, h20, p3, 10bp plug, cyan, start 8[136], end 20[389], 52mer | 92 |
| | 20170216_cc6hb_v3_queen, c7, h19, p2, 10bp plug, cyan, start 7[137], end 19[388], 52mer | 93 |
| | 20170216_cc6hb_v3_queen, c7, h18, p1, 10bp plug, cyan, start 8[220], end 18[389], 52mer | 94 |
| | 20170216_cc6hb_v3_queen, c7, h17, p0, 10bp plug, cyan, start 7[221], end 17[388], 52mer | 95 |
| | 20170216_cc6hb_v3_queen, c8, h21, p0, 10bp plug, cyan, start 8[94], end 21[430], 52mer | 96 |
| | 20170216_cc6hb_v3_queen, c8, h20, p1, 10bp plug, cyan, start 9[95], end 20[431], 52mer | 97 |
| | 20170216_cc6hb_v3_queen, c8, h19, p2, 10bp plug, cyan, start 8[178], end 19[430], 52mer | 98 |
| | 20170216_cc6hb_v3_queen, c8, hl8, p3, 10bp plug, cyan, start 9[179], end 18[431], 52mer | 99 |
| | 20170216_cc6hb_v3_queen, c8, h17, p4, 10bp plug, cyan, start 8[262], end 17[430], 52mer | 100 |
| | 20170216_cc6hb_v3_queen, edge, na, na, na, black, start 0[136], end 20[53], 42mer | 101 |
| | 20170216_cc6hb_v3_queen, edge, na, na, na, black, start 0[220], end 18[53], 42mer | 102 |
| | 20170216_cc6hb_v3_queen, edge, na, na, na, black, start 16[94], end 21[766], 42mer | 103 |
| | 20170216_cc6hb_v3_queen, edge, na, na, na, black, start 16[178], end 19[766], 42mer | 104 |
| | 20170216_cc6hb_v3_queen, edge, na, na, na, black, start 16[262], end 17[766], 42mer | 105 |
| | 20170216_cc6hb_v3_queen, edge, na, na, na, black, start 17[53], end 0[221], 42mer | 106 |
| | 20170216_cc6hb_v3_queen, edge, na, na, na, black, start 17[95], end 1[262], 42mer | 107 |
| | 20170216_cc6hb_v3_queen, edge, na, na, na, black, start 17[179], end 3[262], 42mer | 108 |
| | 20170216_cc6hb_v3_queen, edge, na, na, na, black, start 17[263], end 5[262], 42mer | 109 |
| | 20170216_cc6hb_v3_queen, edge, na, na, na, black, start 17[347], end 7[262], 42mer | 110 |
| | 20170216_cc6hb_v3_queen, edge, na, na, na, black, start 17[431], end 9[262], 42mer | 111 |
| | 20170216_cc6hb_v3_queen, edge, na, na, na, black, start 17[515], end 11[262], 42mer | 112 |
| | 20170216_cc6hb_v3_queen, edge, na, na, na, black, start 17[599], end 13[262], 42mer | 113 |
| | 20170216_cc6hb_v3_queen, edge, na, na, na, black, start 17[683], end 15[262], 42mer | 114 |
| | 20170216_cc6hb_v3_queen, edge, na, na, na, black, start 18[766], end 16[179], 42mer | 115 |
| | 20170216_cc6hb_v3_queen, edge, na, na, na, black, start 19[53], end 0[137], 42mer | 116 |
| | 20170216_cc6hb_v3_queen, edge, na, na, na, black, start 20[766], end 16[95], 42mer | 117 |
| | 20170216_cc6hb_v3_queen, edge, na, na, na, black, start 21 [53], end 0[53], 42mer | 118 |
| | 20170216_cc6hb_v3_queen, edge, na, na, na, black, start 21[137], end 2[53], 42mer | 119 |
| | 20170216_cc6hb_v3_queen, edge, na, na, na, black, start 21[221], end 4[53], 42mer | 120 |
| | 20170216_cc6hb_v3_queen, edge, na, na, na, black, start 21[305], end 6[53], 42mer | 121 |
| | 20170216_cc6hb_v3_queen, edge, na, na, na, black, start 21[389], end 8[53], 42mer | 122 |
| | 20170216_cc6hb_v3_queen, edge, na, na, na, black, start 21[473], end 10[53], 42mer | 123 |
| | 20170216_cc6hb_v3_queen, edge, na, na, na, black, start 21 [557], end 12[53], 42mer | 124 |
| | 20170216_cc6hb_v3_queen, edge, na, na, na, black, start 21[641], end 14[53], 42mer | 125 |
| | 20170216_cc6hb_v3_queen, edge, na, na, na, black, start 21[725], end 16[53], 42mer | 126 |
| | 20170216_cc6hb_v3_queen, reference sheet, na, na, na, puke green, start 22[79], end 24[72], 40mer | 127 |
| | 20170216_cc6hb_v3_queen, reference sheet, na, na, na, puke green, start 22[111], end 24[104], 40mer | 128 |
| | 20170216_cc6hb_v3_queen, reference sheet, na, na, na, puke green, start 22[143], end 24[136], 40mer | 129 |
| | 20170216_cc6hb_v3_queen, reference sheet, na, na, na, puke green, start 22[175], end 23[175], 32mer | 130 |
| GTGAGACGGGCGTCTATCA | 20170216_cc6hb_v3_queen, reference sheet, na, na, na, puke green, start 23[53], end 22[56], 19mer | 131 |
| GTGGTTTTTGTTTCCTGTGTGAAA | 20170216_cc6hb_v3_queen, reference sheet, na, na, na, puke green, start 24[71], end 25[79], 24mer | 132 |
| | 20170216_cc6hb_v3_queen, reference sheet, na, na, na, puke green, start 24[87], end 22[80], 40mer | 133 |
| GGGGAGAGATTCCACACAACATAC | 20170216_cc6hb_v3_queen, reference sheet, na, na, na, puke green, start 24[103], end 25[111], 24mer | 134 |
| | 20170216_cc6hb_v3_queen, reference sheet, na, na, na, puke green, start 24[119], end 22[112], 40mer | 135 |
| TGCCAGCTGTGTAAAGCCTGGGGT | 20170216_cc6hb_v3_queen, reference sheet, na, na, na, puke green, start 24[135], end 25[143], 24mer | 136 |
| | 20170216_cc6hb_v3_queen, reference sheet, na, na, na, puke green, start 24[151], end 22[144], 40mer | 137 |
| | 20170216_cc6hb_v3_queen, reference sheet, na, na, na, puke green, start 24[175], end 25[175], 32mer | 138 |
| GTCATAGCTCTTTTCACCA | 20170216_cc6hb_v3_queen, reference sheet, na, na, na, puke green, start 25[56], end 24[53], 19mer | 139 |
| TTGTTATCCGCTCACAGCGGTTTG | 20170216_cc6hb_v3_queen, reference sheet, na, na, na, puke green, start 25[80], end 24[88], 24mer | 140 |
| GAGCCGGAAGCATAAAGCATTAAT | 20170216_cc6hb_v3_queen, reference sheet, na, na, na, puke green, start 25[112], end 24[120], 24mer | 141 |
| GCCTAATGAGTGAGCTGCTTTCCA | 20170216_cc6hb_v3_queen, reference sheet, na, na, na, puke green, start 25[144], end 24[152], 24mer | 142 |
| | 20170216_cc6hb_v3_queen, cell not used, na, na, na, red, start 18[178], end 2[179], 42mer | 143 |
| | 20170216_cc6hb_v3_queen, cell not used, na, na, na, red, start 17[137], end 2[221], 42mer | 144 |
| | 20170216_cc6hb_v3_queen, cell not used, na, na, na, red, start 17[221], end 4[221], 42mer | 145 |
| | 20170216_cc6hb_v3_queen, cell not used, na, na, na, red, start 17[305], end 6[221], 42mer | 146 |
| | 20170216_cc6hb_v3_queen, cell not used, na, na, na, red, start 17[389], end 8[221], 42mer | 147 |
| | 20170216_cc6hb_v3_queen, cell not used, na, na, na, red, start 17[473], end 10[221], 42mer | 148 |
| | 20170216_cc6hb_v3_queen, cell not used, na, na, na, red, start 17[557], end 12[221], 42mer | 149 |
| | 20170216_cc6hb_v3_queen, cell not used, na, na, na, red, start 17[641], end 14[221], 42mer | 150 |
| | 20170216_cc6hb_v3_queen, cell not used, na, na, na, red, start 17[725], end 16[221], 42mer | 151 |
| | 20170216_cc6hb_v3_queen, cell not used, na, na, na, red, start 18[94], end 0[179], 42mer | 152 |
| | 20170216_cc6hb_v3_queen, cell not used, na, na, na, red, start 18[136], end 1[220], 42mer | 153 |
| | 20170216_cc6hb_v3_queen, cell not used, na, na, na, red, start 18[220], end 3[220], 42mer | 154 |
| | 20170216_cc6hb_v3_queen, cell not used, na, na, na, red, start 18[262], end 4[179], 42mer | 155 |
| | 20170216_cc6hb_v3_queen, cell not used, na, na, na, red, start 18[304], end 5[220], 42mer | 156 |
| | 20170216_cc6hb_v3_queen, cell not used, na, na, na, red, start 18[346], end 6[179], 42mer | 157 |
| | 20170216_cc6hb_v3_queen, cell not used, na, na, na, red, start 18[388], end 7[220], 42mer | 158 |
| | 20170216_cc6hb_v3_queen, cell not used, na, na, na, red, start 18[430], end 8[179], 42mer | 159 |
| | 20170216_cc6hb_v3_queen, cell not used, na, na, na, red, start 18[472], end 9[220], 42mer | 160 |
| | 20170216_cc6hb_v3_queen, cell not used, na, na, na, red, start 18[514], end 10[179], 42mer | 161 |
| | 20170216_cc6hb_v3_queen, cell not used, na, na, na, red, start 18[556], end 11[220], 42mer | 162 |
| | 20170216_cc6hb_v3_queen, cell not used, na, na, na, red, start 18[598], end 12[179], 42mer | 163 |
| | 20170216_cc6hb_v3_queen, cell not used, na, na, na, red, start 18[640], end 13[220], 42mer | 164 |
| | 20170216_cc6hb_v3_queen, cell not used, na, na, na, red, start 18[682], end 14[179], 42mer | 165 |
| | 20170216_cc6hb_v3_queen, cell not used, na, na, na, red, start 18[724], end 15[220], 42mer | 166 |
| | 20170216_cc6hb_v3_queen, cell not used, na, na, na, red, start 19[95], end 1[178], 42mer | 167 |
| | 20170216_cc6hb_v3_queen, cell not used, na, na, na, red, start 19[137], end 2[137], 42mer | 168 |
| | 20170216_cc6hb_v3_queen, cell not used, na, na, na, red, start 19[179], end 3[178], 42mer | 169 |
| | 20170216_cc6hb_v3_queen, cell not used, na, na, na, red, start 19[221], end 4[137], 42mer | 170 |
| | 20170216_cc6hb_v3_queen, cell not used, na, na, na, red, start 19[263], end 5[178], 42mer | 171 |
| | 20170216_cc6hb_v3_queen, cell not used, na, na, na, red, start 19[305], end 6[137], 42mer | 172 |
| | 20170216_cc6hb_v3_queen, cell not used, na, na, na, red, start 19[347], end 7[178], 42mer | 173 |
| | 20170216_cc6hb_v3_queen, cell not used, na, na, na, red, start 19[389], end 8[137], 42mer | 174 |
| | 20170216_cc6hb_v3_queen, cell not used, na, na, na, red, start 19[431], end 9[178], 42mer | 175 |
| | 20170216_cc6hb_v3_queen, cell not used, na, na, na, red, start 19[473], end 10[137], 42mer | 176 |
| | 20170216_cc6hb_v3_queen, cell not used, na, na, na, red, start 19[515], end 11[178], 42mer | 177 |
| | 20170216_cc6hb_v3_queen, cell not used, na, na, na, red, start 19[557], end 12[137], 42mer | 178 |
| | 20170216_cc6hb_v3_queen, cell not used, na, na, na, red, start 19[599], end 13[178], 42mer | 179 |
| | 20170216_cc6hb_v3_queen, cell not used, na, na, na, red, start 19[641], end 14[137], 42mer | 180 |
| | 20170216_cc6hb_v3_queen, cell not used, na, na, na, red, start 19[683], end 15[178], 42mer | 181 |
| | 20170216_cc6hb_v3_queen, cell not used, na, na, na, red, start 19[725], end 16[137], 42mer | 182 |
| | 20170216_cc6hb_v3_queen, cell not used, na, na, na, red, start 20[94], end 0[95], 42mer | 183 |
| | 20170216_cc6hb_v3_queen, cell not used, na, na, na, red, start 20[136], end 1[136], 42mer | 184 |
| | 20170216_cc6hb_v3_queen, cell not used, na, na, na, red, start 20[178], end 2[95], 42mer | 185 |
| | 20170216_cc6hb_v3_queen, cell not used, na, na, na, red, start 20[220], end 3[136], 42mer | 186 |
| | 20170216_cc6hb_v3_queen, cell not used, na, na, na, red, start 20[262], end 4[95], 42mer | 187 |
| | 20170216_cc6hb_v3_queen, cell not used, na, na, na, red, start 20[304], end 5[136], 42mer | 188 |
| | 20170216_cc6hb_v3_queen, cell not used, na, na, na, red, start 20[346], end 6[95], 42mer | 189 |
| | 20170216_cc6hb_v3_queen, cell not used, na, na, na, red, start 20[388], end 7[136], 42mer | 190 |
| | 20170216_cc6hb_v3_queen, cell not used, na, na, na, red, start 20[430], end 8[95], 42mer | 191 |
| | 20170216_cc6hb_v3_queen, cell not used, na, na, na, red, start 20[472], end 9[136], 42mer | 192 |
| | 20170216_cc6hb_v3_queen, cell not used, na, na, na, red, start 20[514], end 10[95], 42mer | 193 |
| | 20170216_cc6hb_v3_queen, cell not used, na, na, na, red, start 20[556], end 11[136], 42mer | 194 |
| | 20170216_cc6hb_v3_queen, cell not used, na, na, na, red, start 20[598], end 12[95], 42mer | 195 |
| | 20170216_cc6hb_v3_queen, cell not used, na, na, na, red, start 20[640], end 13[136], 42mer | 196 |
| | 20170216_cc6hb_v3_queen, cell not used, na, na, na, red, start 20[682], end 14[95], 42mer | 197 |
| | 20170216_cc6hb_v3_queen, cell not used, na, na, na, red, start 20[724], end 15[136], 42mer | 198 |
| | 20170216_cc6hb_v3_queen, cell not used, na, na, na, red, start 21 [95], end 1[94], 42mer | 199 |
| | 20170216_cc6hb_v3_queen, cell not used, na, na, na, red, start 21[179], end 3[94], 42mer | 200 |
| | 20170216_cc6hb_v3_queen, cell not used, na, na, na, red, start 21[263], end 5[94], 42mer | 201 |
| | 20170216_cc6hb_v3_queen, cell not used, na, na, na, red, start 21[347], end 7[94], 42mer | 202 |
| | 20170216_cc6hb_v3_queen, cell not used, na, na, na, red, start 21 [431], end 9[94], 42mer | 203 |
| | 20170216_cc6hb_v3_queen, cell not used, na, na, na, red, start 21[515], end 11[94], 42mer | 204 |
| | 20170216_cc6hb_v3_queen, cell not used, na, na, na, red, start 21[599], end 13[94], 42mer | 205 |
| | 20170216_cc6hb_v3_queen, cell not used, na, na, na, red, start 21[683], end 15[94], 42mer | 206 |
| | 20170407_cc6hb_v3-1_queen, c0, h21, p0, 7 bp plug, cyan, start 0[94], end 21[94], 49mer | 207 |
| | 20170407_cc6hb_v3-1_queen, c0, h20, p1, 7 bp plug, cyan, start 1[95], end 20[95], 49mer | 208 |
| | 20170407_cc6hb_v3-1_queen, c0, h19, p2, 7 bp plug, cyan, start 0[178], end 19[94], 49mer | 209 |
| | 20170407_cc6hb_v3-1_queen, c0, h18, p3, 7 bp plug, cyan, start 1[179], end 18[95], 49mer | 210 |
| | 20170407_cc6hb_v3-1_queen, c0, h17, p4, 7 bp plug, cyan, start 0[262], end 17[94], 49mer | 211 |
| | 20170407_cc6hb_v3-1_queen, c1, h21, p4, 7 bp plug, cyan, start 1[53], end 21[136], 49mer | 212 |
| | 20170407_cc6hb_v3-1_queen, c1, h20, p3, 7 bp plug, cyan, start 2[136], end 20[137], 49mer | 213 |
| | 20170407_cc6hb_v3-1_queen, c1, h19, p2, 7 bp plug, cyan, start 1[137], end 19[136], 49mer | 214 |
| | 20170407_cc6hb_v3-1_queen, c1, h18, p1, 7 bp plug, cyan, start 2[220], end 18[137], 49mer | 215 |
| | 20170407_cc6hb_v3-1_queen, c1, h17, p0, 7 bp plug, cyan, start 1[221], end 17[136], 49mer | 216 |
| | 20170407_cc6hb_v3-1_queen, c2, h21, p0, 7 bp plug, cyan, start 2[94], end 21[178], 49mer | 217 |
| | 20170407_cc6hb_v3-1_queen, c2, h20, p1, 7 bp plug, cyan, start 3[95], end 20[179], 49mer | 218 |
| | 20170407_cc6hb_v3-1_queen, c2, h19, p2, 7 bp plug, cyan, start 2[178], end 19[178], 49mer | 219 |
| | 20170407_cc6hb_v3-1_queen, c2, h18, p3, 7 bp plug, cyan, start 3[179], end 18[179], 49mer | 220 |
| | 20170407_cc6hb_v3-1_queen, c2, h17, p4, 7 bp plug, cyan, start 2[262], end 17[178], 49mer | 221 |
| | 20170407_cc6hb_v3-1_queen, c3, h21, p4, 7 bp plug, cyan, start 3[53], end 21[220], 49mer | 222 |
| | 20170407_cc6hb_v3-1_queen, c3, h20, p3, 7 bp plug, cyan, start 4[136], end 20[221], 49mer | 223 |
| | 20170407_cc6hb_v3-1_queen, c3, hl9, p2, 7 bp plug, cyan, start 3[137], end 19[220], 49mer | 224 |
| | 20170407_cc6hb_v3-1_queen, c3, h18, p1, 7 bp plug, cyan, start 4[220], end 18[221], 49mer | 225 |
| | 20170407_cc6hb_v3-1_queen, c3, h17, p0, 7 bp plug, cyan, start 3[221], end 17[220], 49mer | 226 |
| | 20170407_cc6hb_v3-1_queen, c4, h21, p0, 7 bp plug, cyan, start 4[94], end 21[262], 49mer | 227 |
| | 20170407_cc6hb_v3-1_queen, c4, h20, p1, 7 bp plug, cyan, start 5[95], end 20[263], 49mer | 228 |
| | 20170407_cc6hb_v3-1_queen, c4, h19, p2, 7 bp plug, cyan, start 4[178], end 19[262], 49mer | 229 |
| | 20170407_cc6hb_v3-1_queen, c4, h18, p3, 7 bp plug, cyan, start 5[179], end 18[263], 49mer | 230 |
| | 20170407_cc6hb_v3-1_queen, c4, h17, p4, 7 bp plug, cyan, start 4[262], end 17[262], 49mer | 231 |
| | 20170407_cc6hb_v3-1_queen, c5, h21, p4, 7 bp plug, cyan, start 5[53], end 21[304], 49mer | 232 |
| | 20170407_cc6hb_v3-1_queen, c5, h20, p3, 7 bp plug, cyan, start 6[136], end 20[305], 49mer | 233 |
| | 20170407_cc6hb_v3-1_queen, c5, h19, p2, 7 bp plug, cyan, start 5[137], end 19[304], 49mer | 234 |
| | 20170407_cc6hb_v3-1_queen, c5, h18, p1, 7 bp plug, cyan, start 6[220], end 18[305], 49mer | 235 |
| | 20170407_cc6hb_v3-1_queen, c5, h17, p0, 7 bp plug, cyan, start 5[221], end 17[304], 49mer | 236 |
| | 20170407_cc6hb_v3-1_queen, c6, h21, p0, 7 bp plug, cyan, start 6[94], end 21[346], 49mer | 237 |
| | 20170407_cc6hb_v3-1_queen, c6, h20, p1, 7 bp plug, cyan, start 7[95], end 20[347], 49mer | 238 |
| | 20170407_cc6hb_v3-1_queen, c6, h19, p2, 7 bp plug, cyan, start 6[178], end 19[346], 49mer | 239 |
| | 20170407_cc6hb_v3-1_queen, c6, hl8, p3, 7 bp plug, cyan, start 7[179], end 18[347], 49mer | 240 |
| | 20170407_cc6hb_v3-1_queen, c6, h17, p4, 7 bp plug, cyan, start 6[262], end 17[346], 49mer | 241 |
| | 20170407_cc6hb_v3-1_queen, c9, h21, p4, 7 bp plug, cyan, start 9[53], end 21[472], 49mer | 242 |
| | 20170407_cc6hb_v3-1_queen, c9, h20, p3, 7 bp plug, cyan, start 10[136], end 20[473], 49mer | 243 |
| | 20170407_cc6hb_v3-1_queen, c9, h19, p2, 7 bp plug, cyan, start 9[137], end 19[472], 49mer | 244 |
| | 20170407_cc6hb_v3-1_queen, c9, h18, p1, 7 bp plug, cyan, start 10[220], end 18[473], 49mer | 245 |
| | 20170407_cc6hb_v3-1_queen, c9, h17, p0, 7 bp plug, cyan, start 9[221], end 17[472], 49mer | 246 |
| | 20170407_cc6hb_v3-1_queen, c10, h21, p0, 7 bp plug, cyan, start 10[94], end 21[514], 49mer | 247 |
| | 20170407_cc6hb_v3-1_queen, c10, h20, p1, 7 bp plug, cyan, start 11[95], end 20[515], 49mer | 248 |
| | 20170407_cc6hb_v3-1_queen, c10, hl9, p2, 7 bp plug, cyan, start 10[178], end 19[514], 49mer | 249 |
| | 20170407_cc6hb_v3-1_queen, c10, hl8, p3, 7 bp plug, cyan, start 11[179], end 18[515], 49mer | 250 |
| | 20170407_cc6hb_v3-1_queen, c10, hl7, p4, 7 bp plug, cyan, start 10[262], end 17[514], 49mer | 251 |
| | 20170407_cc6hb_v3-1_queen, c11, h21, p4, 7 bp plug, cyan, start 11[53], end 21[556], 49mer | 252 |
| | 20170407_cc6hb_v3-1_queen, c11, h20, p3, 7 bp plug, cyan, start 12[136], end 20[557], 49mer | 253 |
| | 20170407_cc6hb_v3-1_queen, c11, h19, p2, 7 bp plug, cyan, start 11[137], end 19[556], 49mer | 254 |
| | 20170407_cc6hb_v3-1_queen, c11, h18, p1, 7 bp plug, cyan, start 12[220], end 18[557], 49mer | 255 |
| | 20170407_cc6hb_v3-1_queen, c11, h17, p0, 7 bp plug, cyan, start 11[221], end 17[556], 49mer | 256 |
| | 20170407_cc6hb_v3-1_queen, c12, h21, p0, 7 bp plug, cyan, start 12[94], end 21[598], 49mer | 257 |
| | 20170407_cc6hb_v3-1_queen, c12, h20, p1, 7 bp plug, cyan, start 13[95], end 20[599], 49mer | 258 |
| | 20170407_cc6hb_v3-1_queen, c12, hl9, p2, 7 bp plug, cyan, start 12[178], end 19[598], 49mer | 259 |
| | 20170407_cc6hb_v3-1_queen, c12, hl8, p3, 7 bp plug, cyan, start 13[179], end 18[599], 49mer | 260 |
| | 20170407_cc6hb_v3-1_queen, c12, hl7, p4, 7 bp plug, cyan, start 12[262], end 17[598], 49mer | 261 |
| | 20170407_cc6hb_v3-1_queen, c13, h21, p4, 7 bp plug, cyan, start 13[53], end 21[640], 49mer | 262 |
| | 20170407_cc6hb_v3-1_queen, c13, h20, p3, 7 bp plug, cyan, start 14[136], end 20[641], 49mer | 263 |
| | 20170407_cc6hb_v3-1_queen, c13, h19, p2, 7 bp plug, cyan, start 13[137], end 19[640], 49mer | 264 |
| | 20170407_cc6hb_v3-1_queen, c13, h18, p1, 7 bp plug, cyan, start 14[220], end 18[641], 49mer | 265 |
| | 20170407_cc6hb_v3-1_queen, c13, h17, p0, 7 bp plug, cyan, start 13[221], end 17[640], 49mer | 266 |
| | 20170407_cc6hb_v3-1_queen, c14, h21, p0, 7 bp plug, cyan, start 14[94], end 21[682], 49mer | 267 |
| | 20170407_cc6hb_v3-1_queen, c14, h20, p1, 7 bp plug, cyan, start 15[95], end 20[683], 49mer | 268 |
| | 20170407_cc6hb_v3-1_queen, c14, hl9, p2, 7 bp plug, cyan, start 14[178], end 19[682], 49mer | 269 |
| | 20170407_cc6hb_v3-1_queen, c14, hl8, p3, 7 bp plug, cyan, start 15[179], end 18[683], 49mer | 270 |
| | 20170407_cc6hb_v3-1_queen, c14, hl7, p4, 7 bp plug, cyan, start 14[262], end 17[682], 49mer | 271 |
| | 20170407_cc6hb_v3-1_queen, c15, h21, p4, 7 bp plug, cyan, start 15[53], end 21[724], 49mer | 272 |
| | 20170407_cc6hb_v3-1_queen, c15, h20, p3, 7 bp plug, cyan, start 16[136], end 20[725], 49mer | 273 |
| | 20170407_cc6hb_v3-1_queen, c15, h19, p2, 7 bp plug, cyan, start 15[137], end 19[724], 49mer | 274 |
| | 20170407_cc6hb_v3-1_queen, c15, h18, p1, 7 bp plug, cyan, start 16[220], end 18[725], 49mer | 275 |
| | 20170407_cc6hb_v3-1_queen, c15, h17, p0, 7 bp plug, cyan, start 15[221], end 17[724], 49mer | 276 |
| | 20170407_cc6hb_v3-1_queen, c0, h21, p0, 10 bp plug, cyan, start 0[94], end 21[94], 52mer | 277 |
| | 20170407_cc6hb_v3-1_queen, c0, h20, p1, 10 bp plug, cyan, start 1[95], end 20[95], 52mer | 278 |
| | 20170407_cc6hb_v3-1_queen, c0, h19, p2, 10 bp plug, cyan, start 0[178], end 19[94], 52mer | 279 |
| | 20170407_cc6hb_v3-1_queen, c0, h18, p3, 10 bp plug, cyan, start 1[179], end 18[95], 52mer | 280 |
| | 20170407_cc6hb_v3-1_queen, c0, h17, p4, 10 bp plug, cyan, start 0[262], end 17[94], 52mer | 281 |
| | 20170407_cc6hb_v3-1_queen, c1, h21, p4, 10 bp plug, cyan, start 1[53], end 21[136], 52mer | 282 |
| | 20170407_cc6hb_v3-1_queen, c1, h20, p3, 10 bp plug, cyan, start 2[136], end 20[137], 52mer | 283 |
| | 20170407_cc6hb_v3-1_queen, c1, h19, p2, 10 bp plug, cyan, start 1[137], end 19[136], 52mer | 284 |
| | 20170407_cc6hb_v3-1_queen, c1, h18, p1, 10 bp plug, cyan, start 2[220], end 18[137], 52mer | 285 |
| | 20170407_cc6hb_v3-1_queen, c1, h17, p0, 10 bp plug, cyan, start 1[221], end 17[136], 52mer | 286 |
| | 20170407_cc6hb_v3-1_queen, c2, h21, p0, 10 bp plug, cyan, start 2[94], end 21[178], 52mer | 287 |
| | 20170407_cc6hb_v3-1_queen, c2, h20, p1, 10 bp plug, cyan, start 3[95], end 20[179], 52mer | 288 |
| | 20170407_cc6hb_v3-1_queen, c2, h19, p2, 10 bp plug, cyan, start 2[178], end 19[178], 52mer | 289 |
| | 20170407_cc6hb_v3-1_queen, c2, hl8, p3, 10 bp plug, cyan, start 3[179], end 18[179], 52mer | 290 |
| | 20170407_cc6hb_v3-1_queen, c2, h17, p4, 10 bp plug, cyan, start 2[262], end 17[178], 52mer | 291 |
| | 20170407_cc6hb_v3-1_queen, c3, h21, p4, 10 bp plug, cyan, start 3[53], end 21[220], 52mer | 292 |
| | 20170407_cc6hb_v3-1_queen, c3, h20, p3, 10 bp plug, cyan, start 4[136], end 20[221], 52mer | 293 |
| | 20170407_cc6hb_v3-1_queen, c3, h19, p2, 10 bp plug, cyan, start 3[137], end 19[220], 52mer | 294 |
| | 20170407_cc6hb_v3-1_queen, c3, h18, p1, 10 bp plug, cyan, start 4[220], end 18[221], 52mer | 295 |
| | 20170407_cc6hb_v3-1_queen, c3, h17, p0, 10 bp plug, cyan, start 3[221], end 17[220], 52mer | 296 |
| | 20170407_cc6hb_v3-1_queen, c4, h21, p0, 10 bp plug, cyan, start 4[94], end 21[262], 52mer | 297 |
| | 20170407_cc6hb_v3-1_queen, c4, h20, p1, 10 bp plug, cyan, start 5[95], end 20[263], 52mer | 298 |
| | 20170407_cc6hb_v3-1_queen, c4, h19, p2, 10 bp plug, cyan, start 4[178], end 19[262], 52mer | 299 |
| | 20170407_cc6hb_v3-1_queen, c4, hl8, p3, 10 bp plug, cyan, start 5[179], end 18[263], 52mer | 300 |
| | 20170407_cc6hb_v3-1_queen, c4, h17, p4, 10 bp plug, cyan, start 4[262], end 17[262], 52mer | 301 |
| | 20170407_cc6hb_v3-1_queen, c5, h21, p4, 10 bp plug, cyan, start 5[53], end 21[304], 52mer | 302 |
| | 20170407_cc6hb_v3-1_queen, c5, h20, p3, 10 bp plug, cyan, start 6[136], end 20[305], 52mer | 303 |
| | 20170407_cc6hb_v3-1_queen, c5, h19, p2, 10 bp plug, cyan, start 5[137], end 19[304], 52mer | 304 |
| | 20170407_cc6hb_v3-1_queen, c5, h18, p1, 10 bp plug, cyan, start 6[220], end 18[305], 52mer | 305 |
| | 20170407_cc6hb_v3-1_queen, c5, h17, p0, 10 bp plug, cyan, start 5[221], end 17[304], 52mer | 306 |
| | 20170407_cc6hb_v3-1_queen, c6, h21, p0, 10 bp plug, cyan, start 6[94], end 21[346], 52mer | 307 |
| | 20170407_cc6hb_v3-1_queen, c6, h20, p1, 10 bp plug, cyan, start 7[95], end 20[347], 52mer | 308 |
| | 20170407_cc6hb_v3-1_queen, c6, h19, p2, 10 bp plug, cyan, start 6[178], end 19[346], 52mer | 309 |
| | 20170407_cc6hb_v3-1_queen, c6, hl8, p3, 10 bp plug, cyan, start 7[179], end 18[347], 52mer | 310 |
| | 20170407_cc6hb_v3-1_queen, c6, h17, p4, 10 bp plug, cyan, start 6[262], end 17[346], 52mer | 311 |
| | 20170407_cc6hb_v3-1_queen, c9, h21, p4, 10 bp plug, cyan, start 9[53], end 21[472], 52mer | 312 |
| | 20170407_cc6hb_v3-1_queen, c9, h20, p3, 10 bp plug, cyan, start 10[136], end 20[473], 52mer | 313 |
| | 20170407_cc6hb_v3-1_queen, c9, h19, p2, 10 bp plug, cyan, start 9[137], end 19[472], 52mer | 314 |
| | 20170407_cc6hb_v3-1_queen, c9, h18, p1, 10 bp plug, cyan, start 10[220], end 18[473], 52mer | 315 |
| | 20170407_cc6hb_v3-1_queen, c9, h17, p0, 10 bp plug, cyan, start 9[221], end 17[472], 52mer | 316 |
| | 20170407_cc6hb_v3-1_queen, c10, h21, p0, 10 bp plug, cyan, start 10[94], end 21[514], 52mer | 317 |
| | 20170407_cc6hb_v3-1_queen, c10, h20, p1, 10 bp plug, cyan, start 11[95], end 20[515], 52mer | 318 |
| | 20170407_cc6hb_v3-1_queen, c10, h19, p2, 10 bp plug, cyan, start 10[178], end 19[514], 52mer | 319 |
| | 20170407_cc6hb_v3-1_queen, c10, hl8, p3, 10 bp plug, cyan, start 11[179], end 18[515], 52mer | 320 |
| | 20170407_cc6hb_v3-1_queen, c10, h17, p4, 10 bp plug, cyan, start 10[262], end 17[514], 52mer | 321 |
| | 20170407_cc6hb_v3-1_queen, c11, h21, p4, 10 bp plug, cyan, start 11[53], end 21[556], 52mer | 322 |
| | 20170407_cc6hb_v3-1_queen, c11, h20, p3, 10 bp plug, cyan, start 12[136], end 20[557], 52mer | 323 |
| | 20170407_cc6hb_v3-1_queen, c11, h19, p2, 10 bp plug, cyan, start 11[137], end 19[556], 52mer | 324 |
| | 20170407_cc6hb_v3-1_queen, c11, h18, p1, 10 bp plug, cyan, start 12[220], end 18[557], 52mer | 325 |
| | 20170407_cc6hb_v3-1_queen, c11, h17, p0, 10 bp plug, cyan, start 11[221], end 17[556], 52mer | 326 |
| | 20170407_cc6hb_v3-1_queen, c12, h21, p0, 10 bp plug, cyan, start 12[94], end 21[598], 52mer | 327 |
| | 20170407_cc6hb_v3-1_queen, c12, h20, p1, 10 bp plug, cyan, start 13[95], end 20[599], 52mer | 328 |
| | 20170407_cc6hb_v3-1_queen, c12, h19, p2, 10 bp plug, cyan, start 12[178], end 19[598], 52mer | 329 |
| | 20170407_cc6hb_v3-1_queen, c12, hl8, p3, 10 bp plug, cyan, start 13[179], end 18[599], 52mer | 330 |
| | 20170407_cc6hb_v3-1_queen, c12, h17, p4, 10 bp plug, cyan, start 12[262], end 17[598], 52mer | 331 |
| | 20170407_cc6hb_v3-1_queen, c13, h21, p4, 10 bp plug, cyan, start 13[53], end 21[640], 52mer | 332 |
| | 20170407_cc6hb_v3-1_queen, c13, h20, p3, 10 bp plug, cyan, start 14[136], end 20[641], 52mer | 333 |
| | 20170407_cc6hb_v3-1_queen, c13, h19, p2, 10 bp plug, cyan, start 13[137], end 19[640], 52mer | 334 |
| | 20170407_cc6hb_v3-1_queen, c13, h18, p1, 10 bp plug, cyan, start 14[220], end 18[641], 52mer | 335 |
| | 20170407_cc6hb_v3-1_queen, c13, h17, p0, 10 bp plug, cyan, start 13[221], end 17[640], 52mer | 336 |
| | 20170407_cc6hb_v3-1_queen, c14, h21, p0, 10 bp plug, cyan, start 14[94], end 21[682], 52mer | 337 |
| | 20170407_cc6hb_v3-1_queen, c14, h20, p1, 10 bp plug, cyan, start 15[95], end 20[683], 52mer | 338 |
| | 20170407_cc6hb_v3-1_queen, c14, h19, p2, 10 bp plug, cyan, start 14[178], end 19[682], 52mer | 339 |
| | 20170407_cc6hb_v3-1_queen, c14, hl8, p3, 10 bp plug, cyan, start 15[179], end 18[683], 52mer | 340 |
| | 20170407_cc6hb_v3-1_queen, c14, h17, p4, 10 bp plug, cyan, start 14[262], end 17[682], 52mer | 341 |
| | 20170407_cc6hb_v3-1_queen, c15, h21, p4, 10 bp plug, cyan, start 15[53], end 21[724], 52mer | 342 |
| | 20170407_cc6hb_v3-1_queen, c15, h20, p3, 10 bp plug, cyan, start 16[136], end 20[725], 52mer | 343 |
| | 20170407_cc6hb_v3-1_queen, c15, h19, p2, 10 bp plug, cyan, start 15[137], end 19[724], 52mer | 344 |
| | 20170407_cc6hb_v3-1_queen, c15, h18, p1, 10 bp plug, cyan, start 16[220], end 18[725], 52mer | 345 |
| | 20170407_cc6hb_v3-1_queen, c15, h17, p0, 10 bp plug, cyan, start 15[221], end 17[724], 52mer | 346 |

**Table 2. Exemplary 250nm Six-helix Bundle Sequences**

| **Sequence** | **Comment** | **SEQ ID NO:** |
|---|---|---|
| | 20170608 cc6hb v4 - base 250nm 6hb, grey standard seq, start 0[41], end 4[21], 49mer | 347 |
| | 20170608 cc6hb v4 - base 250nm 6hb, grey standard seq, start 0[83], end 4[70], 42mer | 348 |
| | 20170608 cc6hb v4 - base 250nm 6hb, grey standard seq, start 0[125], end 4[112], 42mer | 349 |
| | 20170608 cc6hb v4 - base 250nm 6hb, grey standard seq, start 0[167], end 4[154], 42mer | 350 |
| | 20170608 cc6hb v4 - base 250nm 6hb, grey standard seq, start 0[209], end 4[196], 42mer | 351 |
| | 20170608 cc6hb v4 - base 250nm 6hb, grey standard seq, start 0[251], end 4[238], 42mer | 352 |
| | 20170608 cc6hb v4 - base 250nm 6hb, grey standard seq, start 0[293], end 4[280], 42mer | 353 |
| | 20170608 cc6hb v4 - base 250nm 6hb, grey standard seq, start 0[335], end 4[322], 42mer | 354 |
| | 20170608 cc6hb v4 - base 250nm 6hb, grey standard seq, start 0[377], end 4[364], 42mer | 355 |
| | 20170608 cc6hb v4 - base 250nm 6hb, grey standard seq, start 0[419], end 4[406], 42mer | 356 |
| | 20170608 cc6hb v4 - base 250nm 6hb, grey standard seq, start 0[461], end 4[448], 42mer | 357 |
| | 20170608 cc6hb v4 - base 250nm 6hb, grey standard seq, start 0[503], end 4[490], 42mer | 358 |
| | 20170608 cc6hb v4 - base 250nm 6hb, grey standard seq, start 0[545], end 4[532], 42mer | 359 |
| | 20170608 cc6hb v4 - base 250nm 6hb, grey standard seq, start 0[587], end 4[574], 42mer | 360 |
| | 20170608 cc6hb v4 - base 250nm 6hb, grey standard seq, start 0[629], end 4[616], 42mer | 361 |
| | 20170608 cc6hb v4 - base 250nm 6hb, grey standard seq, start 0[671], end 4[658], 42mer | 362 |
| | 20170608 cc6hb v4 - base 250nm 6hb, grey standard seq, start 0[713], end 4[700], 42mer | 363 |
| | 20170608 cc6hb v4 - base 250nm 6hb, grey standard seq, start 0[755], end 4[742], 42mer | 364 |
| GAACAAACGGCGGATTGACAATAATTCG | 20170608 cc6hb v4 - base 250nm 6hb, grey standard seq, start 1[7], end 1[34], 28mer | 365 |
| | 20170608 cc6hb v4 - base 250nm 6hb, grey standard seq, start 1[35], end 1[76], 42mer | 366 |
| | 20170608 cc6hb v4 - base 250nm 6hb, grey standard seq, start 1[77], end 1[118], 42mer | 367 |
| | 20170608 cc6hb v4 - base 250nm 6hb, grey standard seq, start 1[119], end 1[160], 42mer | 368 |
| | 20170608 cc6hb v4 - base 250nm 6hb, grey standard seq, start 1[161], end 1[202], 42mer | 369 |
| | 20170608 cc6hb v4 - base 250nm 6hb, grey standard seq, start 1[203], end 1[244], 42mer | 370 |
| | 20170608 cc6hb v4 - base 250nm 6hb, grey standard seq, start 1[245], end 1[286], 42mer | 371 |
| | 20170608 cc6hb v4 - base 250nm 6hb, grey standard seq, start 1[287], end 1[328], 42mer | 372 |
| | 20170608 cc6hb v4 - base 250nm 6hb, grey standard seq, start 1[329], end 1[370], 42mer | 373 |
| | 20170608 cc6hb v4 - base 250nm 6hb, grey standard seq, start 1[371], end 1[412], 42mer | 374 |
| | 20170608 cc6hb v4 - base 250nm 6hb, grey standard seq, start 1[413], end 1[454], 42mer | 375 |
| | 20170608 cc6hb v4 - base 250nm 6hb, grey standard seq, start 1[455], end 1[496], 42mer | 376 |
| | 20170608 cc6hb v4 - base 250nm 6hb, grey standard seq, start 1[497], end 1[538], 42mer | 377 |
| | 20170608 cc6hb v4 - base 250nm 6hb, grey standard seq, start 1[539], end 1[580], 42mer | 378 |
| | 20170608 cc6hb v4 - base 250nm 6hb, grey standard seq, start 1[581], end 1[622], 42mer | 379 |
| | 20170608 cc6hb v4 - base 250nm 6hb, grey standard seq, start 1[623], end 1[664], 42mer | 380 |
| | 20170608 cc6hb v4 - base 250nm 6hb, grey standard seq, start 1[665], end 1[706], 42mer | 381 |
| | 20170608 cc6hb v4 - base 250nm 6hb, grey standard seq, start 1[707], end 1[755], 49mer | 382 |
| | 20170608 cc6hb v4 - base 250nm 6hb, grey standard seq, start 2[55], end 2[14], 42mer | 383 |
| | 20170608 cc6hb v4 - base 250nm 6hb, grey standard seq, start 3[14], end 3[48], 35mer | 384 |
| | 20170608 cc6hb v4 - base 250nm 6hb, grey standard seq, start 3[49], end 3[90], 42mer | 385 |
| | 20170608 cc6hb v4 - base 250nm 6hb, grey standard seq, start 3[91], end 3[132], 42mer | 386 |
| | 20170608 cc6hb v4 - base 250nm 6hb, grey standard seq, start 3[133], end 3[174], 42mer | 387 |
| | 20170608 cc6hb v4 - base 250nm 6hb, grey standard seq, start 3[175], end 3[216], 42mer | 388 |
| | 20170608 cc6hb v4 - base 250nm 6hb, grey standard seq, start 3[217], end 3[258], 42mer | 389 |
| | 20170608 cc6hb v4 - base 250nm 6hb, grey standard seq, start 3[259], end 3[300], 42mer | 390 |
| | 20170608 cc6hb v4 - base 250nm 6hb, grey standard seq, start 3[301], end 3[342], 42mer | 391 |
| | 20170608 cc6hb v4 - base 250nm 6hb, grey standard seq, start 3[343], end 3[384], 42mer | 392 |
| | 20170608 cc6hb v4 - base 250nm 6hb, grey standard seq, start 3[385], end 3[426], 42mer | 393 |
| | 20170608 cc6hb v4 - base 250nm 6hb, grey standard seq, start 3[427], end 3[468], 42mer | 394 |
| | 20170608 cc6hb v4 - base 250nm 6hb, grey standard seq, start 3[469], end 3[510], 42mer | 395 |
| | 20170608 cc6hb v4 - base 250nm 6hb, grey standard seq, start 3[511], end 3[552], 42mer | 396 |
| | 20170608 cc6hb v4 - base 250nm 6hb, grey standard seq, start 3[553], end 3[594], 42mer | 397 |
| | 20170608 cc6hb v4 - base 250nm 6hb, grey standard seq, start 3[595], end 3[636], 42mer | 398 |
| | 20170608 cc6hb v4 - base 250nm 6hb, grey standard seq, start 3[637], end 3[678], 42mer | 399 |
| | 20170608 cc6hb v4 - base 250nm 6hb, grey standard seq, start 3[679], end 3[720], 42mer | 400 |
| | 20170608 cc6hb v4 - base 250nm 6hb, grey standard seq, start 3[721], end 2[756], 49mer | 401 |
| | 20170608 cc6hb v4 - base 250nm 6hb, grey standard seq, start 4[69], end 5[55], 42mer | 402 |
| | 20170608 cc6hb v4 - base 250nm 6hb, grey standard seq, start 4[111], end 5[97], 42mer | 403 |
| | 20170608 cc6hb v4 - base 250nm 6hb, grey standard seq, start 4[153], end 5[139], 42mer | 404 |
| | 20170608 cc6hb v4 - base 250nm 6hb, grey standard seq, start 4[195], end 5[181], 42mer | 405 |
| | 20170608 cc6hb v4 - base 250nm 6hb, grey standard seq, start 4[237], end 5[223], 42mer | 406 |
| | 20170608 cc6hb v4 - base 250nm 6hb, grey standard seq, start 4[279], end 5[265], 42mer | 407 |
| | 20170608 cc6hb v4 - base 250nm 6hb, grey standard seq, start 4[321], end 5[307], 42mer | 408 |
| | 20170608 cc6hb v4 - base 250nm 6hb, grey standard seq, start 4[363], end 5[349], 42mer | 409 |
| | 20170608 cc6hb v4 - base 250nm 6hb, grey standard seq, start 4[405], end 5[391], 42mer | 410 |
| | 20170608 cc6hb v4 - base 250nm 6hb, grey standard seq, start 4[447], end 5[433], 42mer | 411 |
| | 20170608 cc6hb v4 - base 250nm 6hb, grey standard seq, start 4[489], end 5[475], 42mer | 412 |
| | 20170608 cc6hb v4 - base 250nm 6hb, grey standard seq, start 4[531], end 5[517], 42mer | 413 |
| | 20170608 cc6hb v4 - base 250nm 6hb, grey standard seq, start 4[573], end 5[559], 42mer | 414 |
| | 20170608 cc6hb v4 - base 250nm 6hb, grey standard seq, start 4[615], end 5[601], 42mer | 415 |
| | 20170608 cc6hb v4 - base 250nm 6hb, grey standard seq, start 4[657], end 5[643], 42mer | 416 |
| | 20170608 cc6hb v4 - base 250nm 6hb, grey standard seq, start 4[699], end 5[685], 42mer | 417 |
| | 20170608 cc6hb v4 - base 250nm 6hb, grey standard seq, start 4[741], end 5[727], 42mer | 418 |
| CGCATTAGACGGGAAGAGCCT | 20170608 cc6hb v4 - base 250nm 6hb, grey standard seq, start 4[769], end 5[762], 21mer | 419 |
| AAGACAATGTGAGCGAGTAACAACCCGT | 20170608 cc6hb v4 - base 250nm 6hb, grey standard seq, start 5[21], end 0[7], 28mer | 420 |
| | 20170608 cc6hb v4 - base 250nm 6hb, yellow standard seq, start 5[56], end 0[42], 42mer | 421 |
| | 20170608 cc6hb v4 - base 250nm 6hb, yellow standard seq, start 5[98], end 0[84], 42mer | 422 |
| | 20170608 cc6hb v4 - base 250nm 6hb, yellow standard seq, start 5[140], end 0[126], 42mer | 423 |
| | 20170608 cc6hb v4 - base 250nm 6hb, yellow standard seq, start 5[182], end 0[168], 42mer | 424 |
| | 20170608 cc6hb v4 - base 250nm 6hb, yellow standard seq, start 5[224], end 0[210], 42mer | 425 |
| | 20170608 cc6hb v4 - base 250nm 6hb, yellow standard seq, start 5[266], end 0[252], 42mer | 426 |
| | 20170608 cc6hb v4 - base 250nm 6hb, yellow standard seq, start 5[308], end 0[294], 42mer | 427 |
| | 20170608 cc6hb v4 - base 250nm 6hb, yellow standard seq, start 5[350], end 0[336], 42mer | 428 |
| | 20170608 cc6hb v4 - base 250nm 6hb, yellow standard seq, start 5[392], end 0[378], 42mer | 429 |
| | 20170608 cc6hb v4 - base 250nm 6hb, yellow standard seq, start 5[434], end 0[420], 42mer | 430 |
| | 20170608 cc6hb v4 - base 250nm 6hb, yellow standard seq, start 5[476], end 0[462], 42mer | 431 |
| | 20170608 cc6hb v4 - base 250nm 6hb, yellow standard seq, start 5[518], end 0[504], 42mer | 432 |
| | 20170608 cc6hb v4 - base 250nm 6hb, yellow standard seq, start 5[560], end 0[546], 42mer | 433 |
| | 20170608 cc6hb v4 - base 250nm 6hb, yellow standard seq, start 5[602], end 0[588], 42mer | 434 |
| | 20170608 cc6hb v4 - base 250nm 6hb, yellow standard seq, start 5[644], end 0[630], 42mer | 435 |
| | 20170608 cc6hb v4 - base 250nm 6hb, yellow standard seq, start 5[686], end 0[672], 42mer | 436 |
| | 20170608 cc6hb v4 - base 250nm 6hb, yellow standard seq, start 5[728], end 0[714], 42mer | 437 |
| | 20170608 cc6hb v4 - base 250nm 6hb, magenta standard seq, start 2[97], end 2[56], 42mer | 438 |
| | 20170608 cc6hb v4 - base 250nm 6hb, magenta standard seq, start 2[139], end 2[98], 42mer | 439 |
| | 20170608 cc6hb v4 - base 250nm 6hb, magenta standard seq, start 2[181], end 2[140], 42mer | 440 |
| | 20170608 cc6hb v4 - base 250nm 6hb, magenta standard seq, start 2[223], end 2[182], 42mer | 441 |
| | 20170608 cc6hb v4 - base 250nm 6hb, magenta standard seq, start 2[265], end 2[224], 42mer | 442 |
| | 20170608 cc6hb v4 - base 250nm 6hb, magenta standard seq, start 2[307], end 2[266], 42mer | 443 |
| | 20170608 cc6hb v4 - base 250nm 6hb, magenta standard seq, start 2[349], end 2[308], 42mer | 444 |
| | 20170608 cc6hb v4 - base 250nm 6hb, magenta standard seq, start 2[391], end 2[350], 42mer | 445 |
| | 20170608 cc6hb v4 - base 250nm 6hb, magenta standard seq, start 2[433], end 2[392], 42mer | 446 |
| | 20170608 cc6hb v4 - base 250nm 6hb, magenta standard seq, start 2[475], end 2[434], 42mer | 447 |
| | 20170608 cc6hb v4 - base 250nm 6hb, magenta standard seq, start 2[517], end 2[476], 42mer | 448 |
| | 20170608 cc6hb v4 - base 250nm 6hb, magenta standard seq, start 2[559], end 2[518], 42mer | 449 |
| | 20170608 cc6hb v4 - base 250nm 6hb, magenta standard seq, start 2[601], end 2[560], 42mer | 450 |
| | 20170608 cc6hb v4 - base 250nm 6hb, magenta standard seq, start 2[643], end 2[602], 42mer | 451 |
| | 20170608 cc6hb v4 - base 250nm 6hb, magenta standard seq, start 2[685], end 2[644], 42mer | 452 |
| | 20170608 cc6hb v4 - base 250nm 6hb, magenta standard seq, start 2[726], end 2[686], 41mer | 453 |
| AGAGAGATAACCCACAAGTAAGCAGATAG | 20170608 cc6hb v4 - base 250nm 6hb, magenta standard seq, start 2[755], end 2[727], 29mer | 454 |
| | 20170608, cc6hb v4 - 250nm 16 component square, default 6hb, miniscaf, node-0 | 455 |
| | 20170608, cc6hb v4 - 250nm 16 component square, default 6hb, miniscaf, node-1 | 456 |
| | 20170608, cc6hb v4 - 250nm 16 component square, default 6hb, miniscaf, node-2 | 457 |
| | 20170608, cc6hb v4 - 250nm 16 component square, default 6hb, miniscaf, node-3 | 458 |
| | 20170608, cc6hb v4 - 250nm 16 component square, default 6hb, miniscaf, node-4 | 459 |
| | 20170608, cc6hb v4 - 250nm 16 component square, default 6hb, miniscaf, node-5 | 460 |
| | 20170608, cc6hb v4 - 250nm 16 component square, default 6hb, miniscaf, node-6 | 461 |
| | 20170608, cc6hb v4 - 250nm 16 component square, default 6hb, miniscaf, node-7 | 462 |
| | 20170608, cc6hb v4 - 250nm 16 component square, default 6hb, miniscaf, node-8 | 463 |
| | 20170608, cc6hb v4 - 250nm 16 component square, default 6hb, miniscaf, node-9 | 464 |
| | 20170608, cc6hb v4 - 250nm 16 component square, default 6hb, miniscaf, node-10 | 465 |
| | 20170608, cc6hb v4 - 250nm 16 component square, default 6hb, miniscaf, node-11 | 466 |
| | 20170608, cc6hb v4 - 250nm 16 component square, default 6hb, miniscaf, node-12 | 467 |
| | 20170608, cc6hb v4 - 250nm 16 component square, default 6hb, miniscaf, node-13 | 468 |
| | 20170608, cc6hb v4 - 250nm 16 component square, default 6hb, miniscaf, node-14 | 469 |
| | 20170608, cc6hb v4 - 250nm 16 component square, default 6hb, miniscaf, node-15 | 470 |
| | 20170608, cc6hb v4 - 250nm 16 component square, default 6hb, miniscaf, node-16 | 471 |
| | cc6hbv3_miniscaf_10s_n4 | 472 |
| | cc6hbv3_miniscaf_10s_n5 | 473 |
| | cc6hbv3_miniscaf_10s_n6 | 474 |
| | cc6hbv3_miniscaf_10s_n7 | 475 |
| | cc6hbv3_miniscaf_10s_n8 | 476 |
| | cc6hbv3_yellow_term_10s_n4 | 477 |
| | cc6hbv3_yellow_term_10s_n5 | 478 |
| | cc6hbv3_yellow_term_10s_n6 | 479 |
| | cc6hbv3_yellow_term_10s_n7 | 480 |
| | cc6hbv3_yellow_term_10s_n8 | 481 |

**Table 3. Exemplary 440 nm Six-helix Bundle**

| **Sequence** | **Comment** | **SEQ ID NO:** |
|---|---|---|
| | 6hb_440nm, start 0[76], end 5[62] | 482 |
| | 6hb_440nm, start 0[118], end 5[104] | 483 |
| | 6hb_440nm, start 0[160], end 5[146] | 484 |
| | 6hb_440nm, start 0[202], end 5[188] | 485 |
| | 6hb_440nm, start 0[244], end 5[230] | 486 |
| | 6hb_440nm, start 0[286], end 5[272] | 487 |
| | 6hb_440nm, start 0[328], end 5[314] | 488 |
| | 6hb_440nm, start 0[370], end 5[356] | 489 |
| | 6hb_440nm, start 0[412], end 5[398] | 490 |
| | 6hb_440nm, start 0[454], end 5[440] | 491 |
| | 6hb_440nm, start 0[496], end 5[482] | 492 |
| | 6hb_440nm, start 0[538], end 5[524] | 493 |
| | 6hb_440nm, start 0[580], end 5[566] | 494 |
| | 6hb_440nm, start 0[622], end 5[608] | 495 |
| | 6hb_440nm, start 0[664], end 5[650] | 496 |
| | 6hb_440nm, start 0[706], end 5[692] | 497 |
| | 6hb_440nm, start 0[748], end 5[734] | 498 |
| | 6hb_440nm, start 0[790], end 5[776] | 499 |
| | 6hb_440nm, start 0[832], end 5[818] | 500 |
| | 6hb_440nm, start 0[874], end 5[860] | 501 |
| | 6hb_440nm, start 0[916], end 5[902] | 502 |
| | 6hb_440nm, start 0[958], end 5[944] | 503 |
| | 6hb_440nm, start 0[1000], end 5[986] | 504 |
| | 6hb_440nm, start 0[1042], end 5[1028] | 505 |
| | 6hb_440nm, start 0[1084], end 5[1070] | 506 |
| | 6hb_440nm, start 0[1126], end 5[1112] | 507 |
| | 6hb_440nm, start 0[1168], end 5[1154] | 508 |
| | 6hb_440nm, start 0[1210], end 5[1196] | 509 |
| | 6hb_440nm, start 0[1252], end 5[1238] | 510 |
| | 6hb_440nm, start 0[1294], end 5[1280] | 511 |
| | 6hb_440nm, start 0[1336], end 5[1322] | 512 |
| | 6hb_440nm, start 1[38], end 1[79] | 513 |
| | 6hb_440nm, start 1[80], end 1[121] | 514 |
| | 6hb_440nm, start 1[122], end 1[163] | 515 |
| | 6hb_440nm, start 1 [164], end 1[205] | 516 |
| | 6hb_440nm, start 1 [206], end 1[247] | 517 |
| | 6hb_440nm, start 1 [248], end 1[289] | 518 |
| | 6hb_440nm, start 1[290], end 1[331] | 519 |
| | 6hb_440nm, start 1 [332], end 1[373] | 520 |
| | 6hb_440nm, start 1[374], end 1[415] | 521 |
| | 6hb_440nm, start 1[416], end 1[457] | 522 |
| | 6hb_440nm, start 1[458], end 1[499] | 523 |
| | 6hb_440nm, start 1 [500], end 1[541] | 524 |
| | 6hb_440nm, start 1 [542], end 1[583] | 525 |
| | 6hb_440nm, start 1 [584], end 1[625] | 526 |
| | 6hb_440nm, start 1 [626], end 1[667] | 527 |
| | 6hb_440nm, start 1^{┌}668^{┐}, end 1^{┌}709^{┐} | 528 |
| | 6hb_440nm, start 1 [710], end 1[751] | 529 |
| | 6hb_440nm, start 1 [752], end 1[793] | 530 |
| | 6hb_440nm, start 1 [794], end 1[835] | 531 |
| | 6hb_440nm, start 1 [836], end 1[877] | 532 |
| | 6hb_440nm, start 1 [878], end 1[919] | 533 |
| | 6hb_440nm, start 1 [920], end 1 [961] | 534 |
| | 6hb_440nm, start 1 [962], end 1 [1003] | 535 |
| | 6hb_440nm, start 1 [1004], end 1 [1045] | 536 |
| | 6hb_440nm, start 1[1046], end 1[1087] | 537 |
| | 6hb_440nm, start 1[1088], end 1[1129] | 538 |
| | 6hb_440nm, start 1 [1130], end 1 [1171] | 539 |
| | 6hb_440nm, start 1[1172], end 1[1213] | 540 |
| | 6hb_440nm, start 1 [1214], end 1 [1255] | 541 |
| | 6hb_440nm, start 1[1256], end 1[1297] | 542 |
| | 6hb_440nm, start 1[1298], end 1[1343] | 543 |
| | 6hb_440nm, start 1 [1344], end 5[1364] | 544 |
| | 6hb_440nm, start 2[62], end 3[45] | 545 |
| | 6hb_440nm, start 2[104], end 2[63] | 546 |
| | 6hb_440nm, start 2[146], end 2[105] | 547 |
| | 6hb_440nm, start 2[188], end 2[147] | 548 |
| | 6hb_440nm, start 2[230], end 2[189] | 549 |
| | 6hb_440nm, start 2[272], end 2[231] | 550 |
| | 6hb_440nm, start 2[314], end 2[273] | 551 |
| | 6hb_440nm, start 2[356], end 2[315] | 552 |
| | 6hb_440nm, start 2[398], end 2[357] | 553 |
| | 6hb_440nm, start 2[440], end 2[399] | 554 |
| | 6hb_440nm, start 2[482], end 2[441] | 555 |
| | 6hb_440nm, start 2[524], end 2[483] | 556 |
| | 6hb_440nm, start 2^{┌}566^{┐}, end 2^{┌}525^{┐} | 557 |
| | 6hb_440nm, start 2[608], end 2[567] | 558 |
| | 6hb_440nm, start 2[650], end 2[609] | 559 |
| | 6hb_440nm, start 2[692], end 2[651] | 560 |
| | 6hb_440nm, start 2[734], end 2[693] | 561 |
| | 6hb_440nm, start 2[776], end 2[735] | 562 |
| | 6hb_440nm, start 2[818], end 2[777] | 563 |
| | 6hb_440nm, start 2[860], end 2[819] | 564 |
| | 6hb_440nm, start 2[902], end 2[861] | 565 |
| | 6hb_440nm, start 2[944], end 2[903] | 566 |
| | 6hb_440nm, start 2[986], end 2[945] | 567 |
| | 6hb_440nm, start 2[1028], end 2[987] | 568 |
| | 6hb_440nm, start 2[1070], end 2[1029] | 569 |
| | 6hb_440nm, start 2[1112], end 2[1071] | 570 |
| | 6hb_440nm, start 2[1154], end 2[1113] | 571 |
| | 6hb_440nm, start 2[1196], end 2[1155] | 572 |
| | 6hb_440nm, start 2[1238], end 2[1197] | 573 |
| | 6hb_440nm, start 2[1280], end 2[1239] | 574 |
| | 6hb_440nm, start 2[1322], end 2[1281] | 575 |
| | 6hb_440nm, start 2[1364], end 2[1323] | 576 |
| | 6hb_440nm, start 3[46], end 3[87] | 577 |
| | 6hb_440nm, start 3[88], end 3[129] | 578 |
| | 6hb_440nm, start 3[130], end 3[171] | 579 |
| | 6hb_440nm, start 3[172], end 3[213] | 580 |
| | 6hb_440nm, start 3[214], end 3[255] | 581 |
| | 6hb_440nm, start 3[256], end 3[297] | 582 |
| | 6hb_440nm, start 3[298], end 3[339] | 583 |
| | 6hb_440nm, start 3[340], end 3[381] | 584 |
| | 6hb_440nm, start 3[382], end 3[423] | 585 |
| | 6hb_440nm, start 3^{┌}424^{┐}, end 3^{┌}465^{┐} | 586 |
| | 6hb_440nm, start 3[466], end 3[507] | 587 |
| | 6hb_440nm, start 3[508], end 3[549] | 588 |
| | 6hb_440nm, start 3[550], end 3[591] | 589 |
| | 6hb_440nm, start 3[592], end 3[633] | 590 |
| | 6hb_440nm, start 3[634], end 3[675] | 591 |
| | 6hb_440nm, start 3[676], end 3[717] | 592 |
| | 6hb_440nm, start 3[718], end 3[759] | 593 |
| | 6hb_440nm, start 3[760], end 3[801] | 594 |
| | 6hb_440nm, start 3[802], end 3[843] | 595 |
| | 6hb_440nm, start 3[844], end 3[885] | 596 |
| | 6hb_440nm, start 3[886], end 3[927] | 597 |
| | 6hb_440nm, start 3[928], end 3[969] | 598 |
| | 6hb_440nm, start 3[970], end 3[1011] | 599 |
| | 6hb_440nm, start 3[1012], end 3[1053] | 600 |
| | 6hb_440nm, start 3[1054], end 3[1095] | 601 |
| | 6hb_440nm, start 3[1096], end 3[1137] | 602 |
| | 6hb_440nm, start 3[1138], end 3[1179] | 603 |
| | 6hb_440nm, start 3[1180], end 3[1221] | 604 |
| | 6hb_440nm, start 3[1222], end 3[1263] | 605 |
| | 6hb_440nm, start 3[1264], end 3[1305] | 606 |
| | 6hb_440nm, start 3[1306], end 3[1347] | 607 |
| | 6hb_440nm, start 3[1348], end 2[1365] | 608 |
| | 6hb_440nm, start 4[48], end 0[35] | 609 |
| | 6hb_440nm, start 4[90], end 0[77] | 610 |
| | 6hb_440nm, start 4[132], end 0[119] | 611 |
| | 6hb_440nm, start 4[174], end 0[161] | 612 |
| | 6hb_440nm, start 4[216], end 0[203] | 613 |
| | 6hb_440nm, start 4[258], end 0[245] | 614 |
| | 6hb_440nm, start 4^{┌}300^{┐}, end 0^{┌}287^{┐} | 615 |
| | 6hb_440nm, start 4[342], end 0[329] | 616 |
| | 6hb_440nm, start 4[384], end 0[371] | 617 |
| | 6hb_440nm, start 4[426], end 0[413] | 618 |
| | 6hb_440nm, start 4[468], end 0[455] | 619 |
| | 6hb_440nm, start 4[510], end 0[497] | 620 |
| | 6hb_440nm, start 4[552], end 0[539] | 621 |
| | 6hb_440nm, start 4[594], end 0[581] | 622 |
| | 6hb_440nm, start 4[636], end 0[623] | 623 |
| | 6hb_440nm, start 4[678], end 0[665] | 624 |
| | 6hb_440nm, start 4[720], end 0[707] | 625 |
| | 6hb_440nm, start 4[762], end 0[749] | 626 |
| | 6hb_440nm, start 4[804], end 0[791] | 627 |
| | 6hb_440nm, start 4[846], end 0[833] | 628 |
| | 6hb_440nm, start 4[888], end 0[875] | 629 |
| | 6hb_440nm, start 4[930], end 0[917] | 630 |
| | 6hb_440nm, start 4[972], end 0[959] | 631 |
| | 6hb_440nm, start 4[1014], end 0[1001] | 632 |
| | 6hb_440nm, start 4[1056], end 0[1043] | 633 |
| | 6hb_440nm, start 4[1098], end 0[1085] | 634 |
| | 6hb_440nm, start 4[1140], end 0[1127] | 635 |
| | 6hb_440nm, start 4[1182], end 0[1169] | 636 |
| | 6hb_440nm, start 4[1224], end 0[1211] | 637 |
| | 6hb_440nm, start 4[1266], end 0[1253] | 638 |
| | 6hb_440nm, start 4[1308], end 0[1295] | 639 |
| | 6hb_440nm, start 4[1350], end 0[1337] | 640 |
| | 6hb_440nm, start 5[63], end 4[49] | 641 |
| | 6hb_440nm, start 5[105], end 4[91] | 642 |
| | 6hb_440nm, start 5[147], end 4[133] | 643 |
| | 6hb_440nm, start 5^{┌}189^{┐}, end 4^{┌}175^{┐} | 644 |
| | 6hb_440nm, start 5[231], end 4[217] | 645 |
| | 6hb_440nm, start 5[273], end 4[259] | 646 |
| | 6hb_440nm, start 5[315], end 4[301] | 647 |
| | 6hb_440nm, start 5[357], end 4[343] | 648 |
| | 6hb_440nm, start 5[399], end 4[385] | 649 |
| | 6hb_440nm, start 5[441], end 4[427] | 650 |
| | 6hb_440nm, start 5[483], end 4[469] | 651 |
| | 6hb_440nm, start 5[525], end 4[511] | 652 |
| | 6hb_440nm, start 5[567], end 4[553] | 653 |
| | 6hb_440nm, start 5[609], end 4[595] | 654 |
| | 6hb_440nm, start 5[651], end 4[637] | 655 |
| | 6hb_440nm, start 5[693], end 4[679] | 656 |
| | 6hb_440nm, start 5[735], end 4[721] | 657 |
| | 6hb_440nm, start 5[777], end 4[763] | 658 |
| | 6hb_440nm, start 5[819], end 4[805] | 659 |
| | 6hb_440nm, start 5[861], end 4[847] | 660 |
| | 6hb_440nm, start 5[903], end 4[889] | 661 |
| | 6hb_440nm, start 5[945], end 4[931] | 662 |
| | 6hb_440nm, start 5[987], end 4[973] | 663 |
| | 6hb_440nm, start 5[1029], end 4[1015] | 664 |
| | 6hb_440nm, start 5[1071], end 4[1057] | 665 |
| | 6hb_440nm, start 5[1113], end 4[1099] | 666 |
| | 6hb_440nm, start 5[1155], end 4[1141] | 667 |
| | 6hb_440nm, start 5[1197], end 4[1183] | 668 |
| | 6hb_440nm, start 5[1239], end 4[1225] | 669 |
| | 6hb_440nm, start 5[1281], end 4[1267] | 670 |
| | 6hb_440nm, start 5[1323], end 4[1309] | 671 |
| | 6hb_440nm, start 5[1365], end 4[1351] | 672 |
| | 6hb_440nm, 7bp socket end distal to queen, start 5[280], end 4[259] | 673 |
| | 6hb_440nm, 7bp socket end distal to queen, start 5[322], end 4[301] | 674 |
| | 6hb_440nm, 7bp socket end distal to queen, start 5[364], end 4[343] | 675 |
| | 6hb_440nm, 7bp socket end distal to queen, start 5[406], end 4[385] | 676 |
| | 6hb_440nm, 7bp socket end distal to queen, start 5[448], end 4[427] | 677 |
| | 6hb_440nm, 10bp socket end distal to queen, start 5[283], end 4[259] | 678 |
| | 6hb_440nm, 10bp socket end distal to queen, start 5[325], end 4[301] | 679 |
| | 6hb_440nm, 10bp socket end distal to queen, start 5[367], end 4[343] | 680 |
| | 6hb_440nm, 10bp socket end distal to queen, start 5[409], end 4[385] | 681 |
| | 6hb_440nm, 10bp socket end distal to queen, start 5[451], end 4[427] | 682 |

The indefinite articles "a" and "an," as used herein in the specification and in the claims, unless clearly indicated to the contrary, should be understood to mean "at least one."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, *i.e.,* to mean including but not limited to.

## Claims

1. A crisscross nucleic acid slat, comprising:
(a) a first plurality of at least four nucleic acid strands aligned parallel to each other, each strand of the first plurality having a length of 15-30 nucleotides; and
(b) a second plurality of at least four nucleic acid strands aligned parallel to each, each strand of the second plurality having a length of 15-30 nucleotides, wherein the at least four nucleic acid strands of the first plurality are bound to and perpendicular to the at least four nucleic acid strands of the second plurality, a single nucleic acid strand of (b) binds to multiple nucleic acid strands of (a), each through a single cooperative binding site, and a single nucleic acid strand of (a) binds to multiple nucleic acid strands of (b), each through a single cooperative binding site.

2. A nucleic acid nanostructure, comprising a nucleic acid scaffold strand folded into multiple stacked parallel loops that are bound to at least one crisscross nucleic acid slat of claim 1.

3. A nucleic acid nanostructure, comprising a stack of parallel nucleic acid loops bound to a plurality of nucleic acid slats of claim 1.

4. A biomolecule detection method, comprising
(a) combining in a reaction mixture
(i) a sample comprising a biomolecule;
(ii) a nucleic acid strand capable of self-assembling into a nanostructure that comprises stacked parallel strands;
(iii) least two crisscross nucleic acid slats of claim 1, wherein the two slats bind to the stacked parallel strands of (ii), and wherein at least two of the slats are linked to a biomolecule binding partner that specifically binds to the biomolecule in the sample;
(b) incubating the reaction mixture under conditions that permit binding of the biomolecule binding partners to the biomolecule and assembly of the nanostructure into stacked parallel strands; and
(c) incubating the reaction mixture of (b) in the presence of a plurality of crisscross nucleic acid slats of claim 1, wherein the crisscross nucleic acid slats bind to the stacked parallel strands to form a stable three-dimensional nanostructure.

5. The method of claim 4, wherein said method further comprises imaging the three-dimensional nanostructure.

6. A biomolecule detection method, comprising:
combining in a reaction mixture
(a) a sample comprising a biomolecule and
(b) a nucleic acid nanostructure comprising (i) a nucleic acid scaffold strand and nucleic acid staple strands capable of assembling into multiple stacked parallel loops and (ii) two crisscross nucleic acid slats of claim 1 that are programmed to bind to the loops of (i),
wherein a biomolecule binding partner that specifically binds to the biomolecule is linked to each of the crisscross nucleic acid slats such that in the presence of the biomolecule the biomolecule binding partners bind to the biomolecule and the nucleic acid nanostructure folds into multiple stacked parallel loops; and
incubating the reaction mixture to assemble multiple stacked parallel loops.

7. The method of claim 6, wherein optionally said method further comprises combining the reaction mixture with a plurality of crisscross nucleic acid slats of claim 1 to form a three-dimensional nanostructure.

## Patentansprüche

1. Kreuzweise Nukleinsäure-Lamelle ("crisscross nucleic acid slat"), umfassend:
(a) eine erste Vielzahl von wenigstens vier parallel zueinander ausgerichteten Nukleinsäuresträngen, wobei jeder Strang der ersten Vielzahl eine Länge von 15-30 Nukleotiden hat; und
(b) eine zweite Vielzahl von wenigstens vier parallel zueinander ausgerichteten Nukleinsäuresträngen, wobei jeder Strang der zweiten Vielzahl eine Länge von 15-30 Nukleotiden hat, wobei die wenigstens vier Nukleinsäurestränge der ersten Vielzahl an die wenigstens vier Nukleinsäurestränge der zweiten Vielzahl gebunden sind und senkrecht zu diesen sind, ein einzelner Nukleinsäurestrang von (b) an mehrere Nukleinsäurestränge von (a) bindet, jeweils durch eine einzige kooperative Bindungsstelle, und ein einzelner Nukleinsäurestrang von (a) an mehrere Nukleinsäurestränge von (b) bindet, jeweils durch eine einzige kooperative Bindungsstelle.

2. Nukleinsäure-Nanostruktur, umfassend einen Nukleinsäure-Gerüststrang ("nucleic acid scaffold strand"), der in mehrere gestapelte parallele Schleifen gefaltet ist, die an wenigstens eine kreuzweise Nukleinsäure-Lamelle gemäß Anspruch 1 gebunden sind.

3. Nukleinsäure-Nanostruktur, umfassend einen Stapel von parallelen Nukleinsäure-Schleifen, die an eine Vielzahl von Nukleinsäure-Lamellen gemäß Anspruch 1 gebunden sind.

4. Biomolekül-Nachweisverfahren, umfassend:
(a) Vereinen in einem Reaktionsgemisch
(i) eine Biomolekül-umfassende Probe;
(ii) einen Nukleinsäurestrang, der fähig ist, sich selbst in eine Nanostruktur zusammenzusetzen, die gestapelte parallele Stränge umfasst;
(iii) wenigstens zwei kreuzweise Nukleinsäure-Lamellen gemäß Anspruch 1, wobei die zwei Lamellen an die gestapelten parallelen Stränge von (ii) binden, und wobei wenigstens zwei von den Lamellen mit einem Biomolekül-Bindungspartner verknüpft sind, der spezifisch an das Biomolekül in der Probe bindet;
(b) Inkubieren des Reaktionsgemisches unter Bedingungen, die das Binden der Biomolekül-Bindungspartner an das Biomolekül und Zusammensetzen der Nanostruktur zu gestapelten parallelen Strängen erlauben; und
(c) Inkubieren des Reaktionsgemisches aus (b) in Gegenwart von einer Vielzahl von kreuzweisen Nukleinsäure-Lamellen gemäß Anspruch 1, wobei die kreuzweisen Nukleinsäure-Lamellen an die gestapelten parallelen Stränge binden, um eine stabile dreidimensionale Nanostruktur zu bilden.

5. Verfahren gemäß Anspruch 4, wobei das Verfahren weiter Bildgebung der dreidimensionalen Nanostruktur umfasst.

6. Biomolekül-Nachweisverfahren, umfassend:
Vereinen in einem Reaktionsgemisch
(a) eine Biomolekül-umfassende Probe und
(b) eine Nukleinsäure-Nanostruktur, umfassend (i) einen Nukleinsäure-Gerüststrang und Nukleinsäure-Klammerstränge ("nucleic acid staple strands"), die fähig sind, in mehrere gestapelte parallele Schleifen zusammengesetzt zu werden und (ii) zwei kreuzweise Nukleinsäure-Lamellen gemäß Anspruch 1, die programmiert sind, an die Schleifen von (i) zu binden,
wobei ein Biomolekül-Bindungspartner, der spezifisch an das Biomolekül bindet, mit jedem der kreuzweisen Nukleinsäure-Lamellen so verknüpft ist, dass in Gegenwart des Biomoleküls die Biomolekül-Bindungspartner an das Biomolekül binden, und die Nukleinsäure-Nanostruktur in mehrere gestapelte parallele Schleifen gefaltet wird; und
Inkubieren des Reaktionsgemisches, um die mehreren gestapelten parallelen Schleifen zusammenzusetzen.

7. Verfahren gemäß Anspruch 6, wobei das Verfahren optional weiter Vereinen des Reaktionsgemisches mit einer Vielzahl von kreuzweisen Nukleinsäure-Lamellen gemäß Anspruch 1 umfasst, um eine dreidimensionale Nanostruktur zu bilden.

## Revendications

1. Lamelle d'acide nucléique entrecroisée, comprenant :
(a) une première pluralité d'au moins quatre brins d'acide nucléique alignés parallèlement les uns aux autres, chaque brin de la première pluralité présentant une longueur de 15-30 nucléotides ; et
(b) une seconde pluralité d'au moins quatre brins d'acide nucléique alignés parallèlement les uns aux autres, chaque brin de la seconde pluralité présentant une longueur de 15-30 nucléotides, dans laquelle les au moins quatre brins d'acide nucléique de la première pluralité sont liés et perpendiculaires aux au moins quatre brins d'acide nucléique de la seconde pluralité, un seul brin d'acide nucléique de (b) se lie à de multiples brins d'acide nucléique de (a), chacun par l'intermédiaire d'un seul site de liaison coopératif, et un seul brin d'acide nucléique de (a) se lie à de multiples brins d'acide nucléique de (b), chacun par l'intermédiaire d'un seul site de liaison coopératif.

2. Nanostructure d'acide nucléique, comprenant un brin d'échafaudage d'acide nucléique replié en de multiples boucles parallèles empilées qui sont liées à au moins une lamelle d'acide nucléique entrecroisée selon la revendication 1.

3. Nanostructure d'acide nucléique, comprenant un empilement de boucles d'acide nucléique parallèles liées à une pluralité de lamelles d'acide nucléique selon la revendication 1.

4. Procédé de détection de biomolécule, comprenant les étapes consistant à
(a) combiner dans un mélange réactionnel
(i) un échantillon comprenant une biomolécule ;
(ii) un brin d'acide nucléique capable de s'auto-assembler en une nanostructure qui comprend des brins parallèles empilés ;
(iii) au moins deux lamelles d'acide nucléique entrecroisées selon la revendication 1, dans lequel les deux lamelles se lient aux brins parallèles empilés de (ii), et dans lequel au moins deux des lamelles sont liées à un partenaire se liant à une biomolécule qui se lie spécifiquement à la biomolécule dans l'échantillon ;
(b) mettre en incubation le mélange réactionnel dans des conditions qui permettent la liaison des partenaires se liant à une biomolécule à la biomolécule et l'assemblage de la nanostructure en brins parallèles empilés ; et
(c) mettre en incubation le mélange réactionnel de (b) en présence d'une pluralité de lamelles d'acide nucléique entrecroisées selon la revendication 1, dans lequel les lamelles d'acide nucléique entrecroisées se lient aux brins parallèles empilés pour former une nanostructure tridimensionnelle stable.

5. Procédé selon la revendication 4, dans lequel ledit procédé comprend en outre l'imagerie de la nanostructure tridimensionnelle.

6. Procédé de détection de biomolécule, comprenant les étapes consistant à :
combiner dans un mélange réactionnel
(a) un échantillon comprenant une biomolécule et
(b) une nanostructure d'acide nucléique comprenant (i) un brin d'échafaudage d'acide nucléique et des brins courts d'acide nucléique capables de s'assembler en de multiples boucles parallèles empilées et (ii) deux lamelles d'acide nucléique entrecroisées selon la revendication 1 qui sont programmées pour se lier aux boucles de (i),
dans lequel un partenaire se liant à une biomolécule qui se lie spécifiquement à la biomolécule est lié à chacune des lamelles d'acide nucléique entrecroisées de telle sorte qu'en présence de la biomolécule, les partenaires se liant à une biomolécule se lient à la biomolécule et la nanostructure d'acide nucléique se replie en de multiples boucles parallèles empilées ; et
mettre en incubation le mélange réactionnel pour assembler de multiples boucles parallèles empilées.

7. Procédé selon la revendication 6, dans lequel facultativement ledit procédé comprend en outre la combinaison du mélange réactionnel avec une pluralité de lamelles d'acide nucléique entrecroisées selon la revendication 1 pour former une nanostructure tridimensionnelle.
